(19) Europäisches Patentamt
European Patent Office
Office européen des brevets

(11) EP 2 832 845 A1

(12) **EUROPEAN PATENT APPLICATION**
published in accordance with Art. 153(4) EPC

(43) Date of publication:
04.02.2015 Bulletin 2015/06

(51) Int Cl.:
*C12M 1/00* *(2006.01)*    *C12M 1/34* *(2006.01)*
*C12Q 1/02* *(2006.01)*    *G01N 21/27* *(2006.01)*
*G01N 21/64* *(2006.01)*    *G01N 33/48* *(2006.01)*
*G01N 33/483* *(2006.01)*    *G01N 37/00* *(2006.01)*

(21) Application number: 13769361.0

(22) Date of filing: 29.03.2013

(86) International application number:
PCT/JP2013/059453

(87) International publication number:
WO 2013/147114 (03.10.2013 Gazette 2013/40)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 30.03.2012 JP 2012081250
20.09.2012 JP 2012207517

(71) Applicants:
• Kanagawa Academy Of Science And Technology
Kawasaki-shi, Kanagawa 213-0012 (JP)
• On-Chip Cellomics Consortium
Tokyo 100-0005 (JP)
• National University Corporation
Tokyo Medical and Dental University
Bunkyo-ku
Tokyo 113-8510 (JP)

(72) Inventors:
• YASUDA Kenji
Tokyo 113-8510 (JP)
• KIM Hyonchol
Kawasaki-shi
Kanagawa 213-0012 (JP)
• TERAZONO Hideyuki
Kawasaki-shi
Kanagawa 213-0012 (JP)
• HATTORI Akihiro
Tokyo 100-0005 (JP)

(74) Representative: Pohlman, Sandra M.
df-mp Dörries Frank-Molnia & Pohlman
Patentanwälte Rechtsanwälte PartG mbB
Theatinerstrasse 16
80333 München (DE)

(54) **IMAGING CELL SORTER**

(57)    The present invention provides a cell enrichment/purification device having a function of continuously enriching cells, a function of locating the cells in a particular area of a flow path in a continuous array after the cell enrichment, a function of recognizing the shape of the cells and fluorescence emission from the cells at the same time in units of one cell based on an image, and a function of recognizing the cells based on the information on the shape and fluorescence emission to separate/purify the cells.

FIG. 4

EP 2 832 845 A1

**Description**

TECHNICAL FIELD

The present invention relates to a cell recovery device.

BACKGROUND ART

In biological tissues in multi-cell organisms, each of the various cells plays its own role to keep the function thereof in a coordinated manner. When a part of the tissue becomes a cancerous (herein, the term "cancer" encompasses cancers and tumors), the neoplasm of that part becomes different from the area around that part. Such a cancer area and a normal tissue area in close proximity to the cancer area are not necessarily separated along a border, and the area around the cancer area is influenced in some way. Therefore, in order to analyze the function of an organ tissue, a small number of cells present in a narrow area needs to be separated in a short time, as simply as possible, and with minimum loss.

In the field of regenerative medicine, there is an attempt to separate a stem cell of an organ from the tissue and re-culture the stem cell for differentiation-induction in order to regenerate a target tissue and finally regenerate an organ.

For identifying or for separating a cell, some distinguishing features are needed. In general, cells are distinguished as follows.

1) Morphological cell classification by visual observation: this is used, for example, for inspecting a bladder cancer or a urethra cancer by inspection of heterocysts appearing in the urine, classification of heterocyst in the blood, cancer inspection by cytodiagnosis on the tissue.

2) Cell classification by cell surface antigen (marker) being stained using a fluorescent antibody test: A cell surface antigen generally called a "CD marker" is stained with a fluorescence-labeled antibody specific thereto. This is used for cell separation or flow cytometry by use of a cell sorter or cancer inspection by use of tissue staining. Needless to say, this method is widely used for cytophysiological studies and industrial use of cells as well as for medicine.

3) In an example of stem cell separation, target stem cells are separated as follows. Stem cells are roughly separated from other cells using a fluorescent dye introduced into the cells as a reporter, and then the cells are actually cultured. Since no effective marker for stem cells has been established, the cells are actually cultured and only the differentiated-induced cells are utilized to substantially separate the target cells.

It is important for biological and medical analyses to separate and recover specific cells in the cultured solution as described above. For separating cells by the difference in the specific gravity of the cells, velocity sedimentation is usable. However, in the case where there is almost no difference in the specific gravity, for example, in the case where unsensitized cells and sensitized cells are to be distinguished, the cells need to be separated one by one based on information obtained by staining with fluorescent antibody or information obtained by visual checking.

For such a technology, a cell sorter, for example, is available, which is operated as follows. After cells are treated with fluorescence staining, each cell is isolated and caused to be contained in a charged droplet, and the droplets are dripped one by one. While the droplets are falling down, a high electric field is applied in an optional direction in a planar direction normal to the falling direction, based on whether or not there is fluorescence in the cell in the droplet and the magnitude of light scattering, so that the falling direction is controlled. Thus, the cells are fractionated into a plurality of containers located below and recovered. (Non-patent Document 1: Kamarck, M. E., Methods Enzymol., Vol. 151, pp. 150-165 (1987))

However, this method has the problem of having high costs; the device is large; a high electric field of several thousand volts is necessary; a large amount of samples enriched to a certain concentration is necessary; there is a risk that the cells may be damaged at the stage of creating the droplets; samples cannot be directly observed; and the like. In order to solve these problems, a cell sorter including microchannels formed by use of a microprocessing technology has been developed, such that cells flowing in a layered flow in the channel are separated while being directly observed with a microscope (Non-patent Document 2: Micro Total Analysis, 98, pp.77-80 (Kluwer Academic Publishers, 1988; Non-patent Document 3: Analytical Chemistry, 70, pp. 1909-1915 (1988)). However, with this cell sorter, which uses said microprocessing technology, the response speed of sample separation to the observation means is low. In order to use this cell sorter practically, a separation method providing a higher response speed without damaging the samples is needed. There are also the following problems. The cell concentration of the sample solutions to be used needs to be increased to a certain level in advance; when the cell concentration is low, the separation efficiency of the device cannot be sufficiently raised. In a case, where samples in a trace amount are enriched in another device, it is difficult to recover the enriched solutions without loss of cells, and without problems undesirable for regenerative medicine or the like, for example, the problem that the cells are contaminated on a complicated pre-processing stage occur.

In order to solve these problems, the present inventors have previously developed a cell analysis/separation device, which utilizes a microprocessing technology. This cell analysis/separation device fractionizes samples based on the small structure of the samples and the fluorescence distribution in the samples, and thus can analyze and separate the cell samples in a simpler manner without damaging the samples (Patent Document 1: Japanese Laid-Open Patent Publication No. 2003-107099; Patent Document 2: Japanese Laid-Open Patent Publication No. 2004-85323; Patent Document 3: WO2004/101731). These cell sorters are sufficiently practical on a laboratory level. For general-purpose uses, however, new technological development is necessary on the liquid transfer method, recovery method, and pre-processing including sample preparation.

Today, the level of detection of cancer tissues has been remarkably raised by improvement of MRI (magnetic resonance imaging) and CT (computed tomography). For identifying whether the tumor is malignant or benign, there is no technique exceeding the evaluation by use of biopsy. A known problem of a malignant tumor is metastasis of the tumor to another organ by an ability of infiltrating from the tissue of the cancer cell itself into the blood vessel or lymphatic vessel. Such malignant tumor cells circulating in the peripheral blood are called "circulating tumor cells (CTCs), and it is considered that about several hundred cancer cells are present in 100,000 blood cells (including erythrocytes). Recently, anticancer medicines against specific targets have been developed one after another. Therefore, once the type of the malignant tumor in the blood is identified, an anticancer medicine effectively destroying the cells can be selected. If a technology of monitoring CTCs flowing in the blood is realized, such a technology can measure, quantitatively, the presence of malignant tumor cells flowing in the blood, which cause cancer metastasis, evaluate the effect of the administered anticancer medicine quantitatively and continuously, and thus prevent unnecessary or excessive administration of an anticancer medicine, and also detect presence/absence of recurrence, for the first time in history.

For genetic diagnosis or expression analysis, polymerase chain reaction (hereinafter, abbreviated as PCR) is a method for amplifying a particular nucleotide sequence from a mixture of various types of nucleic acids. In PCR, a particular nucleic acid sequence can be amplified by performing at least one cycle of the following steps: the step of adding, into the mixture various types of nucleic acids, a DNA template such as, for example, genomic DNA or complementary DNA obtained by reverse transcription from messenger RNA, two or more types of primers, thermostable enzymes, salt such as magnesium or the like, and four types of deoxyribonucleoside triphosphates (dATP, dCTP, dGTP and dTTP), and then splitting the nucleic acids into single chains; the step of binding the primers into the separated nucleic acids; and the step of allowing hybridization using, as a template, the nucleic acids bound to the primers by the thermostable enzymes. In PCR, thermal cycling is performed by increasing and decreasing the temperature of a reaction vessel used for DNA amplification reaction. There are various usable mechanisms for changing the temperature, including, for example, a mechanism in which the temperature of the reaction vessel containing a sample is changed through heat exchange using a heater, a Peltier element or hot air; a mechanism in which the temperature is changed by alternately bringing the reaction vessel into contact with heater blocks or liquid baths of different temperatures; and a mechanism in which the temperature is changed by running a sample through a flow channel that has regions of different temperatures. Currently, the fastest commercially available device is, for example, Light Cycler from Roche. The Light Cycler has a mechanism where a sample, DNA polymerase, small sections of DNA as primers and a fluorescent dye label for measurement are placed into each of a plurality of glass capillary tubes, and the temperatures of small amounts of liquid droplets in the capillary tubes are changed by blowing hot air at a temperature intended for the liquid droplets, for example, at two temperatures of 55°C and 95°C, while at the same time, the glass capillary tubes are irradiated with excitation light colored with a fluorescent dye to measure the resulting fluorescence intensity.

By any of these methods, the temperature of the sample can be repeatedly changed.

CITATION LIST

PATENT LITERATURE

Patent Document 1: Japanese Laid-Open Patent Publication No. 2003-107099
Patent Document 2: Japanese Laid-Open Patent Publication No. 2004-85323
Patent Document 3: WO2004/101731

NON-PATENT LITERATURE

Non-patent Document 1: Kamarck, M. E., Methods Enzymol., Vol. 151, pp. 150-165 (1987)

Non-patent Document 2: Micro Total Analysis, 98, pp. 77-80 (Kluwer Academic Publishers, 1988)
Non-patent Document 3: Analytical Chemistry, 70, pp. 1909-1915 (1988)

## SUMMARY OF INVENTION

### TECHNICAL PROBLEM

Although it is considered as important on the clinical level to check the presence of CTCs regarding the metastasis of cancer, a diagnostic standard using this as an index of metastasis of cancer has not yet been established. A reason for this is as follows. The CTCs are diversified and rare. Therefore, with a conventional method of examining the presence/absence of such a rare mutant gene in the tissue, wherein a sampled specimen is regarded as a uniform tissue, the detection sensitivity needs to be extremely high.

Conventionally, analysis of the gene in the cell or of the expression is performed without checking whether the fluorescence-labeled cancer cell in the blood forms a cell cluster with other cells or is a solitary cell. Therefore, the information obtained as a result of the analysis represents an ensemble average, namely, includes information about cells other than the target cancer cell. This involves a problem that correct information about the target cancer cell is not obtained.

Conventionally, the diagnosis needs to be performed to provide a higher S/N ratio by use of means that recovers the cells in units of one cell and also by use of means that performs genetic diagnosis/expression analysis in units of a trace amount of cells after such rare cells are sorted and enriched.

The currently used cell analysis method has the problem of not including an analysis on whether the target cell is in an apoptotic state or not at the time of cell recovery.

A cell having a hard shell at a surface, for example, a spore of Bacillus anthracis, involves the problem that the contents in the cell are not eluted to a sample solution and thus cannot be analyzed unless the shell is removed in some method. Usually, a spore cell is cultured to be germinated. When the cell is germinated, the contents therein are eluted to the sample solution by the same procedure as used for a common cell. Therefore, cell culturing means is incorporated into the analysis means to perform cell analysis. In this case, however, the cell needs to be cultured for at least several hours or even for the whole day. This causes a problem that, for example, the culturing step extends the measurement time, complicates the procedure, or causes contamination. According to one fracturing technique for high-speed analysis, a mixture of a fracturing medium such as a glass ball or the like and a sample is put into a fracturing vessel, and the fracturing vessel is supplied with, for example, ultrasonic vibration to cause random collisions, so that the cell is fractured. Such a method has a problem that the cell is not efficiently fractured despite the sample solution being heated by the vibration, and also a problem that the sample solution and the sample are needed in a large amount. Thus, when cells in an extremely small amount are fractured, there is also an issue of sample recovery ratio.

In the case where the size of a sample flowing in a micro-flow path is large, the height of the flow path needs to be increased. This causes a problem that an image used to observe the sample is blurred.

In the case where a sample flows horizontally for a long time in a flow path that is located horizontally, there is an undesirable possibility that the sample is gradually precipitated and ends up occluding the flow path.

In the case where an external force is applied to particulates such as cells or the like in the form of a repulsive force in order to purify and recover the cells, it is difficult to gather the cells to one position in the flow path.

Application of an external electric field at merely one point is not sufficient to provide a sufficiently large external force to the sample, and thus there is an undesirable possibility that the cells cannot be transferred sufficiently.

A specifically quantified index for purifying a cardiac muscle cell by image recognition is unclear from the viewpoint of making the process automatic.

Narrowing an aqueous solution of sample by use of side sheath liquids may undesirably dilute the aqueous solution of sample.

During recovery of sample particulates from an aqueous solution, when the ionic strength of an electrolyte in the sample solution reaches a certain level, it may possibility be made difficult to transfer the particulates by use of an electric field.

One method for confirming the presence of a cancer cell has been filed for a patent by the present inventors (Japanese Laid-Open Patent Publication No. 2011-257241). According to this technology, a cell cluster is identified by image recognition. However, this technology cannot confirm a phenomenon of multinucleation, which is a feature of a cancer cell that can be recognized based on an image. This technology cannot provide an evaluation in combination with an existing staining technique using a cancer cell marker, either. Regarding a method for acquiring microscopic images of a plurality of wavelengths, the present inventors have devised an absorption imaging spectroscopy technology as an absorption microscope and filed for a patent as Japanese Laid-Open Patent Publication No. 2012-055267 and WO2012/060163, but none of these technologies includes a technology of measuring a plurality of fluorescence intensities.

Japanese Laid-Open Patent Publication No. 2011-257241 discloses a technology for detecting the presence/absence of a cancer cell in the blood. According to this technology, a fluorescence dye is attached to an antibody selectively bonded to a molecule (cancer marker) present only on a surface of a cancer cell, and the resultant antibody is reacted with blood. In this case, a cancer cell, if being present in the blood, emits fluorescence. This technology is realized by the following method and device structure. Blood flowing in a microchip while containing a cancer cell is irradiated with fluorescence excitation light, and then the fluorescence emitted from the cancer cell is caused to pass, a plurality of times, a mirror (dichroic mirror) that reflects light of a particular wavelength and transmits light of the other wavelengths. Thus, at each of steps, fluorescence of a particular wavelength band is extracted. The amount of the fluorescence of each particular wavelength band is measured by a light detector. The amount of fluorescence from the cancer cell, which is to be detected when the light passes the dichroic mirror, is attenuated in accordance with the number of times the light passes the dichroic mirror. Therefore, the fluorescence intensity measured at each wavelength band involves an error caused by the passage through the dichroic mirror. Especially, it is difficult to detect weak fluorescence at a wavelength on later stages. For this reason, it is difficult to quantitatively detect the multiple-stained cancer marker molecule. Alternatively, in order to allow a large number of fluorescence dyes to be used at the same time, a cancer cell may be irradiated with excitation light of a plurality of different wavelengths, so that the emitted fluorescence of a plurality of wavelengths can be detected at the same time. In order to realize this, light needs to pass a plurality of dichroic mirrors having excitation wavelengths that overlap the fluorescence wavelengths, so that light is divided into components each having a different wavelength. This complicates the device structure and also requires each wavelength band to be narrowed. This further attenuates signal fluorescence. In addition, it is difficult in terms of principle to divide light into components in close wavelength bands.

SOLUTION TO PROBLEM

In the light of such a situation, the present inventors provide a cell analysis device capable of identifying, at high speed, the type, state and number (concentration) of cancer cells flowing in the blood while having a metastatic ability.
The present invention provides the following device, system, and method.

(1) A cell analysis device system, comprising:

(A) a first device that performs enrichment, staining and washing on a cell sample solution from a test subject;
(B) a second device that performs enrichment, separation and purification on the sample solution of the stained cells from the first device;
(C) a third device that performs gene analysis/expression analysis on the purified cells in the cell sample solution from the second device;
(D) a fourth device that continuously transfers the cell sample solution from the first through third devices; and
(E) a control/analysis section that controls an operation of each of the devices and analyzes the cell sample;

wherein:

(a) the first device includes:

a chamber including a filter that performs enrichment, staining and washing on the cells in the cell sample solution;
a vessel that accommodates the cell sample solution, a staining liquid and a washing liquid; and
a mechanism that sequentially introduces the solution and the liquids in the vessel into the chamber;

(b) the second device includes:

a cell sorter chip including a flow path in which the cell sample solution containing cells that contains a target cell flows, the flow path including a first flow path in which the cell enrichment is performed and a second flow path which is branched from the first flow path and in which detection of the enriched cells and sorting of the target cell are performed;
a mechanism that applies an external force to the cells flowing in the flow path so that the cells flowing in the flow path are enriched in the first flow path and converged to a desired direction in the second flow path; and
an optical system including light radiation means that directs light toward the cells flowing in the second flow path and a high-speed camera that acquires an image of the cells at an image capturing rate of at least 200 frames/second; and

(c) the third device includes:

a reaction tank in which the sample solution is added for reaction;
a heat exchange tank that exchanges heat with the reaction tank; and
a temperature control mechanism that controls the temperature of the heat exchange tank.

(2) The cell analysis device system according to (1) above, further comprising, on a stage before the third device that performs gene analysis/expression analysis on the purified cells in the cell sample solution, a cell fracture mechanism that causes contents in the cells, transferred by the fourth device that transfers the cell sample solution, to be eluted to the sample solution by cell fracturing;
wherein the control/analysis section controls each of the sections such that the cell sample solution from the second device is transferred to the cell fracture mechanism by the fourth device that transfers the cell sample solution and such that the sample solution fractured by the cell fracture mechanism is transferred to the third device by the fourth device.

(3) The cell analysis device system according to (2) above, wherein:

the cell fracture mechanism includes:

a vessel that accommodates the cell sample;
a fracturing rotator that fractures the cells in the vessel; and
a grinding agent that fractures the cells in the vessel;
wherein the cell sample and the grinding agent are put into the vessel, and the cell sample is fractured by a motion of the fracturing rotator that is rotated and revolved in a strictly controlled manner.

(4) The cell analysis device system according to (3) above, wherein:

the cell fracture mechanism further includes a rotation shaft; and
the fracturing rotator is pressed from above by the rotation shaft to rotate in the vessel, and the frictional force and the degree of slippage between the fracturing rotator and the rotation shaft are controlled by the pressure between the fracturing rotator and the rotation shaft.

(5) The cell analysis device system according to (4) above, wherein the cell fracture mechanism has a mechanism of causing the rotation axis of the fracturing rotator and the rotation axis of the rotation shaft to be shifted from each other, so that a force of pressing the fracturing rotator to a side surface of the vessel at a right angle is generated.
(6) The cell analysis device system according to (4) above, wherein the cell fracture mechanism has a mechanism of raising the fracturing rotator in the vessel by a magnetic force of the rotation shaft, an electrostatic force or a suction power generated by the difference in gas pressure.
(7) The analysis device system according to any one of (3) through (6) above, wherein the cell fracture mechanism includes a driving mechanism having a plurality of automatically exchangeable vessels mounted thereon, so that contamination between different cell samples is prevented.
(8) The analysis device system according to any one of (3) through (7) above, wherein the vessel of the cell fracture mechanism, in an unused state, is closed with an air-tight seal while accommodating the fracturing rotator therein, and thus it is proven that neither the vessel nor the fracturing rotator is contaminated when fracturing the cell sample.
(9) An image-detecting one-cell separation/purification device, comprising:

(i) a cell sorter chip including a flow path in which a cell sample solution containing cells that contains a target cell flows, the flow path including a first flow path in which cell enrichment is performed and a second flow path which is branched from the first flow path and in which detection of the enriched cells and sorting of the target cell are performed;
(ii) a mechanism that applies an external force to the cells flowing in the flow path so that the cells flowing in the flow path are enriched in the first flow path and converged to a desired direction in the second flow path;
(iii) an optical system including light radiation means that directs light toward the cells flowing in the second flow path and a high-speed camera that acquires an image of each of the cells at an image capturing rate of at least 200 frames/second; and
(iv) a control/analysis section that controls an operation of each of the sections and analyzes the image of the cell captured by the optical system.

(10) The device according to (9) above, wherein the external force is an ultrasonic radiation pressure, a gravitational force, an electrostatic force or a dielectric electrophoretic force.

(11) The device according to (9) or (10) above, wherein the cell sample containing the target cell is derived from blood.

(12) The device according to any one of (9) through (11) above, wherein the target cell includes a cancer cell.

(13) The device according to any one of (9) through (12) above, wherein the control/analysis section binarizes the image of the cell obtained from the optical system, and detects, at a one-cell level, and identifies each of the cells by use of at least one index selected from the group consisting of the luminance centroid, the surface area, the perimeter length, the longer diameter and the shorter diameter of the binarized image.

(14) The device according to (13) above, wherein the cells in the cell sample solution are fluorescence-labeled, the optical system further includes fluorescence detection means, and information on the fluorescence image of the cell is used by the control/analysis section as an additional index.

The present invention further provides the following on-chip cell sorter and on-chip cell sorter system.

(15) An on-chip cell sorter in which one sample flow path, and two buffer solution flow paths that are located symmetrically to both of two sides of the sample flow path and each have the same length and the same cross-sectional area as those of the sample flow path, are located so as to be joined together; the flow paths are, after being joined together, branched again at a downstream position into a central recovery flow path and two waste liquid flow paths that are located to the sides of the central recovery flow path and each have the same length and the same cross-sectional area as those of the central recovery flow path; a sheath liquid reservoir that covers entrances of the three flow paths and a sample solution reservoir filled with the sample are located such that the ratio between the cross-sectional areas thereof is 2:1, which is the same as the ratio between the flow path numbers, so that even if the solution or the liquid flows, the two reservoirs have the same liquid surface level; similarly, also on the downstream side, a waste liquid reservoir and a cell recovery reservoir are located such that the ratio between the cross-sectional areas thereof is 2:1; a mechanism that identifies a cell by use of a high-speed camera and fluorescence detection is provided upstream of the joining point; and gel electrodes are located symmetrically so as to contact the joining point, so that an electric field is applied only to a cell to be eliminated.

(16) The cell sorter system according to (15) above, wherein the cell sorter includes, regarding the reservoirs, a plug located at a top surface of the sheath liquid reservoir; means that applies pressurized air through the plug, means that additionally provides a liquid to the sheath liquid reservoir and the sample reservoir continuously; and an electric sensor capable of measuring the liquid surface level of both of the sheath liquid reservoir and the sample reservoir.

(17) The cell sorter according to (15) above, wherein in the reservoirs of the cell sorter, vessels in which the sample solution and two sheath liquids are separately stored are located at upstream entrances of the three flow paths respectively.

(18) An image-recognizing on-chip cell sorter, wherein a cell in a division period is selectively recovered based on whether there is an image of a nucleus or not in a cell image.

(19) An on-chip cell sorter system, wherein in order to avoid image blur, a flash is fired once in each frame at each frame rate of a high-speed camera with the flash time being set as:

flash time = pixel size/flow rate.

(20) An on-chip cell sorter system, wherein an optical system is used which includes a combination of an objective lens having a numerical aperture of 0.3 or less and a zoom lens and in which a focal depth and a depth of field are maintained at a level approximately equal to a surface level of a micro-flow path.

(21) An on-chip cell sorter system, wherein a sample solution flows vertically from an upstream part to a downstream part.

(22) An on-chip cell sorter system, wherein an inner wall of a micro-flow path that faces a surface, on which an electrode is located to generate a repulsive force to sample particulates, protrudes upward.

(23) An on-chip cell sorter system, wherein a pair of gel electrodes each including a series of columns arrayed at a certain interval are located parallel to a flow path, so that gel in a sol solution state is prevented from leaking to the flow path by use of a surface tension of the solution.

(24) An on-chip cell sorter system, wherein based on shapes of cells acquired by image recognition, a cell having R of less than 1.1 is purified as a cardiac muscle cell where R is represented by the following expression:
[Expression 1]

$$R = \frac{l}{\sqrt{4\pi S}}$$

(1)

(25) An on-chip cell sorter system, wherein oil, which has a specific gravity smaller than that of water and thus is not well mixed with water, is used as a side sheath liquid.

(26) An on-chip cell sorter system, wherein an aqueous solution of sample, from which sample particulates are to be recovered, has a conductivity of $10^2$ $\mu$S/cm or less.

[1] An on-chip cell sorter system, comprising:

a cell sorter chip including a first flow path in which a specimen solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the external force application mechanism.

[2] The on-chip cell sorter system according to [1] above, which is structured such that a time lag between the timing when the cell identification result is obtained from the digital image acquired by the optical system and the timing when the external force is applied by the external force application mechanism is minimized.

[3] An on-chip cell sorter system, comprising:

a cell sorter chip including a first flow path in which a specimen solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the external force application mechanism; wherein the optical system includes a microscope including an objective lens having a numerical aperture of 0.3 or less and a zoom lens optically coupled to the objective lens.

[4] An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the external force application mechanism; wherein the cell sorter chip is located such that the first flow path is substantially parallel to a direction of the gravitational force and thus the sample solution flows substantially vertically from an upstream part to the downstream part of the first flow path.

[5] An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the external force application mechanism; wherein the external force application mechanism includes a gel electrode or a metal electrode that applies an electric force to particulates containing cells flowing in the first flow path, and the sample solution has a conductivity of $10^2$ μS/cm or less.

[6] The on-chip cell sorter system according to any one of [1] through [5] above, further comprising another external force application mechanism that applies, to the cells in the sample solution, an external force for arraying the cells in a third area upstream of the first area in the upstream part of the first flow path.

[7] The on-chip cell sorter system according to [6] above, wherein the other external force application mechanism that applies to the cells in the sample solution is an external force that uses an electric force or a sheath flow.

[8] An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path;

a control section that controls an operation of the optical system and the external force application mechanism;

a reservoir that is in fluid communication with an upstream part of the first flow path and accommodates a buffer solution for a sheath liquid; and

a sample solution introduction flow path which is in fluid communication with the upstream part of the first flow path and from which the sample solution containing the cells is introduced into the first flow path;

wherein a tip part, of the sample solution introduction flow path, that is in fluid communication with the first flow path extends to a position downstream of a position at which the buffer solution is introduced into the first flow path.

[9] An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;
a first external force application mechanism that applies, to the cells in the sample solution flowing in the first flow path, an external force for arraying the cells in a preliminary area upstream of the branch point;
an optical system that acquires a digital image of a cell in the sample solution flowing in the first flow path in a first area upstream of the branch point and downstream of the preliminary area, so that the target cell is identified by digital analysis performed on the image;
a second external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and
a control section that controls an operation of the optical system and the first and second external force application mechanisms;
wherein the first external force application mechanism is a comb-like electrode that is located on a surface of the first flow path and provides a repulsive force to particulates containing the cells in the sample solution, a cross-section of the first flow path perpendicular to a flow direction therein being tapered or protruding toward a center of a surface facing the surface on which the electrode is located, so that the arraying of the particulates is promoted.

[10] An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;
a first external force application mechanism that applies, to the cells in the sample solution flowing in the first flow path, an external force for arraying the cells in a preliminary area upstream of the branch point;
an optical system that acquires a digital image of a cell in the sample solution flowing in the first flow path in a first area upstream of the branch point and downstream of the preliminary area, so that the target cell is identified by digital analysis performed on the image;
a second external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path, while the cell other than the target cell is guided to the waste liquid recovery flow path; and
a control section that controls an operation of the optical system and the first and second external force application mechanisms;
wherein the second external force application mechanism includes gel electrodes located so as to contact the sample solution at both of two side sides of the first flow path via an array of slits provided at a certain interval along the two side surfaces of the first flow path, so that in the case where the gel is in a sol solution state, the sol solution is prevented, by use of a surface tension of the sol solution, from leaking to the first flow path.

[11] An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

a first external force application mechanism that applies, to the cells in the sample solution flowing in the first flow path, an external force for arraying the cells in a preliminary area upstream of the branch point;

an optical system that acquires a digital image of a cell in the sample solution flowing in the first flow path in a first area upstream of the branch point and downstream of the preliminary area, so that the target cell is identified by digital analysis performed on the image;

a second external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the first and second external force application mechanisms;

wherein the first external force application mechanism includes a pair of flow paths which are in fluid communication with an upstream part of the first flow path and in which a side sheath liquid, for forming a side sheath flow, flows, and the side sheath liquid consists of oil, which has a specific gravity smaller than that of water and thus is not well mixed with water.

[12] The on-chip cell sorter system according to any one of [9] through [11] above, which is structured such that a time lag between the timing when the cell identification result is obtained from the digital image acquired by the optical system and the timing when the external force is applied by the second external force application mechanism is minimized.

[13] The on-chip cell sorter system according to any one of [1] through [12] above, wherein the external force application mechanism that guides each of the cells to either the target cell recovery flow path or the waste liquid recovery flow path includes a gel electrode or a metal electrode that applies an electric force to the cells.

[14] The on-chip cell sorter system according to any one of [1] through [13] above, wherein:

the target cell is a cardiac muscle cell; and

based on shapes of the cells acquired by image recognition performed by the optical system, a cell having R of less than 1.1 is identified as the cardiac muscle cell where R is represented by the following expression:

[Expression 4]

$$R = \frac{l}{\sqrt{4\pi S}} \qquad (1)$$

[15] A method for sorting target cells in a sample solution by use of the on-chip cell sorter system according to any one of claims [1] through [14] above.

The present invention also provides the following on-chip cell sorter system and a method and an optical module for identifying a cancer cell candidate in the blood from a cell sample solution derived from a test subject by use of the system.

<1> An on-chip cell sorter system, comprising:

a cell sorter chip including a flow path in which a specimen solution containing fluorescence-stained cells derived from a test subject flows;

an optical system including a bright-field light source and a fluorescence source that emit light toward the cells;

a detection system that acquires, at the same time, a bright-field image of each of the cells in the sample solution flowing in the flow path of the cell sorter chip, a fluorescence intensity of a fluorescence-labeling substance bonded to the cell, and a fluorescence image of the cell;

control/analysis means that identifies a multinucleated cell and/or a cell cluster flowing in the flow path based on the bright-field image, the fluorescence intensity and the fluorescence image; and

means that selectively recovers the identified multinucleated cell and/or cell cluster.

<2> The on-chip cell sorter system according to <1> above, wherein the control/analysis means acquires:

i) at least one piece of data selected from the group consisting of a size (surface area) of the cell, a perimeter length of the cell, and a value of R, which represents a surface roughness of the cell obtained from the surface area and the perimeter length; and

ii) at least one piece of data selected from the group consisting of a wavelength spectrum of fluorescence of the fluorescence labeling substance bonded to the cell, an intensity spectrum of the fluorescence, a coordinate in the cell of the center of gravity of at least one fluorescence-stained region in the cell, and a surface area of the region; and

identifies the multinucleated cell and/or the cell cluster flowing in the flow path based on the data.

<3> The on-chip cell sorter system according to <1> or <2> above, further comprising means that measures a nucleic acid sequence of a gene derived from the selectively recovered multinucleated cell and/or cell cluster.

<4> The on-chip cell sorter system according to any one of <1> through <3> above, further comprising an image division mechanism having a function of dividing a light receiving surface of one high-speed camera so that the bright-field image and the fluorescence image are displayed on the light receiving surface at the same time.

<5> The on-chip cell sorter system according to <4> above, further comprising a mechanism that performs adjustment such that magnification ratios of the bright-field image and the fluorescence image are different from each other.

<6> The on-chip cell sorter system according to any one of <1> through <5> above, which is useable for identifying a cancer cell candidate in the blood.

<7> A method for identifying a cancer cell candidate in the blood from a cell sample solution derived from a test subject by use of the on-chip cell sorter according to any one of <1> through <6> above, the method comprising the steps of:

(1) identifying a cell cluster that is not present in the normal blood as a cancer cell candidate in the blood and selectively recovering the cancer cell candidate;

(2) identifying a multinucleated cell that is not present in the normal blood as a cancer cell candidate in the blood and selectively recovering the cancer cell candidate;

(3) identifying a cytomegalic cell that is not present in the normal blood as a cancer cell candidate in the blood and selectively recovering the cancer cell candidate; and/or

(4) identifying a cell as a cancer cell candidate based on a combination of the step of (1), (2) or (3) and an analysis result that a fluorescent antibody exhibits a fluorescence intensity to one or a plurality of biomarkers for cancer cells, and selectively recovering the cancer cell candidate.

<8> The method according to <7> above, wherein the fluorescent antibody is an EpCam antibody, a K-ras antibody or a cytokeratin antibody.

<9> The method according to <7> or <8> above, wherein:

in the step (1), the identification is performed based on that R > 1.3 in the bright-field image, or based on the size of the cell in the bright-field image and the number and distribution of nuclei in the fluorescence image (i.e., based on that the distance between the centers of gravity of a plurality of adjacent nuclei is 3 $\mu$m or longer);

in the step (2), the identification is performed based on that R < 1.3 in the bright-field image, and based on the number and distribution of nuclei (i.e., based on that the distance between the centers of gravity of a plurality of adjacent nuclei is within 3 $\mu$m);

in the step of (3), the identification is performed based on that R < 1.3 in the bright-field image, and based on that the size of the cell exceeds 20 $\mu$m when being converted into the diameter; or

in the step of (4), a cell fulfilling at least one of the conditions of (1) through (3) is determined as a cancer cell.

<10> An optical module usable in an optical bright-field/fluorescence microscopic system, the optical module comprising:

a first dichroic mirror having an angle adjustment function and thus being capable of fine-adjusting a light reflection direction three-dimensionally;

a filter system into which light having image data and reflected by the dichroic mirror is introduced;

an image size adjustment system, which is formed of a movable light-blocking plate that adjusts an image size, the light that has passed the filter system being introduced into the image size adjustment system;

a second dichroic mirror having an angle adjustment function and thus being capable of fine-adjusting a light reflection direction three-dimensionally, the light that has passed the image size adjustment system being introduced into the second dichroic mirror; and

an optical lens system that compensates for a difference in image forming position, the light that has passed the second dichroic mirror being introduced into the optical lens system;

wherein image enlargement and image reduction can be performed by the optical lens system, so that an image including a bright-field image and a fluorescence image formed at different magnification ratios is generated.

<11> The optical module according to <10> above, which is usable to acquire, at the same time, a bright-field image of a fluorescence-stained cell contained in a sample solution, a fluorescence intensity of a fluorescence labeling substance bonded to the cell, and a fluorescence image of the cell.

The present invention further provides the following on-chip cell sorter system.

An on-chip cell sorter system, comprising:

(1) a cell sorter chip including a flow path in which a sample solution containing fluorescence-stained cells derived from a test subject flows;

an optical system including a bright-field light source and one or at least two fluorescence sources that emit light toward the cells, optical fibers that respectively transmit light of a plurality of wavelengths, and a light-collecting lens that converges light to an observation target at a position irradiated with the light;

a first detection system including optical fiber(s) respectively corresponding to one or at least two fluorescence wavelengths and transmitting fluorescence for detecting a fluorescence intensity of each of the cells in the sample solution flowing in the flow path of the cell sorter chip, a bandpass filter that is located on a stage after the optical fiber(s) and allows transmission of fluorescence of a particular wavelength, and a fluorescence detector, wherein the first detection system acquires, at the same time, a fluorescence intensity of a fluorescence labeling substance bonded to each of the cells, the fluorescence intensity corresponding to each of the one or at least two fluorescence wavelengths;

a second detection system that acquires a bright-field image of each of the cells and a fluorescence image of the cell at the same time;

control/analysis means that controls an operation of each of the systems and identifies a multinucleated cell and/or a cell cluster flowing in the flow path based on the bright-field image, the fluorescence intensity and the fluorescence image; and

means that selectively recovers the identified multinucleated cell and/or cell cluster.

ADVANTAGEOUS EFFECTS OF INVENTION

According to the present invention, a trace amount of target cells in the blood can be purified in units of one cell, and gene information and expression information of the target cells can be analyzed correctly.

According to the present invention, it can be identified whether the target cells are clustered or not (whether the target cells are solitary cells or not).

According to the present invention, it can be determined whether the cells are in an apoptosis state or not.

According to the present invention, it is made possible to separate, purify and recover only the target cells in real time.

According to the present invention, it is made possible to measure only the recovered cells regarding the inner state thereof at a one-cell level and to perform genome analysis/expression analysis on only the recovered cells at a one-cell level.

According to the present invention, it is made possible to re-culture only the recovered cells.

According to the present invention, detailed cell information such as the difference in the cell size, the size ratio between the nucleus inside the cell and the cytoplasm, or the like can be acquired, and the cells can be distinguished and purified based on such information.

According to the present invention, a cell such as a spore of Bacillus anthracis or the like can be analyzed regarding the substance therein at high speed while contamination is minimized.

According to the present invention, cells in a division period in the blood are recovered, and thus cells having a cell division ability such as cancer cells in the blood or stem cells can be recovered.

According to the present invention, multinucleated cells and cell clusters, which are candidates for a cancer cell circulating in the blood, can be effectively recovered.

According to the present invention, it is made possible to excite, at the same time, cells labeled with fluorescence antibodies of a plurality of wavelengths by excitation light of a plurality of wavelengths, and to detect the plurality of emitted fluorescence components at the same time. Thus, cells which may be targets can be effectively recovered.

BRIEF DESCRIPTION OF DRAWINGS

[FIG. 1] FIG. 1 is a schematic view conceptually showing an overall process of cell analysis performed by use of a cell analysis device system according to the present invention, and an example of means in the device corresponding to each of steps.

[FIG. 2] FIG. 2 schematically shows an example of overall structure of the cell analysis device system according to the present invention shown in FIG. 1.

[FIG. 3] FIG. 3 schematically shows an example of structure of a cell enrichment/staining/decoloration module shown in FIG. 2.

[FIG. 4] FIG. 4 schematically shows an example of structure of an image-detecting one-cell separation/purification (cell sorter) module shown in FIG. 2.

[FIG. 5] FIG. 5 schematically shows an example of structure of a one-cell genome analysis/expression analysis module shown in FIG. 2.

[FIG. 6] FIG. 6 schematically shows an example of structure of a liquid transfer module shown in FIG. 2.

[FIG. 7] FIG. 7 is a schematic view conceptually showing an overall process of cell analysis including a cell fracturing step performed by use of a cell analysis device system according to the present invention, and an example of means in the device corresponding to each of steps.

[FIG. 8] FIG. 8 schematically shows an example of fracture mechanism including a vessel, a rotator and a rotation shaft, which is used in the cell fracturing step among the steps shown in FIG. 7.

[FIG. 9] FIG. 9 schematically shows variations of cell fracture mechanism shown in FIG. 8; specifically a mechanism securing the contact closeness between the vessel and the rotator.

[FIG. 10] FIG. 10 provides schematic views showing various examples of the rotator and the rotation shaft in the cell fracture mechanism used in the present invention.

[FIG. 11] FIG. 11 schematically shows all the steps of sample fracturing in the cell fracturing step according to the present invention.

[FIG. 12] FIG. 12 provides conceptual views showing a structure for fracturing cells automatically and continuously in the cell fracturing step according to the present invention.

[FIG. 13] FIG. 13 schematically shows an example of the chip structure of a cell sorter module according to the present invention.

[FIG. 14] FIG. 14 schematically shows an example of the chip structure of a sample reservoir area of a cell sorter module according to the present invention.

[FIG. 15] FIG. 15 schematically shows an example of the structure of an image-detecting one-cell separation/purification (cell sorter) module.

[FIG. 16] FIG. 16 schematically shows an example of a cell purification process performed by the image-detecting one-cell separation/purification (cell sorter) module shown in FIG. 15.

[FIG. 17] FIG. 17 schematically shows an image recognition in a nucleus extinction process at the time of cell division, which is one of indices of identification used for cell purification performed by the image-detecting one-cell separation/purification (cell sorter) module shown in FIG. 15.

[FIG. 18] FIG. 18 schematically shows an example of light emission timing, for a high-speed flash light source in an image-detecting one-cell separation/purification (cell sorter) module, by which image blur is prevented.

[FIG. 19A] FIG. 19A schematically shows an example of the structure of an optical system, in an image-detecting one-cell separation/purification (cell sorter) module, that is provided for preventing image blur (FIG. 19A); and FIG. 19B shows images of a particulate observed by a conventional optical system, which provided in comparison with FIG. 19C showing images of the particulate observed by the optical system according to the present invention.

[FIG. 19B] FIG. 19A schematically shows an example of the structure of an optical system, in an image-detecting one-cell separation/purification (cell sorter) module, that is provided for preventing image blur (FIG. 19A); and FIG. 19B shows images of a particulate observed by a conventional optical system, which provided in comparison with FIG. 19C showing images of the particulate observed by the optical system according to the present invention.

[FIG. 19C] FIG. 19A schematically shows an example of the structure of an optical system, in an image-detecting one-cell separation/purification (cell sorter) module, that is provided for preventing image blur (FIG. 19A); and FIG. 19B shows images of a particulate observed by a conventional optical system, which provided in comparison with FIG. 19C showing images of the particulate observed by the optical system according to the present invention.

[FIG. 20] FIG. 20 schematically shows an example of a structure, in which a cell sorter chip is located such that a liquid flows vertically.

[FIG. 21] FIG. 21 schematically shows an example of a structure, in which a cell sorter chip is located such that a liquid flows vertically.

[FIG. 22] FIG. 22 schematically shows an example of the structure of an area where a sample solution and a buffer solution are joined together in a cell sorter chip.

[FIG. 23] FIG. 23 schematically shows an example of structure of a chip, in a cell sorter system, that includes a cell sorting mechanism.

[FIG. 24] FIG. 24 shows an example of an electrode arrangement for providing an external force in a flow path of a cell sorter system.

[FIG. 25] FIG. 25 shows examples of an electrode arrangement for providing an external force in a flow path of a cell sorter system.

[FIG. 26] FIG. 26 is a schematic view showing an example of cell purification process performed in a flow path of a cell sorter system.

[FIG. 27] FIG. 27 provides schematic views showing an example of gel electrode arrangement for providing an external electric field in a flow path of a cell sorter system.

[FIG. 28] FIG. 28 is a schematic view showing an example of identification process performed to separate cardiac muscle cells and fibroblasts from each other by use of an image in an image-recognizing cell sorter system.

[FIG. 29] FIG. 29 is a schematic view showing an example of the structure of a cell sorter system using a combination of water and oil.

[FIG. 30] FIG. 30 provides schematic views showing an example of structure of an area, in which water and oil are joined together in a cell sorter system using a combination of water and oil.

[FIG. 31] FIG. 31 is a graph showing the relationship between the amount of electrolyte (electroconductivity) in an aqueous solution and the possible cell separation velocity.

[FIG. 32] FIG. 32 schematically shows an example of the structure of an analysis system that performs measurement of the fluorescence intensity and acquisition of a high-speed bright-field microscopic image at the same time.

[FIG. 33] FIG. 33 schematically shows a specific example of structure of an analysis system shown in FIG. 32 that performs measurement of the fluorescence intensity and acquisition of a high-speed bright-field microscopic image at the same time.

[FIG. 34] FIG. 34 schematically shows an example of the structure of an analysis system that performs measurement of the fluorescence intensity, acquisition of a high-speed bright-field microscopic image and acquisition of high-speed fluorescence microscopic images at the same time.

[FIG. 35] FIG. 35 schematically shows an example of the structure of a device that acquires bright-field microscopic images and fluorescence microscopic images at the same time at a light receiving surface of one high-speed camera.

[FIG. 36] FIG. 36 schematically shows an example of a high-speed bright-field microscopic image and high-speed fluorescence microscopic image acquired at the same time by one high-speed camera and an example of analysis information.

[FIG. 37] FIG. 37 shows an example of images of a high-speed bright-field microscopic image and a high-speed fluorescence microscopic image of a stained nucleus that are acquired at the same time at the light receiving surface of one high-speed camera.

[FIG. 38] FIG. 38 schematically shows an example of device structure, by which a plurality of wavelengths of fluorescence excitation light are directed at the same time toward cells by use of an optical fiber array, the fluorescence amounts of the plurality of emitted wavelengths are found at the same time, and fluorescence images of the plurality of wavelengths are acquired at the same time.

[FIG. 39] FIG. 39 provides spectra schematically showing an example of selected set of a plurality of wavelengths of fluorescence excitation light that are to be directed toward cells and a plurality of wavelengths of fluorescence to be detected.

[FIG. 40] FIG. 40 schematically shows a structure in a particular example, an example of which is shown in FIG. 38.

DESCRIPTION OF EMBODIMENTS

A cell analysis device according to the present invention generally includes:

(1) a cell enrichment/staining/decoloration section that continuously performs a process including cell enrichment, staining with fluorescent antibody labeling (or, in the case where re-culturing is to be performed, with a reversible fluorescence labeling marker such as an aptamer or the like when necessary) and washing;

(2) an image-detecting one-cell separation/purification (cell sorter) section that acquires image data on a cell image at a rate of about 10,000 images/sec from cells flowing in a micro-flow path formed in a chip substrate, and purifies 10,000 cells per second in real time based on an analysis result on the image information;

(3) a one-cell genome analysis/expression analysis section that measures an inner state of the cells at a one-cell level;

(4) a liquid transfer section that transfers a sample solution between the sections; and

(5) a control/analysis section that controls an operation of each of the sections and performs the above-described analysis.

In a typical embodiment of the cell analysis device according to the present invention, the above-described three modules (1) through (3) are combined and continuously operated in the above-described order. Since the cells are continuously transferred by the flow path, extinction of a part of a trace amount of cells due to contamination or operation can be minimized.

By use of the cell analysis device according to the present invention, it is detected and checked whether cells are fluorescence-labeled or not on a one-cell level. It is confirmed that the fluorescence-labeled cells are solitary cells that are not clustered. It can also be determined whether the cells are in an apoptotic state or not. Therefore, the cell analysis device according to the present invention can separate and purify cells based on indices, which are not usable for identification by the conventional scattered light-detecting cell sorter technology.

With the cell analysis device according to the present invention, stained cells can be selected and recovered correctly in units of one cell. The state of each of the cells to be recovered, for example, whether the cell is in an apoptotic state or not, can be checked. Gene information/expression information of each cell can be analyzed together with the fluorescence information and the cell state information.

The cell enrichment/staining/decoloration section in (1) above is operated as follows. Cells in a trace amount contained in a reaction solution that is transferred from the module on the immediately previous stage by a non-contact force are continuously caught and enriched. When a certain number of cells are caught, a cell-labeling staining liquid is introduced to stain the cells. After the cells are stained, the reagent that is not bonded to the cells is removed by washing. Then, the cells are transmitted to the next module at a certain concentration. The cell enrichment/staining/decoloration section uses, for example, a cell catching/enrichment technology that utilizes a feature of cells that cells are gathered by a "dielectric electrophoretic force", which is a non-contact force generated by an AC electric field applied by a metal electrode formed in a microscopic flow path.

The means in (2) that performs cell separation/purification in units of one cell based on the image detection results is operated as follows. Detailed information on the cells such as the difference in the cell size, the size ratio between the nucleus inside the cell and the cytoplasm, or the like is acquired as image information, and the cells are purified based on such information. For acquiring an image, a high-speed camera is used. Light emission from a light source is adjusted so as to be suitable to the shutter cycle of the high-speed camera, so that light is emitted from the light source only for a certain time duration among the time period, in which the shutter is clicked. In the case where, for example, the shutter speed is 1/10,000 sec., a target cell is irradiated with light from a light source that is capable of controlling high-speed light emission, such as an LED light source, a pulsed laser light source or the like, for only 1/10 of the shutter speed. Thus, a precise shape of the cell can be acquired.

A conventional cell sorter structured as the above-described separation/purification means on a chip has the following problem. With the conventional cell sorter, a cell to be introduced into the cell sorter is separately enriched by an enrichment step by use of a centrifuge or the like. Therefore, the cell may be contaminated during the step. According to the present invention, the steps are performed on a chip in a closed state except for the steps performed by an optical system. Namely, cell enrichment is performed directly on a chip, and a liquid transfer section and a culture tank for separated cells are also formed on a chip. Owing to this structure, contamination or loss of cells does not occur, and also the procedure is simplified and the process time is shortened. Thus, the device is made easier to use. Since the steps are performed in a closed state, it is made unnecessary to be concerned with contamination also in another case where it is indispensable to prevent contamination of cells derived from a test subject tissue, for example, in separation of stem cells, clinical examinations or the like. According to the present invention, main parts of the cell sorter are put into a chip, and thus complete prevention of cross-contamination of devices or the like is realized. The present invention provides a cross-contamination-free cell separation system, which is indispensable in the medical field, especially in the field of degenerative medicine.

The cells assumed in the present invention range from small cells such as bacteria to large animal cells (e.g., cancer cells) or the like. Thus, the cell size is typically in the range of about 0.5 $\mu$m to 30 $\mu$m. In order to perform cell enrichment and cell separation continuously by use of a flow path that is formed in one surface of the substrate and has both of a cell enrichment function and a cell separation function, a first issue to consider is the flow path width (cross-sectional shape). The flow path is formed in one surface of a substrate to have a thickness of, typically, about 10 to 100 $\mu$m substantially two-dimensionally. An appropriate thickness is about 5 to about 10 $\mu$m for bacteria and is about 10 to about 50 $\mu$m for animal cells.

A cell analysis device according to the present invention typically includes, in one chip, a cell enrichment section having a function of enriching cells, a cell arraying section and a cell separation/purification section having a function of separating and purifying cells after the cell enrichment, and an optical analysis section that identifies and distinguishes cells to be separated and purified. Typically, a sample solution which has not been enriched is introduced into the cell enrichment section from one entrance thereof, and is discharged from a discharge section located in a downstream part of the cell enrichment section. In addition to such basic elements, the device may include means that apply an external force to the cells so as to enrich the cells and direct the cells toward an enriched cell recovery opening located in a side wall of the enrichment section. The external force may be an ultrasonic radiation pressure, a gravitational force, an electrostatic force or a dielectric electrophoretic force, but is not limited to these. In this case, the means that apply an external force are located at a position, at which an external force can be applied in a direction that is perpendicular to the flow of the sample solution in the enrichment section and toward the enriched cell recovery opening.

In the cell separation/purification section, the cells flowing in the flow path are supplied with an external force so as to be arrayed at a center of the flow path, so that all the cells can be transferred downstream to one of the two branched flow paths. Among the arrayed cells, only the cells to be recovered are further supplied with an external force to change the position of the cells. In this manner, only the cells provided with the further external force are introduced into one of the two branched flow paths. A specific external force may be applied by cell arraying means that arrays the cells to nodes of a stationary wave by an ultrasonic radiation pressure. Alternatively, an array of wedge-like electrodes may be combined, so that the cells can be arrayed at the positions of the apexes of the wedges. Still alternatively, means that arrays the cells using a pair of moustache-like electrodes may be used such that the cells are arrayed between the pair of electrodes. Such means allows the cells to be arrayed in a straight line with no need to add a side sheath liquid. Therefore, one of the problems to be solved by the present invention described above, namely, the problem that the cell solution enriched in the previous stage is diluted, can be solved.

The cell detection function of the cell analysis device according to the present invention is provided by the image-detecting one-cell separation/purification section in (2). In the case where cells are to be recognized as an image and evaluated, a CCD camera for observation is set upstream of the flow path branch point, and a cell separation area is provided downstream with respect thereto when necessary. In the case where no image is used and laser light or the like is directed toward cells flowing in the flow path and light scattered when the cells cross the light or the cells are modified with fluorescence, the fluorescence can be detected by a light detector. Also in this case, the flow path branch point, which is the cell separation area, is set downstream of the detection section.

In the case where the cells are to be separated in a sorting section, which is the cell separation area, an external force is applied to the cells in the cell sorting section for example as follows. In the case where a dielectric electrophoretic force is used, a pair of comb-like electrodes are provided to form a flow path where the cell can be separated and discharged. In the case where an electrostatic force is used, a voltage is applied to the electrodes to change the position of the cells in the flow path. At this point, the cells are generally charged negative and therefore are moved toward a positive electrode.

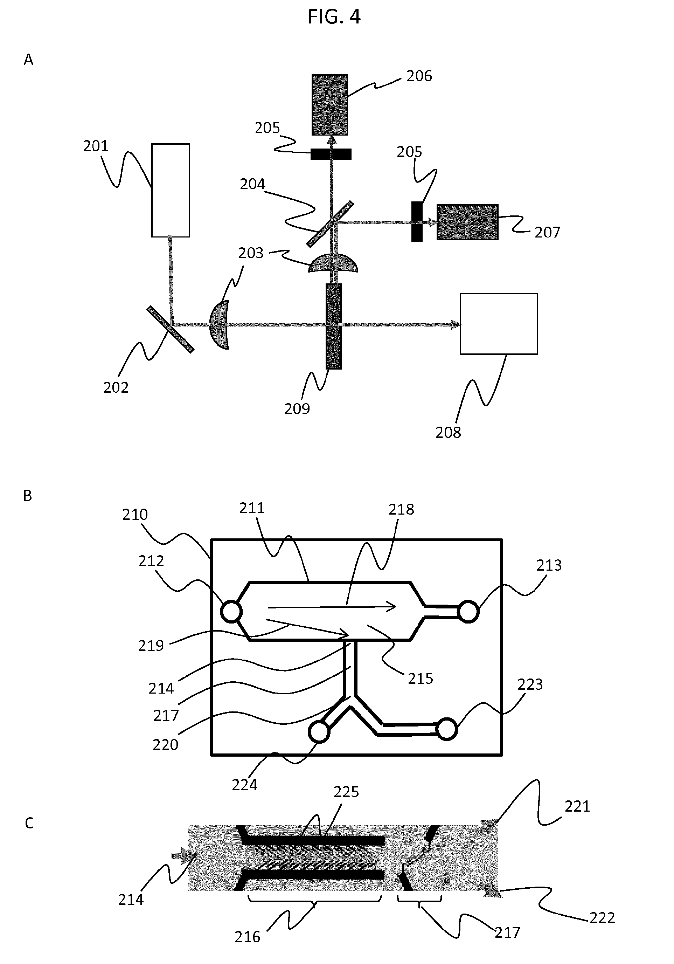

According to the present invention the pressure, by which a sample solution is introduced into the chip, is a driving force for transferring the solution. Therefore, it is desirable that a waste liquid exit (outlet 213) of a cell enrichment section 215, a purified cell exit (cell recovery section 224) of a cell sorting section 217, and a waste liquid exit (waste liquid recovery section 223) of the cell sorting section are structured to have substantially the same pressure (see FIG. 4B). For this purpose, a flow path resistance adjustment section for pressure adjustment is provided immediately before an exit of each of thin flow paths and S-shaped long flow paths.

An algorithm of cell recognition and separation has the following features.

When cells are to be recognized from an image and evaluated, a part of the flow path that is downstream of the joining point of flow paths is observed by a CCD camera, and measurement is performed on a planar range. Thus, the cells are identified by image recognition and traced. In this manner, the cells can be separated with certainty. What is important in this case is the image capturing rate. With a common camera having a video rate of 30 frame/sec., a part of the cells is not recognized in the image. With a capturing rate of at least 200 frames/sec., cells flowing at a relatively high speed in the flow path can be recognized.

Now, image processing methods will be discussed. When the capturing rate is high, highly complicated image processing cannot be performed. The state of cell recognition varies in accordance with the transfer rate or the type of the cells as described above. In some cases, some cells run past the other cells. Therefore, when each cell first appears in the image frame, the cell is numbered. The cell is managed by the same number until disappearing from the image frame. Namely, the cell images transferring through a plurality of continuous frames are managed by numbers. The cells are linked in different frames under the conditions that in each frame, the cells are transferred from an upstream area to a downstream area and that the transfer rate of a specifically numbered cell that is recognized in the image is within a certain range, the starting point being the cell's first appearance in a frame. In this manner, even if some cells run past the other cells, each cell can be traced with certainty.

The cell can be recognized as described above. The cells are each numbered as follows. First, the cell image is binarized, and the center of gravity thereof is found. The luminance centroid, the surface area, the perimeter length, the longer diameter and the shorter diameter of the binarized cell image are found, and the cell is numbered by use of these parameters. Each cell image is automatically stored as an image at this point, which is useful for the user.

Now, cell separation will be discussed. It is required to separate only particular cells among the numbered cells. An index for separation may be the information such as the luminance centroid, the surface area, the perimeter length, the longer diameter, the shorter diameter or the like as described above. Alternatively, fluorescence detection may be performed in addition to the above-described process on the image, and information obtained by use of the fluorescence may be used as an index for separation. In any way, the cells obtained by the detection section are separated in accordance with the numbers thereof. Specifically, based on the image captured at a predetermined time duration, the transfer rates (V) of the numbered cells are calculated. Where the distance from the detection section to the sorting section is (L) and the voltage application time is (T), the voltage application timing is set to (L/V) to (L/V+T). In this manner, when cells of target numbers are between the electrodes, the cells are electrically sorted in accordance with the voltage application time (T) and thus separated.

The high-speed one-cell genome analysis/expression analysis means in (3) used in the present invention has the following structure for achieving the above-described object. For example, for changing the temperature of a sample liquid to a plurality of temperatures, the reaction control device to be used includes means that use, as mediums of heat exchange, liquids that have a large heat capacity and are respectively kept at the plurality of temperatures, to which the temperature of the sample liquid is to be changed. The means change the liquids having a large heat capacity and a plurality of different temperatures at high speed. The reaction control device also includes a microscopic reaction tank, in which the heat exchange between the liquids having a large heat capacity and the sample liquid is rapidly performed. Specifically, a reaction control device used in the present invention includes a microscopic reaction tank formed of a structure and a material suitable to heat exchange, a reaction tank heat exchange tank that allows a liquid of a suitable temperature to each reaction to be circulated outside the microscopic reaction tank, a plurality of liquid reservoir tanks containing a heat source that maintains the temperature of a liquid with high precision, a switching valve system that guides a liquid from any liquid reservoir tank to the outside of the reaction tank in order to change the temperature in the microscopic reaction tank rapidly, and a mixture preventing mechanism that prevents mixture of liquids of different temperatures at the time of switching performed by the valve system.

Controlling the temperature of the reaction tank by use of circulating liquids has the following advantages. First, the problem of temperature overshoot can be solved. The temperature of a liquid kept circulating is constant. Therefore, the temperature of the surface of the reaction tank and the temperature of the liquid are equilibrated instantaneously. The heat capacities of the reaction tank and the sample are negligibly small as compared with that of the refluxing liquid. Therefore, even if a liquid is deprived of heat locally, a heat gradient does not basically occur because the liquid is kept flowing. Needless to say, the temperature of the reaction tank never exceeds the temperature of the liquid. By injecting liquids of different temperatures to the reaction tank heat exchange tank one after another, a temperature change of 30 degrees or greater can be caused within 0.5 sec.

Hereinafter, embodiments of the present invention will be described in more detail with reference to the drawings. These embodiments are merely illustrative and the present invention is not limited to these embodiments.

(Structure of the cell analysis device)

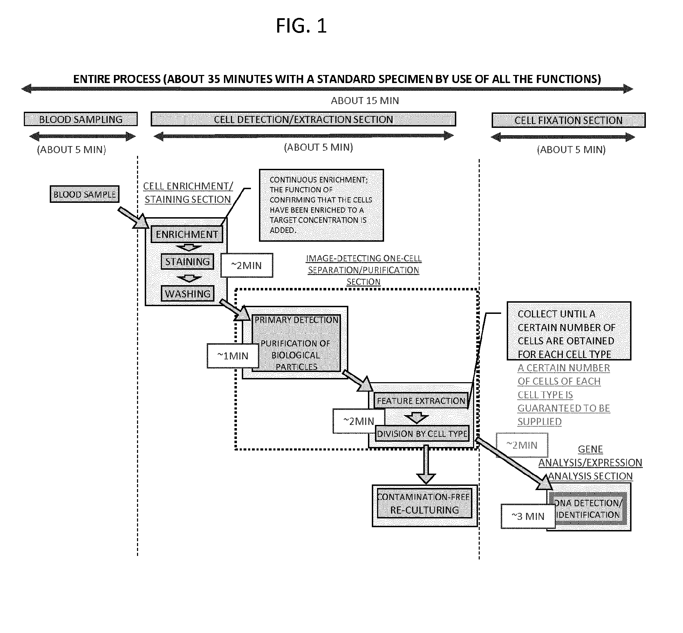

FIG. 1 shows an example of procedure, performed by use of a cell analysis device according to the present invention, from sampling of a blood sample to analysis.

A blood sample sampled from a patient is introduced into a cell enrichment/staining section. Only a cell component is extracted from the blood. A fluorescent labeling agent such as a fluorescent cancer marker or the like is added to be reacted with the sample cell. Then, an excessive portion of the fluorescent labeling agent that was not reacted is removed by washing, so that the reaction product is adjusted to be a solution having a cell concentration optimal for an image-detecting one-cell separation/purification section on the next subsequent stage. Then, the solution is introduced into the image-detecting one-cell separation/purification section.

Next, in the image-detecting one-cell separation/purification section, it is checked, as primary detection, whether there is fluorescence emission or not, based on fluorescent labeling at a one-cell level. In this manner, it can be checked whether the cells can be target cells or not by use of a conventional labeling technology. Then, cells that emit fluorescence and thus can be target cells are imaged by a high-speed camera. The resultant image is analyzed in real time to determine (1) whether the cells emitting fluorescence are each a solitary cell or in a cell mass together with other cells, or (2) whether the cells emitting fluorescence are in a normal state or in, for example, an apoptosis state, in which the nucleus of the cell and the shape of the cell are deformed. Thus, in accordance with the purpose, the normal cells or the cells in an apoptosis state can be recovered and introduced into a gene analysis/expression analysis section on the next stage that is capable of performing analysis on even a trace amount of sample at high speed. Then, each form of cells can be subjected to gene analysis and expression analysis separately. In the case where the cells are in a cell mass, the cell mass is not recovered even when containing cells emitting fluorescence because cells other than the target cells are also contained.

The cells identified and purified at this stage can be re-cultured in a contamination-free state in units of a purified cell, instead of being introduced into the gene analysis/expression analysis section.

The gene analysis/expression analysis section performs gene identification or expression identification on the introduced cells in units of a small number of cells, namely, in units of one cell or in units of a group of the same cells, in which the cells are determined to be the same cells based on information obtained from the image-detecting one-cell separation/purification section.

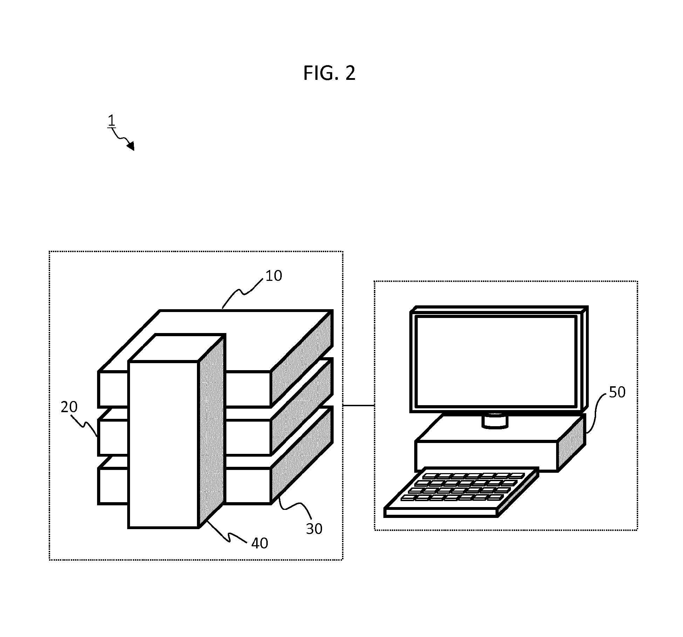

FIG. 2 shows an example of overall structure of a cell analysis device system 1 that realizes the procedure shown in FIG. 1. The device system 1 includes an enrichment/staining/decoloration module 10 that performs a pre-process on cells in a blood sample introduced thereto, an image-detecting one-cell separation/purification module 20 that identifies and purifies each of the cells, a one-cell genome analysis/expression analysis module 30 that performs gene analysis or expression analysis on the purified cells, a liquid transfer module 40 that transfers the sample between the modules, and a control/analysis module (computer) 50 that analyses the analysis results.

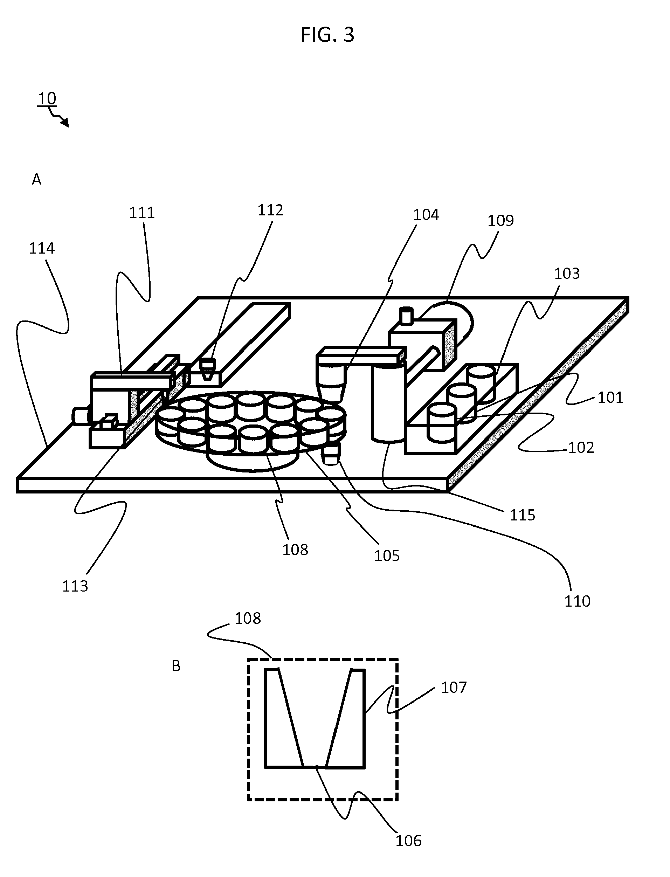

FIG. 3 through FIG. 6 each show an example of structure of each of the modules shown in the example in FIG. 2.

First, FIG. 3 shows an example of structure of the cell enrichment/staining/decoloration module 10 that performs a pre-process on cells in a blood sample derived from a subject (e.g., cancer patient) introduced thereto. In the example in FIG. 3, the cell enrichment/staining/decoloration module 10 is located on a chassis 114 integrally therewith. The module includes vessels or reservoirs (101, 102, 103) that respectively hold solutions of sample cell sample, staining agent, and washing detergent. The solutions can be each introduced via a separation head 104 attached to a rotatable arm 115 into a chamber 107 (a plurality of chambers 107 are referred to collectively as a enrichment chamber 108). The chambers 107 are located on a turn table 105 and have a enrichment/decoloration filter 106 attached to a bottom surface thereof. First, a sample cell sample such as blood or the like is introduced into the enrichment chamber 108. A liquid component thereof is discharged via the filter to a waste liquid recovery tube 110 by a pressure pump 109. In this manner, cell enrichment is performed. Next, a staining liquid is introduced by the use of the separation head 104. After a reaction is continued for a certain time duration, the staining liquid is also discharged by the pressure pump 109. Next, a decoloration agent is introduced into the enrichment chamber 108, and thus an excessive portion of the staining agent is washed and discharged. Then, a diluent generally also acting as a washing detergent is introduced to dilute the cells so that the cell solution has a desired concentration, and the cell solution is introduced into a recovery tube 112 via a recovery head 111 having a recovery chip 113 at a tip thereof.

FIG. 4 shows an example of structure of the image-detecting one-cell separation/purification module 20 that identifies and purifies each cell. As shown in FIG. 4A, the image-detecting one-cell separation/purification module 20 includes an optical system including a light source 201, a mirror 202, a light collection lens 203, a dichroic mirror 204, a filter 205, a light detection element 206 for detecting fluorescence, a high-speed camera 207 and a photodiode 208 for detecting scattered light; and also includes a cell sorter chip 209, into which a cell sample is introduced. In the module shown in FIG. 4A, a plurality of pieces of information can be detected at the same time on the cells passing the cell sorter chip 209 by use of the light source 201 such as a pulse laser, a high-luminance LED light source or the like, the light detection element 208 such as a photodiode or the like that detects passage of the cells based on scattered light, the high-sensitivity light detection element 206 such as a photomultiplier or the light that detects fluorescence, the high-speed camera 207 and the like. The light source may generate light continuously. However, in order to produce an image having a higher spatial resolution with no blur, the light source can generate pulsed light in synchronization with the shutter cycle of the high-speed camera 207. In this manner, an image having a higher temporal resolution can be obtained with a shorter time of light radiation.

A process using an image and a process using fluorescence or scattered light may be combined, needless to say. Image data obtained by the high-speed camera 207 can be displayed on a monitor of the computer 50 so that a user can view the image. In the case where there are a plurality of types of fluorescence to be observed, a plurality of assemblies each including a part of the optical system from the dichroic mirror 204 via the filter 205 to the fluorescence detector 206 may be combined. The number of the assemblies are set in accordance with the number of types of fluorescence to be observed. In such an assembly, the filter 205 is appropriately adjusted so that a plurality of types of excitation light is transmitted through the filter 205, and the cells are irradiated with light of a wavelength that is different from the wavelength of the fluorescence to be detected on a later stage. When such a structure is used, fluorescence observation results on a cell image can be used as data.

An algorithm of cell recognition and separation has the following features.

When cells are to be recognized as an image and evaluated, a part of the flow path that is downstream of the joining point of a plurality of flow paths is observed by a CCD camera, and measurement is performed on a planar range. Thus, the cells are identified by image recognition and traced. In this manner, the cells can be separated with certainty. What is important in this case is the image capturing rate. With a common camera having a video rate of 30 frame/sec., a part of the cells is not recognized in the image. With a capturing rate of at least 200 frames/sec., cells flowing at a relatively high speed in the flow path can be recognized.

Now, image processing methods will be discussed. When the capturing rate is high, highly complicated image processing cannot be performed. The state of cell recognition varies in accordance with the transfer rate or the type of the cells as described above. In some cases, some cells run past the other cells. Therefore, when each cell first appears in the image frame, the cell is numbered. The cell is managed by the same number until disappearing from the image frame. Namely, the cell images transferring through a plurality of continuous frames are managed by numbers. The cells are linked in different frames under conditions that in each frame, the cells are transferred from an upstream area to a downstream area and that the transfer rate of a specifically numbered cell that is recognized in the image is within a certain range the starting point being the cell's first appearance in a frame. In this manner, even if some cells run past the other cells, each cell can be traced with certainty.

The cell can be recognized as described above. The cells are each numbered as follows. First, the cell image is binarized, and the center of gravity thereof is found. The luminance centroid, the surface area, the perimeter length, the longer diameter and the shorter diameter of the binarized cell image are found, and the cell is numbered by use of these parameters. Each cell image is automatically stored as an image at this point, which is useful for the user.

Now, cell separation will be discussed. It is required to separate only particular cells among the numbered cells. An index for separation may be the information such as the luminance centroid, the surface area, the perimeter length, the longer diameter, the shorter diameter or the like as described above. Alternatively, fluorescence detection may be performed in addition to the above-described process on the image, and information obtained by use of the fluorescence may be used as an index for separation. In any way, the cells obtained by the detection section are separated in accordance with the numbers thereof. Specifically, based on the image extracted at a predetermined time duration, the transfer rates (V) of the numbered cells are calculated. Where the distance from the detection section to a sorting section is (L) and the voltage application time is (T), the voltage application timing is set to (L/V) to (L/V+T). In this manner, when cells of target numbers are between the electrodes, the cells are electrically sorted in accordance with the voltage application time (T) and thus separated.

An example of structure for separation/purification of the cells is as follows. The structure includes a series of precision-processed flow paths, located two-dimensionally on a planar chip, in which the cells in the sample solution are enriched, arrayed and purified, and also includes means that causes a force to act on the cells incorporated into the chip.

The cell separation/purification module is provided on the chip. FIG. 4B schematically shows an example of the cell sorter chip 209 provided on the chip. A micro-flow path 211 is provided in the chip substrate 210, and an opening communicating to the flow path is provided in a top surface thereof. The opening acts as a supply opening for samples or necessary buffer solutions (mediums). The flow path may be formed by so-called injection molding of injecting a plastic material such as PMMA or the like into a die or by bonding a plurality of glass substrates. The size of the chip is, for example, 50 x 70 x 1 mm (t), but is not limited to this. In the case where a PMMA plastic material is used, for example, a 0.1 mm-thick laminate film is thermally pressure-contacted. In the case where glass is used, 0.1 mm-thick glass plates are optically adhered together. Owing to this, cells flowing in grooves or through-holes formed in an inner surface of the chip, or in the flow paths or wells, can be observed by an optical microscope of a high magnification. For example, the cells flowing in the flow paths can be observed by use of an objective lens having a numerical aperture of 1.4 and a magnification of 100 through a 0.1 mm-thick laminate film. In the case where a highly light-transmissive plastic material is used, the cells can be observed even from above the chip substrate 210. The cells assumed in the present invention range from small cells such as bacteria to large animal cells such as cancer cells or the like. Thus, the cell size is typically in the range of about 0.5 $\mu$m to 30 $\mu$m, but is not strictly limited to this range. Any size of cells are usable as long as the present invention is effectively usable. In order to perform cell enrichment and cell separation continuously by use of a flow path formed in one surface of the substrate, a first issue to consider is the flow path width (cross-sectional shape). The flow path 211 is formed in one surface of the substrate to have a thickness of, typically, about 10 to 100 $\mu$m substantially two-dimensionally. An appropriate thickness is 5 to 10 $\mu$m for bacteria and is 10 to 50 $\mu$m for animal cells.

On the chip 209, the sample solution is first introduced from an inlet 212 into the micro-flow path 211 by a syringe pump or cell introduction means that does not generate a pulsating flow such as air pressure or the like. The cell-containing sample solution introduced into the micro-flow path 211 flows along a flow line 218 of pre-voltage application particles toward an outlet 213 on the downstream side, and is discharged. There are means provided that continuously apply an external force on the cells so as to enrich the cells and direct the cells in a direction toward a cell-enriched solution inlet 214 located in a part of a side wall of the micro-flow path 211. By the external force, the cells are enriched while advancing along a flow 219 of the post-voltage application cells. As a result, a cell-enriched solution, having a high concentration that is at least 100 times the concentration of the sample solution at the inlet 212, is introduced into the cell-enriched solution inlet 214.

Usable as the external force to be applied to the cells may be an ultrasonic radiation pressure, a gravitational force, an electrostatic force or a dielectric electrophoretic force. In the case where, for example, an ultrasonic radiation pressure is used, an ultrasonic wave is generated so as to advance toward the cell-enriched solution inlet 214 in a direction perpendicular to the flow of the sample solution. The flow 219 of the post-voltage application cells can be formed by the radiation pressure of the ultrasonic wave. The ultrasonic wave may be introduced by bonding a PZT-type piezoelectric element to the surface of the chip 209. According to another method for introducing the ultrasonic wave, an array of comb-like electrodes is located on a surface of a piezoelectric element and is bonded to a surface of a cell enrichment section 215, so that a surface acoustic wave is generated in the cell enrichment section 215. The ultrasonic wave effusing therefrom is introduced into the cell enrichment section 215. In the case where a gravitational force is used, the spatial location of the chip 209 may be adjusted such that the direction of the gravitational force is perpendicular to the flow of the sample solution and is toward the cell-enriched solution inlet 214. Alternatively, the chip 209 may be located on a rotatable discus such that the direction of the gravitational force is perpendicular to the flow of the sample solution and such that the direction toward the cell-enriched solution inlet is the same as the radial direction of the discus. In the case where an electrostatic force is used, electrodes are located on the side wall of the micro-flow path 211 so that the cells are supplied with the external force directed toward the side wall. In this case, which charge is to be applied may be determined based on whether the potential of the surface of each target cell is positive or negative. It should be noted that in the case where an electrostatic force is to be generated, when the potential of the surface of the electrode to be supplied with a current exceeds a certain potential such as a peroxide potential, a perhydroxide potential or the like, bubbles are generated from the electrode. When this occurs, the applied voltage is very weak. Therefore, the flow path distance of the micro-flow path 211 needs to be flexibly adjusted in accordance with the type and strength of the external force to be applied to the cells. In the case where, for example, the external force is an electrostatic force, the micro-flow path 211 needs to be sufficiently long. In the case where a dielectric electrophoretic force is used as an external force, electrodes may be located in the cell enrichment section 215 such that the direction of the dielectric electrophoretic force is perpendicular to the flow of the sample solution and is toward the cell-enriched solution inlet 214.

Next, as shown in FIG. 4C, cells in the cell-enriched solution introduced from the cell-enriched solution inlet 214 are arrayed in a line along the flow in a convergence section 216. Specifically, there are means provided that uses a dielectric electrophoretic force or a stationary wave of an ultrasonic radiation pressure to generate an external force such that the cells are attracted toward a central part of the flow in the convergence section 216. The cells arrayed in a line in this manner are measured in a cell detection area 218 that is on a stage before a cell sorting section 217, so that the type of each cell is determined. Then, each cell is guided to a first outlet 221 or a second outlet 222, which are in two branched downstream areas in a cell sorting/branching section 220. Whether the cell is supplied with an external force in a direction perpendicular to an upstream-to-downstream flow or not controls whether the cell is guided to the first outlet 221 or the second outlet 222.

An actual example of structure of the convergence section 216 will be described. In the case where a dielectric electrophoretic force is used as an external force, an example of an electrode arrangement is as follows. A pair of electrodes 225 having wedges (V-shaped comb-like electrodes for convergence) are located alternately, and an AC voltage is applied to electrode contacts of the V-shaped comb-like electrodes for convergence. Thus, an external force can be applied to the cells so that the cells are transferred toward apexes of the wedges. As a result, the cells can be continuously enriched at the positions of the apexes of the wedges. What is important for the electrodes in this example is the shape of the electrodes located in the flow path. The electrodes have a protrusion in a downstream direction and form an array of comb-like electrodes that are not straight but have acute tips and are axially symmetrical. Owing to such a shape of the electrodes, the cells supplied with the dielectric electrophoretic force are guided and arrayed by the tips of the electrodes by a resultant force of a force of pushing the cells downstream along the flow and a force applied to the cells in a direction toward the acute tips. This occurs regardless of whether the dielectric electrophoretic force acts on the cells as a repulsive force or an attractive force. In other words, the acute tips of the electrode array are located at a position where the cells in the flow path are to be enriched, and thus the cells are gathered at the acute tips by a resultant force of a force of pushing the cells downstream along the flow and the dielectric electrophoretic force toward the acute tips.

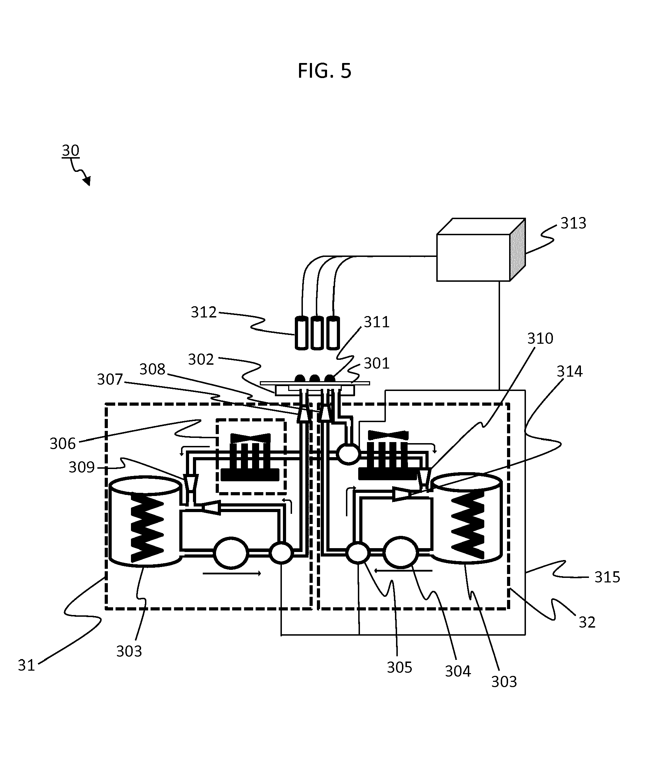

FIG. 5 shows an example of structure of the one-cell genome analysis/expression analysis module 30 that performs gene analysis or expression analysis on purified cells. A reaction tank 301 is formed of an aluminum, nickel or gold thin plate having a plurality of depressions. The plate has a thickness of about 10 to 30 micrometers in the depressions areas. An area between adjacent depressions has a thickness of 100 to 500 micrometers so as to be entirely strong with certainty. The reaction tank 301 is secured to a bottom surface of a quadrangular or circular reaction tank frame, so as to be easily detached from a reaction tank heat exchange tank 302. A liquid to be introduced into the reaction tank heat exchange tank 302 is excessively heated by a heat source located in each of liquid reservoir tanks 303. A stirring mechanism is prepared in order to remove heat rapidly from a surface of the heat source and thus to uniformize the temperature in the liquid reservoir tank 303. A liquid in the liquid reservoir tank 303 is guided through the flow path by a pump 304. The liquid is guided to the reaction tank heat exchange tank 302, or is guided to a bypass flow path and directly returns to the liquid reservoir tank 303, by a switching valve 305. When necessary, the temperature of the liquid is slightly controlled by an assisting temperature control mechanism 306, so that the temperature change in the liquid reservoir tank 303 is suppressed. The reaction tank heat exchange tank 302 basically includes an inlet A (307) and an inlet B (308) through which liquids of different temperatures are introduced. The number of the plurality of inlets matches the number of temperatures to which the temperature of a sample liquid is to be changed. In the case where, for example, a three-temperature system is to be formed, three inlets are prepared. The number of the inlets is not limited to two as in this example. In order to return the liquid in the reaction tank heat exchange tank 302 to the liquid reservoir tank 303, a plurality of outlets, namely, an outlet A (309) and an outlet B (310) are prepared. The number of the outlets is not limited to two. The reaction tank may have any of various shapes. For example, there may be a reaction tank A, a reaction tank B, a reaction tank C and a reaction tank D. The liquid in the reaction tank heat exchange tank 302 may be water, or alternatively may be a liquid having a large heat capacity and a low viscosity. For example, liquid ammonia or the like may be used. When the liquid in the reaction tank heat exchange tank 302 has a boiling point higher than that of water, the sample liquid can have a temperature of 100°C with certainty. When the liquid in the reaction tank heat exchange tank 302 has a freezing point lower than that of water, the liquid circulating in the device can be prevented from freezing while the temperature thereof is changed down to the freezing point of water with certainty.

The reaction tank frame has an optical window through which excitation light having a fluorescent dye and fluorescence are transmitted, so that the fluorescence intensity of the fluorescent dye in the sample liquid, which is changed by a reaction of a sample liquid 311 in the reaction tank 301, can be measured. Such measurement can be performed for one or each of the plurality of reaction tanks 301. A fluorescence detector 312 is also provided, so that the time-wise change in the measured fluorescence intensity of each reaction tank 301 can be measured. In the example in FIG. 5, a plurality of fluorescence detectors 312 each include an excitation light radiation mechanism and a fluorescence detection mechanism, so that different pieces of PCR amplification information on the plurality of reaction tanks 301 to which different primers or different sample liquids are dripped can be each measured independently. Fluorescence intensity data obtained by each fluorescence detector 312 is recorded by a control analysis section 313. The control analysis section 313 has a function of estimating the DNA amount or the mRMA amount in the sample liquid obtained by the PCR. The control analysis section 313 also has a function of acquiring switching information on the switching valve 305 and estimating, based on the time-wise change in the fluorescence intensity, whether or not the temperature of the post-valve switching sample liquid 311 has reached a target temperature, and also a mechanism of controlling the valve switching based on the estimation results. The estimation is performed based on the change of the amount of fluorescence intensity per unit time being decreased or nulled by utilization of that fluorescence extinction based on the motion of water molecules universally possessed by a fluorescent dye depends on the liquid temperature. This estimation is especially useful to check whether or not a high temperature state that denatures the DNA has been achieved.

In this example, one detector is located for each reaction tank 301. Alternatively, a fluorescence excitation light source may be combined with a camera such as a cooled CCD camera that can perform quantitative detection of fluorescence, so that the change in the fluorescence intensity of a plurality of reaction tanks can be measured. Alternatively, in the case where a number of detectors that is smaller than the number of the reaction tanks 301 are used, a mechanical driving mechanism that is movable at high speed on an X-Y plane may be combined with the detectors, so that the fluorescence intensity of all the reaction tanks can be measured.

It is preferable to lyophilize a reagent necessary for the reaction. A lyophilized reagent can be prepared in a bottom part of the reaction tank. Alternatively, a plug-like lyophilized reagent may be formed in a separation chip that is used to separate the sample, so that the reagent can be dissolved in the sample by moving the sample up and down. According to still another method, a lyophilized reagent is formed on a surface of a fiber ball formed of nylon fiber or the like, the fiber ball is inserted into the sample in the reaction tank, and the sample is stirred. In this manner, the lyophilized reagent can be dissolved.

It is inconvenient to directly handle the reaction tank 301 formed of a thin film. It is preferable to secure the reaction tank 301 to the reaction tank frame. Desirably, the reaction tank frame is formed of polystyrene, polycarbonate, PEEK, acrylic resin or the like, which is heat-insulating. It is also desirable that the surface area of the reaction tank frame which is bonded with the reaction tank 301 is suppressed low in order to raise or lower the temperature of the reaction tank 301 rapidly and highly precisely. According to a method for attaching the reaction tank 301 to the reaction tank heat exchange tank 302, a surface of the reaction tank frame is threaded and the reaction tube frame is screwed into the reaction tank heat exchange tank 302. It is desirable to attach a seal to the opening in order to keep the water tightness. Alternatively, a tapered reaction tank frame may be used so that the reaction tank 301 can be attached by use of only a pressure.

Now, a specific example of valve switching mechanism will be described. There are opened at the same time for merely a moment. Owing to this, liquids of different temperatures can be suppressed from being mixed together, and the temperature control on the liquid reservoir tanks of the respective systems is made easier. For performing a PCR, for example, a mixture of 1.0 μL of reaction buffer, 1 μL of 2 mM dNTP (dATP, dCTP, dGTP, dTTP), 1.2 μL of 25 mM magnesium sulfate, 0.125 μL of 10% fetal bovine serum, 0.5 μL of SYBR Green I, 0.6 μL of each of two types of primers, 3.725 μL of sterilized water, 0.25 μL of KOD pLus polymerase, and 1.0 μL of genomic DNA can be used. Regarding the temperature, the PCR may be first performed at 90°C for 10 seconds, and then the cycle of performing the PCR at 90°C for 1 second and then 60°C for 3 seconds may be repeated 40 times.

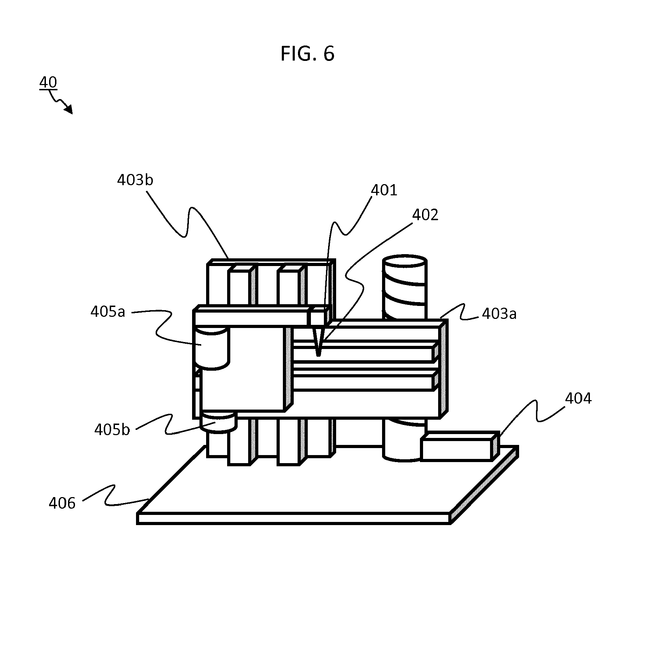

FIG. 6 shows an example of the structure of the liquid transfer module 40 that transfers a sample between the modules. The liquid transfer module 40 includes a separation head 401 and a separation chip 402 through which a liquid is transferred between the modules located on a chassis 406. The liquid transfer module 40 also includes a Z-axis transfer guide 403 and a Z-axis transfer motor 404 that control the separation head in a height direction along a Z axis, and an arm rotation motor 405 as an arm rotation mechanism. The liquid transfer module 40 has the function of controlling the position of the separation head 401 on an X-Y plane by the Z-axis transfer guide 403, the Z-axis transfer motor 404 and the arm rotation motor 405.

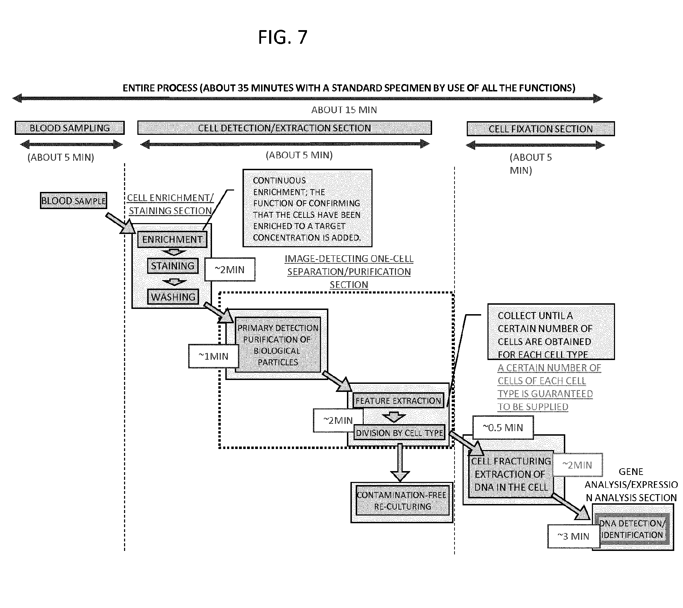

FIG. 7 shows an example of procedure from sampling to analysis of a sample as performed by use of a cell analysis device according to the present invention. The procedure is performed on a sample solution in which a nucleus component in a cell such as spore of Bacillus anthracis or the like is not easily eluted owing to a shell covering the cell. The procedure includes a step of fracturing the shell covering the cell before a step of performing expression analysis on the cell. Owing to the step of fracturing the shell covering the cell, the cell analysis device according to the present invention can analyze a cell such as a spore of Bacillus anthracis or the like with exactly the same means as the blood cell analysis means described above.

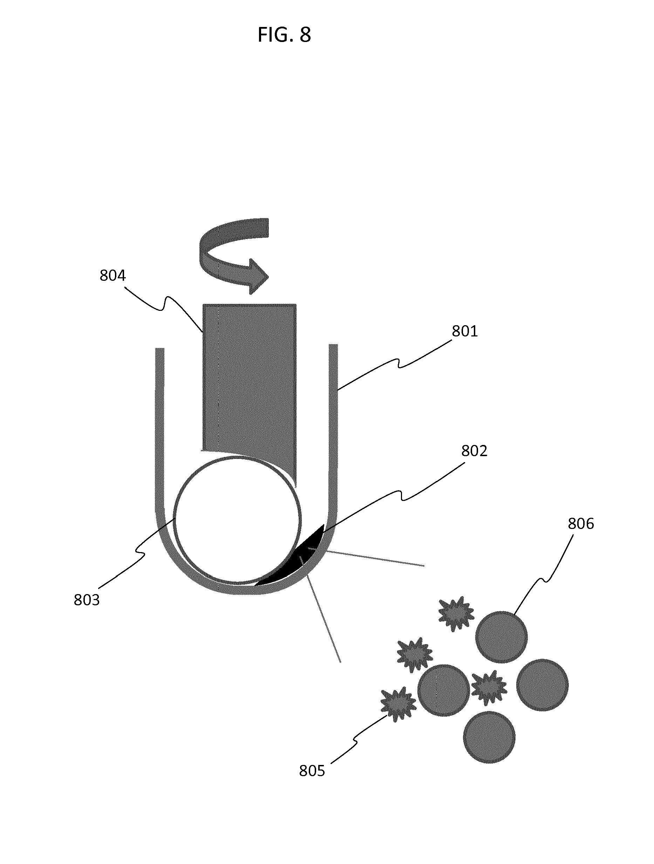

FIG. 8 schematically shows an example of the basic structure provided for analyzing information on a cell such as a spore of Bacillus anthracis or the like; more specifically, information on the gene in the cell and expression thereof. The structure automatically fractures a shell of the spore or the like that covers the cell in a trace amount of sample. A trace amount of sample 802 is put into a vessel 801, and a rotator for fracturing is located in the vessel 801. In order to rotate the rotator 803 in the vessel 801, the rotator 803 is pressed to the vessel 801 by a rotation shaft 804. The rotator 803 is rotated and revolved in the vessel, and thus a sample 805 in the trace amount of sample is ground by a grinding agent 806. After this step, the rotator 803 is removed, and thus the post-process sample 805 can be easily recovered. The rotator 803 and the vessel 801 have a simple structure, and therefore may be handled as expendables with no problem.

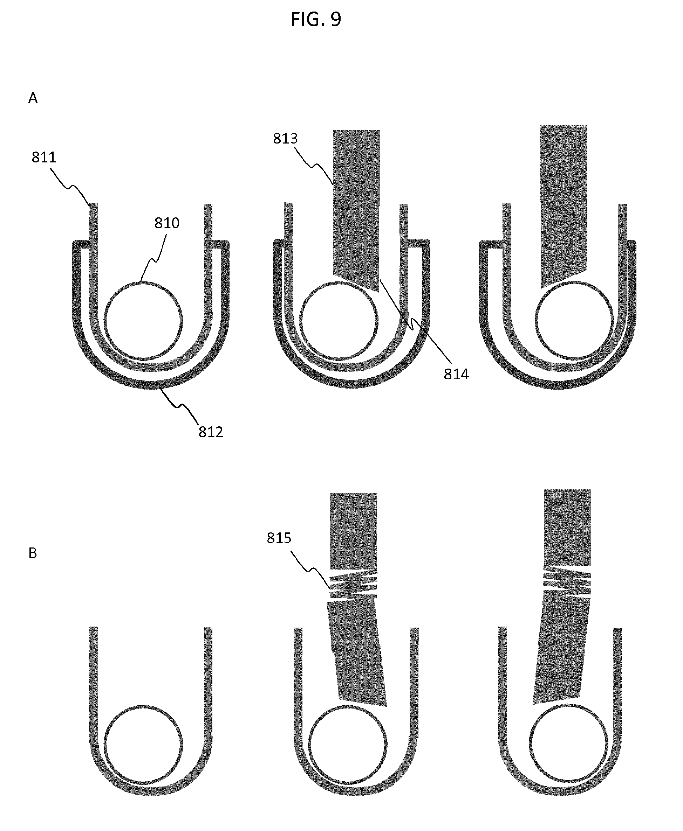

FIG. 9 schematically shows variations of the basic cell fracture mechanism shown in FIG. 8. In order to fracture the sample at a high efficiency, the rotator needs to be in close contact with the vessel. In the example shown in FIG. 9A, a vessel 811 accommodating a rotator 810 is held by a flexible body 812 formed of rubber or the like. A tip 814 of a shaft 813 is cut to be oblique. Therefore, when the shaft 813 is pressed to the rotator 810, the rotator 810 presses the vessel 811 downward and laterally. This deforms the flexible structure 812 and thus absorbs the pressure. As a result, the sample can be fractured while the rotator 810 and the vessel 811 are kept in close contact with each other without providing excessive stress to the rotation shaft 813. As shown in FIG. 9B, a spring mechanism 815 which is deformable vertically and laterally may be incorporated into the rotation shaft as an element that releases the stress.

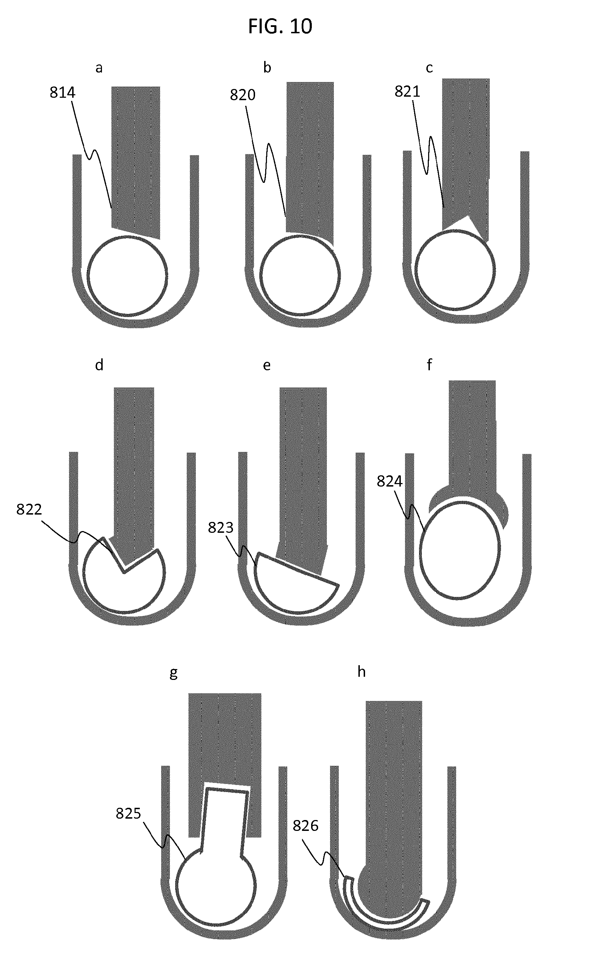

FIG. 10 shows variations of the rotator and the rotation shaft of the cell fracture mechanism usable in the present invention. The tip of the shaft may be cut to be oblique (FIG. 10a), may be recessed to have a mild curve (FIG. 10b), or may be mortar-like (FIG. 10c). The rotator does not need to be completely spherical and may have a shape loosely engageable with the shaft (FIG. 10d). A semicircular rotator may be rotated by a rotation shaft having a tip cut to be oblique (FIG. 10e). The rotator may be egg-shaped (FIG. 10f) or may have a protrusion engageable with the rotation shaft (FIG. 10g). A dish-like rotator may be rotated by the shaft (FIG. 10h).

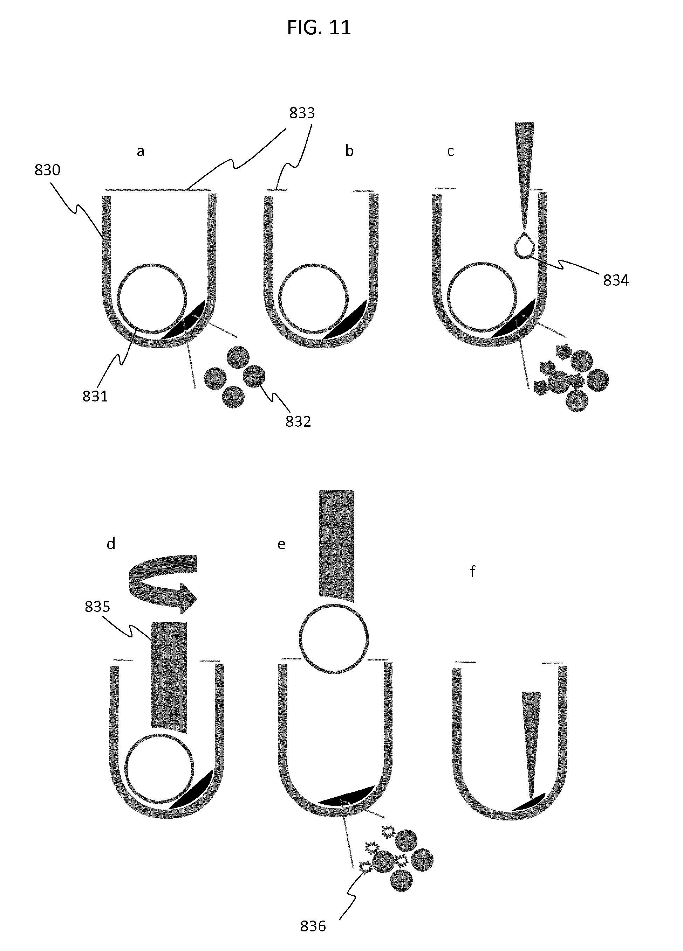

FIG. 11 shows an example of a cell fracture step according to the present invention. In a vessel 830, a rotator 831 and a grinding agent 832 are accommodated in an air-tight manner (FIG. 11a). Immediately before cell fracturing, a seal 833 is broken (FIG. 11b) and a sample 834 containing cells is put into the vessel 830 (FIG. 11c). The rotator 831 is rotated while being pressed by a rotation shaft 835 (FIG. 11d), and thus the cells in the sample are fractured by the grinding agent 832 and a component 836 is eluted (FIG. 11e). After the work of fracturing, the rotator 831 is removed from the vessel 830. Thus, the sample can be easily recovered (FIG. 11f). The rotator may be removed by use of a negative pressure, a magnetic force or an electrostatic force. A mechanism that removes the rotator may be incorporated into the rotation shaft. Needless to say, a dedicated mechanism may be separately prepared.

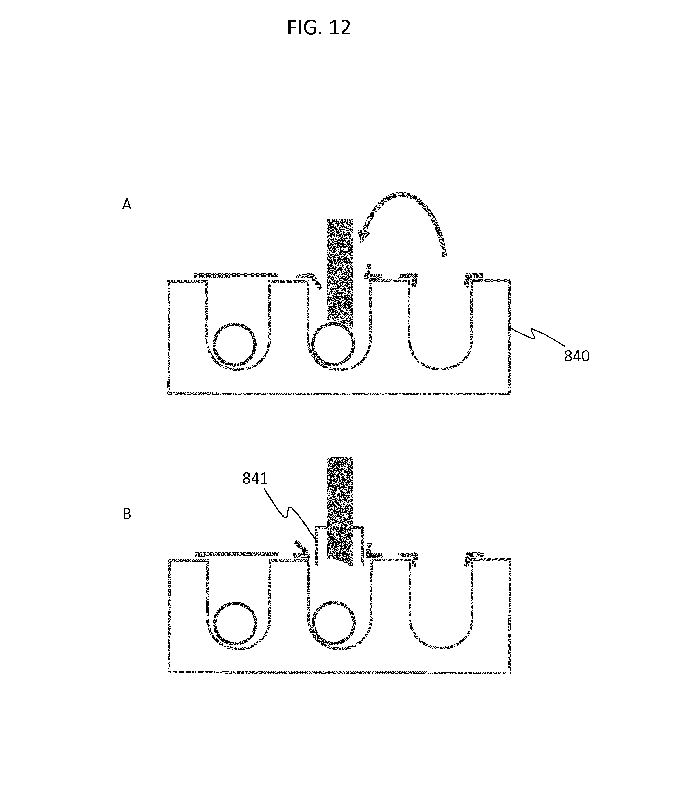

FIG. 12 provides conceptual views of a mechanism that is usable in the case where the cell fracture step according to the present invention is automated. A plurality of vessels 840 are integrally formed, and a rotator is accommodated in each vessel in advance in an air-tight manner. The seal may be broken, for example, by directly pressing the rotation shaft (FIG. 12A), by use of an opening cutter 841 attached to the rotation shaft (FIG. 12B) or the like. The relative positions of the shaft and the vessel can be automatically changed, so that a plurality of samples can be fractured one after another.

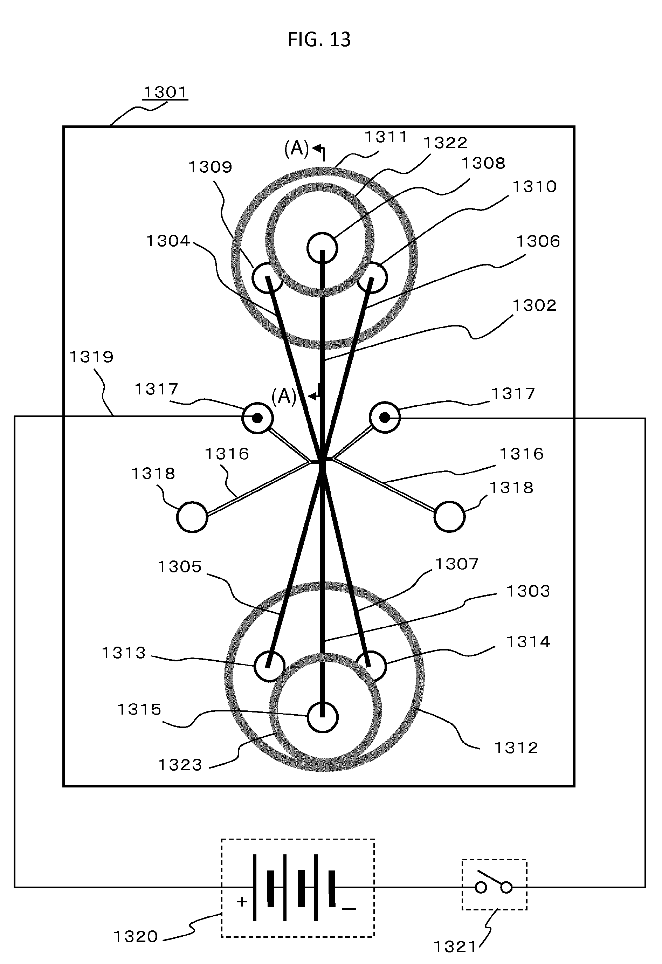

FIG. 13 shows an example of an on-chip cell sorter chip according to the present invention that is usable in the cell analysis device system according to the present invention. In a cell sorter chip 1301, axially symmetrical three flow paths are located in an upstream part (1302, 1304, 1306) and axially symmetrical three flow paths are located in a downstream part (1303, 1305, 1307), on a chip substrate. The flow paths in the upstream part and the flow paths in the downstream part are symmetrical to each other. At the joining point thereof, the three flow paths join together while keeping a laminar flow and branch into the three flow paths on the downstream side while keeping this state. Therefore, the upstream central flow path 1302 in which a sample flows is guided toward the downstream central flow path 1303. Two side sheath flows are guided in the same manner. Specifically, the upstream side flow path 1304 is guided toward the downstream side flow path 1305, and the upstream side flow path 1306 is guided toward the downstream side flow path 1307. Entrances of the three upstream flow paths are respectively linked to entrance openings 1308, 1309 and 1310. Among these entrance openings, the entrance opening 1308 for the upstream central flow path in which the sample flows is linked to a sample solution reservoir 1322. The entrance opening 1308 is typically (but not limited to being) provided with a small annular cap (or plug) so as to be isolated from the entrance openings 1309 and 1310 for the flow paths in which a sheath liquid accumulated in a sheath liquid reservoir 1311 flows. Thus, the sample solution is not spread around. The downstream reservoirs are located in the same manner as the upstream reservoirs. A waste liquid reservoir 1312 is linked to exit openings 1313 and 1314 for the flow paths in which two side sheath liquids flow. By contrast, a purified sample recovering reservoir 1323 is linked to an exit opening 1315 for the recovered purified sample. The exit opening 1315 is typically (but not limited to being) provided with a small annular cap so that the recovered purified sample is not spread to the waste liquid reservoir.

The flow rate may be generated by a gravitational system by use of the difference between the liquid surface level of the sample reservoir and the sheath liquid reservoir and the liquid surface level of the waste liquid/recovered liquid reservoir, or by providing a top surface of each reservoir with a cap and thus using the pressurized air to apply a pressure to the liquid surface. In such a case, in order to generate ideal laminar flows at the joining point, it is preferable that the three flow paths upstream of the joining point and the three flow paths downstream of the joining point respectively match one another in the cross-sectional shape and the length from the joining point to the solution opening. It is also desirable that the cross-sectional surface area of each reservoir for the side sheath flow (or for the waste liquid) and the cross-sectional surface area of the corresponding inner reservoir for the sample/recovered sample is 1 (sample/recovery reservoir) : 2 (side sheath liquid reservoir/waste liquid reservoir). A reason for this is that when the reservoirs have different liquid surface levels, the decreasing ratios of the liquid surface levels are different from one another, which eventually prevents the generation of the laminar flows at the joining point. The ratio between the flow amount of the liquid per unit time at the one sample entrance and the flow amount of the liquid per unit time at the two sheath liquid entrances is 1:2. Therefore, the ratio between the cross-sectional surface areas of the two types of reservoirs is defined as 1:2 so that the reservoirs have the same liquid surface level. This can be generalized as follows: it is desirable that the ratio between the total cross-sectional surface areas of the flow paths linked to the two types of reservoirs matches the ratio between the cross-sectional areas of the two types of reservoirs.

Now, the electrodes are located at the point where all the six flow paths join together and where the three laminar flows with no wall join together. Each of the electrodes is typically formed of a gel electrode. As the gel, for example, agarose gel having Nail dissolved therein is used so that an electrolyte acts as a current carrier. In order to allow the tip of the gel to be in direct contact with the flow path, agarose gel in a sol state is put into each of Y-shaped flow paths 1316 via a corresponding entrance 1317. The Y-shaped flow paths 1316 are provided to be filled with the gel. In order to allow the gel to be transferred toward an exit 1318, the gel is not allowed to enter the cell sorter flow path and is stopped at the border by surface tension. An advantage of using a gel electrode is as follows. A cable 1319 of platinum or the like that is connected to a power source 1310 in order to apply an electric field is inserted into the gel introduction point. Thus, at the border of the gel electrode that is in contact with the flow path, a current can be applied with no bubble being generated even when the voltage is raised to a level at which bubbles are generated in the flow path with a usual metal electrode. The on/off state of the electric field application can be adjusted by use of, for example, a switch 1321.

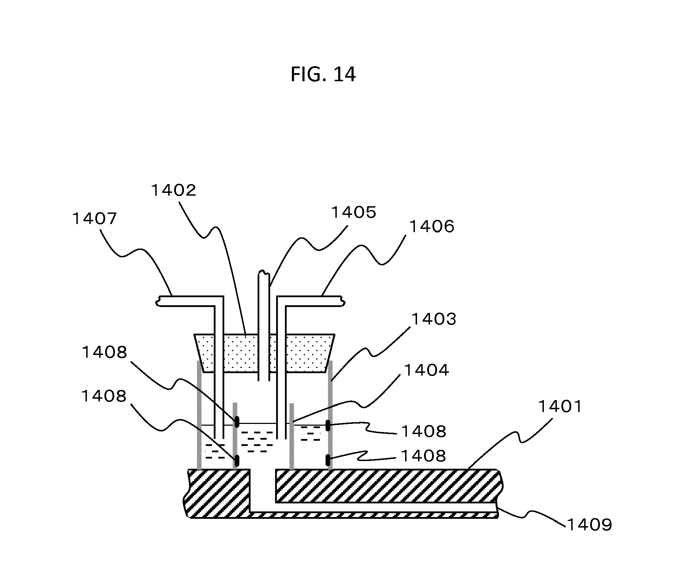

FIG. 14 schematically shows an example of cross-section of the upstream reservoirs taken along line A-A in FIG. 13. In a cell sorter chip 1401, a flow path 1409 is buried. In a typical embodiment, a top surface of an outer sheath liquid reservoir 1403 is covered with a cap 1402, and thus an air pressure having an appropriate flow rate is supplied from a pressurized air introduction pipe 1405. As can be seen from the figure, the flow path 1409 in which a sample flows is linked to a sample reservoir 1404, so that the sample solution and the sheath liquid are not mixed together. In a preferable and typical embodiment, since the ratio between the number of the flow path for the sample reservoir and the number of the flow paths for the sheath liquid reservoir is 1:2, the ratio between the cross-sectional surface area of the sample reservoir and the cross-sectional surface area of the sheath liquid reservoir is 1:2. Thus, the reservoirs linked to the flow paths have the same liquid surface level.

In order to allow a larger amount of sample to be processed, a liquid supply mechanism may be additionally provided. Such a mechanism includes a sample solution introduction tube 1406 or a sheath liquid introduction tube 1407 and a water level measurement sensor 1408 incorporated into a wall surface of each reservoir. The water level measurement sensors 1408 perform conductivity measurement. Owing to this structure, when the water level is below a certain level, the sample solution can be supplied via the tube until the water level is returned to the certain level. The water level measurement sensors 1408 may be each formed of an electrode, an electrode pair or the like located at a lower limit and an upper limit of the water level to be set.

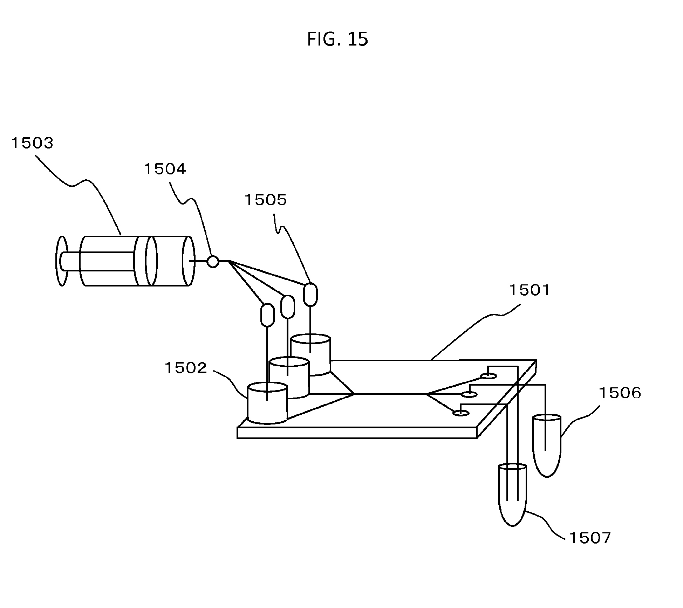

FIG. 15 shows an example of another structure of a cell sorter according to the present invention for handling a larger amount of sample. On a chip 1501, three large reservoirs 1502 are located in upstream parts of three flow paths respectively. A pressure from an air pressure application device 1503 can be distributed to these reservoirs flexibly via a pressure sensor 1504 by use of distribution valves 1505. The sorted (purified) sample and a waste liquid are respectively recovered by a sorted sample recovery reservoir 1506 and a waste liquid recovery reservoir 1507 which are located downstream of the chip.

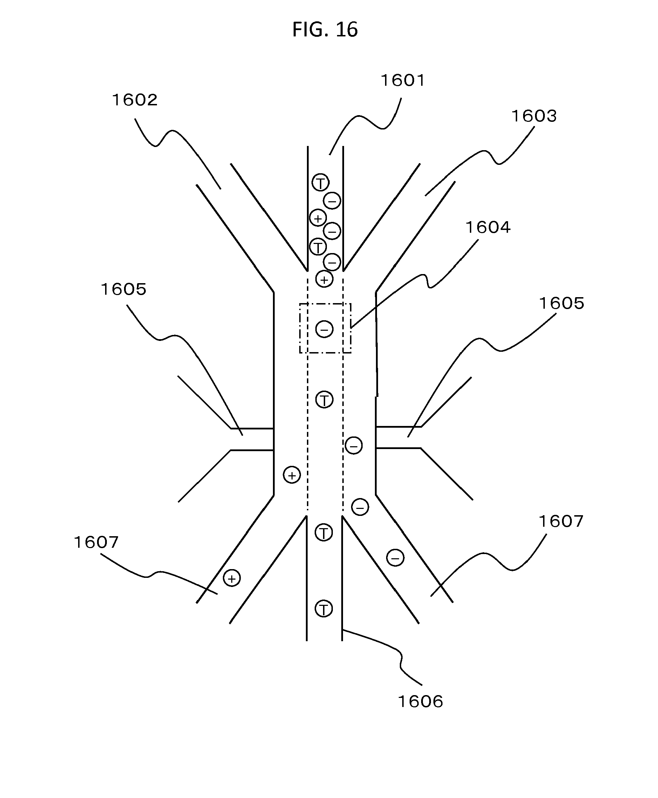

FIG. 16 schematically shows an actual procedure of recovering a sample in a chip. A sample solution flow 1601 flowing from an upstream position advances to a cell monitor area 1604 while being held between two side sheath solution flows 1602 and 1603. In the cell monitor area 1604, the shape of each cell is distinguished and it is checked whether fluorescence labeling is present or absent. Based on the results, the cells are separated at a downstream position. When a cell to be recovered reaches the cell monitor area 1604, the cell is allowed to flow downstream to a sorted sample recovery flow path 1606. When a cell or a particulate to be abolished reaches the cell monitor area 1604, a voltage is applied to two gel electrodes 1605 located to face each other, regardless of whether the charge of the cell or the particulate is positive or negative. Thus, the cell or the particulate is transferred to either one of the two side sheath flows 1607 and thus can be discharged.

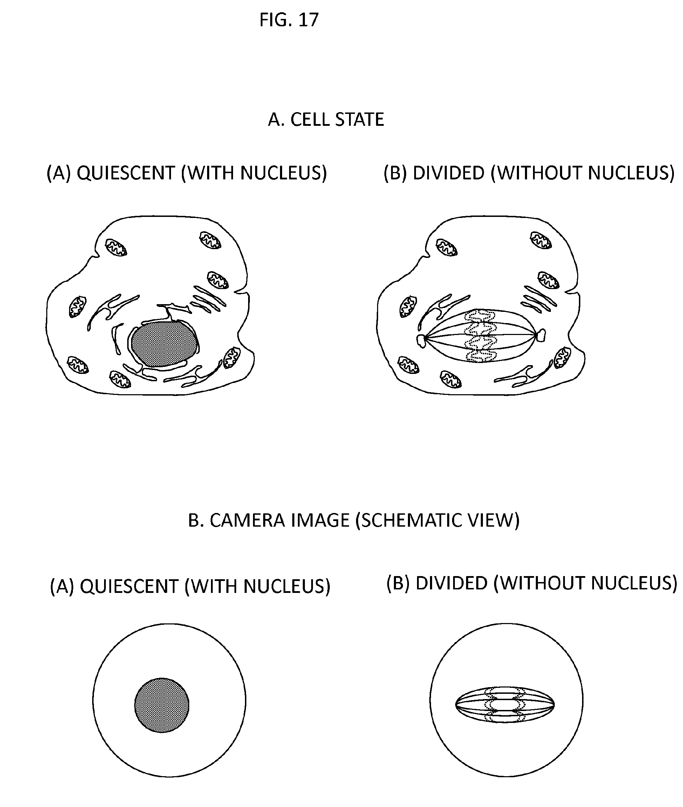

FIG. 17 provides schematic views illustrating one index for recovering a cell by an image-processing cell sorter. Usually, the cell is in the G0 period and contains a nucleus (FIG. 17A(a)). The nucleus is clearly recognized as a black sphere in the cell (FIG. 17B(a)). By contrast, in a cell in a division period, the nucleus has separated (FIG. 17A(b)). Therefore, the nucleus cannot be confirmed to be present even by image recognition of the cell (FIG. 17B(b)). With the conventional labeling technology such as antibody labeling or the like, it is difficult to check the state of the cell. By contrast, according to the present invention, such image recognition can be performed to check the shape of the cell and also to recover the cell in the division period based on the presence/absence of the nucleus in the cell. In general, most of the normal cells flowing in the blood have past the final differentiation stage. However, recovery of cells in the division period in the blood according to the present invention makes it possible to recover cells having a cell division capability such as cancer cells in the blood, stem cells or the like.

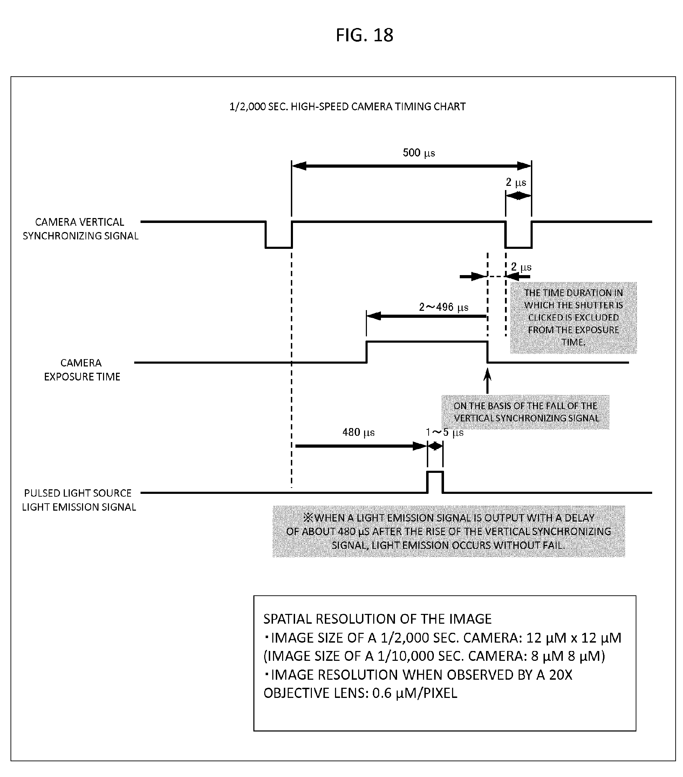

FIG. 18 shows an example of a timing chart of an actual operation of an image-recognizing cell sorter according to the present invention by use of a flash light source. In the case where a high-speed camera is used to observe cells transferring in a chip, blur can be prevented as follows. A spatial resolution for one pixel of the camera is found based on the magnification of the objective lens. In the case where the transfer rate of the sample flow has been determined, the transfer amount of one pixel at the determined flow rate is found. The flash light is turned on for the time duration required for the transfer. Namely, the flash is fired once during each shutter interval with the flash time being set as:

flash time = pixel size/flow rate.

For example, the pixel size of a 1/2,000 sec. camera is 12 $\mu$m x 12 $\mu$m. The pixel resolution obtained when observation is made by a 20x objective lens is 0.6 $\mu$m/pixel. Therefore, when an LED light source capable of firing a flash at a rate of 1 $\mu$s for a flow of 60 cm/s is used, an image with no blur can be actually acquired.

The present invention also provides an on-chip cell sorter system. Illustrative embodiments thereof will be described hereinafter. In such a system, control of each of the sections (e.g., image acquisition and analysis by use of an optical system, application of an external force by an external force application device, etc.) can be performed by a control device including a personal computer or the like, like in the above-described embodiment.

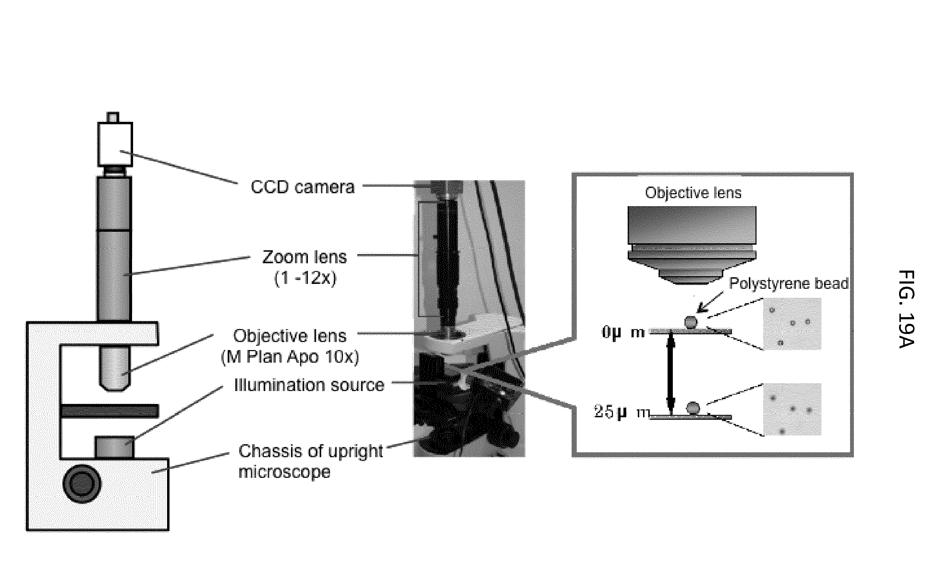

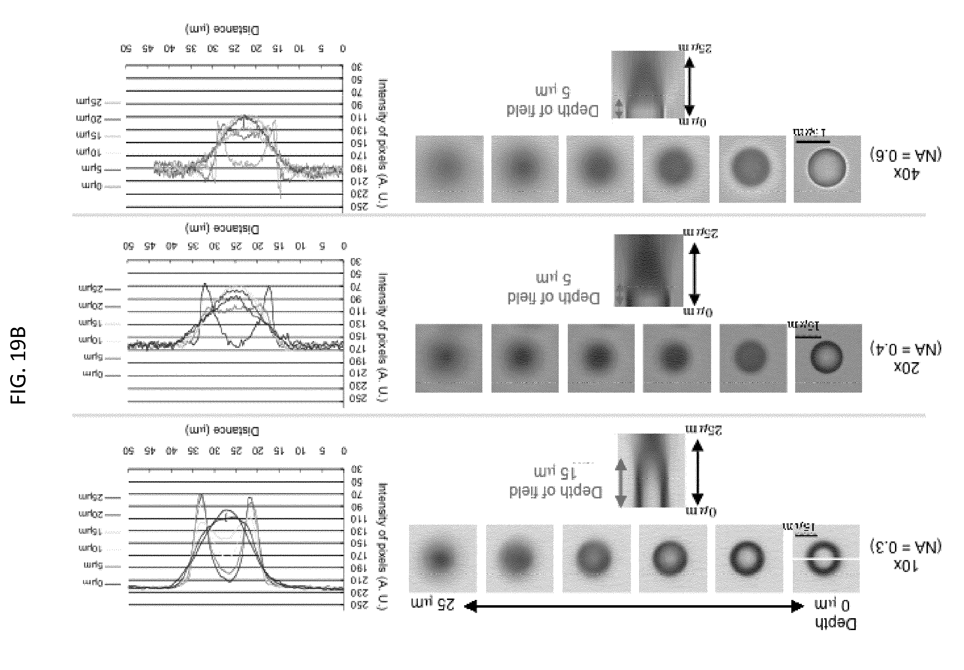

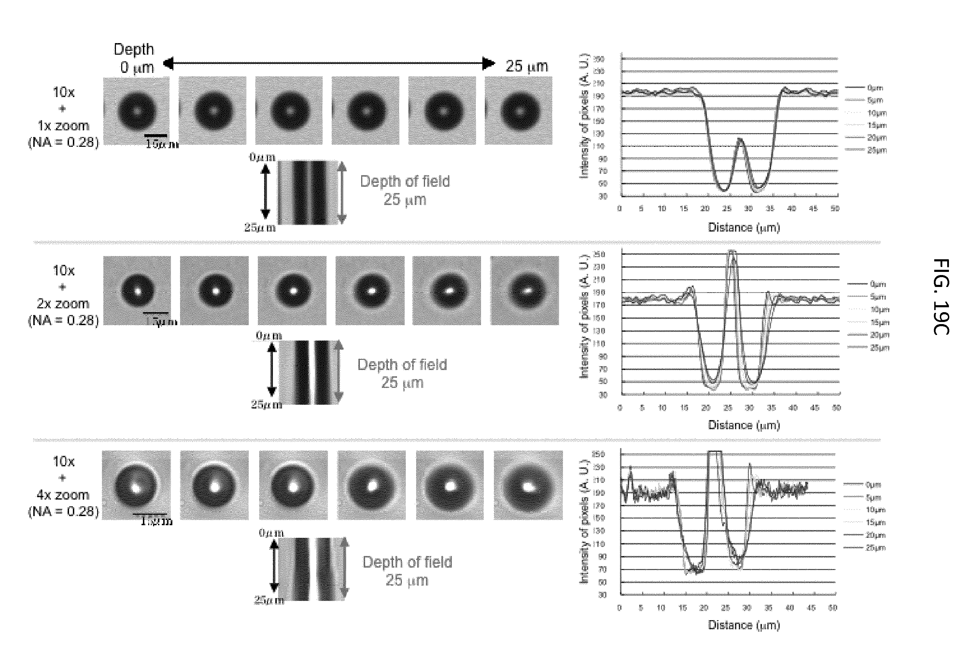

FIG. 19 schematically shows an example of a structure of an optical system that is included in an image-detecting one-cell separation/purification (cell sorter) module and prevents image blur. Usually, in the case where a target particle is observed by an optical system of a microscope, the magnification ratio of the target image is determined based only on the magnification of the objective lens. However, in such a case, the focal depth and the depth of field of the optical system depend on the magnification and the numerical aperture of the objective lens. As the magnification of the objective lens is increased, the focal depth and the depth of field of the optical system are decreased.

In the case where targets in a microscopic flow path are observed and the targets are cells, in order to allow various sizes of samples, including microscopic samples having a size of about several micrometers and clusters having several tens of micrometers, to flow, the flow path needs to have a sufficient width and a sufficient depth for the maximum size of sample to flow. However, in order to distinguish the type of the sample by image recognition, it is more preferable that the resolution of the image is higher. In general, in order to increase the magnification of an optical microscope, it is common to use an objective lens having a higher numerical aperture. However, there has been a problem that with such means, the focal depth is decreased, which results in decrease in the depth of field in the flow path. In order to identify a sample from a higher definition image by use of an image-recognizing cell sorter, the magnification ratio of the target sample is increased while the depth of field is made approximately equal to the surface level of the flow path. This can be realized by first selecting an objective lens having a numerical aperture with which the focal depth and the depth of field thereof are approximately equal to the surface level of the flow path, and then providing a zoom lens in a stage after the objective lens. Specifically, as shown in FIG. 19A, an objective lens and a zoom lens may be combined and a magnified image thereof may be captured by an image capturing device such as a CCD camera or the like.

Specifically, as shown in, for example, FIG. 19B, the height that can be imaged when a 10x objective lens having a numerical aperture (NA) of 0.3 is used is measured. In this case, it is seen that an image having a height of up to about 15 $\mu$m can be captured with no problem. By contrast, with a 20x objective lens having a numerical aperture of 0.4 or a 40x objective lens having a numerical aperture of 0.6, the maximum height of the image that can be captured with no blur is merely about 5 $\mu$m.

From the above results, it has been confirmed that when the focal depth is about 15 $\mu$m or less, an image with no blur is obtained by use of a 10x objective lens having a numerical aperture of 0.3. FIG. 19C shows the results of measurement with a zoom lens having a numerical aperture of 0.28 being provided in a stage after this objective lens. As can be seen, an image obtained by a combination of a 10x objective lens and a 1x zoom lens, an image obtained by a combination of a 10x objective lens and a 2x zoom lens, and an image obtained by a combination of a 10x objective lens and a 4x zoom lens can be observed sufficiently well with no blur regardless of the difference in the magnification of the zoom lens, as long as the focal depth and the depth of field are each 25 $\mu$m or less.

It is seen by these results that when an image-processing cell sorter system is used, an image substantially the same as the image conventionally observed by use of a 40x objective lens can be captured by a combination of a 10x objective lens and a 4x zoom lens. The image has no blur in the height direction of the flow path and is optimal for cell sorting.

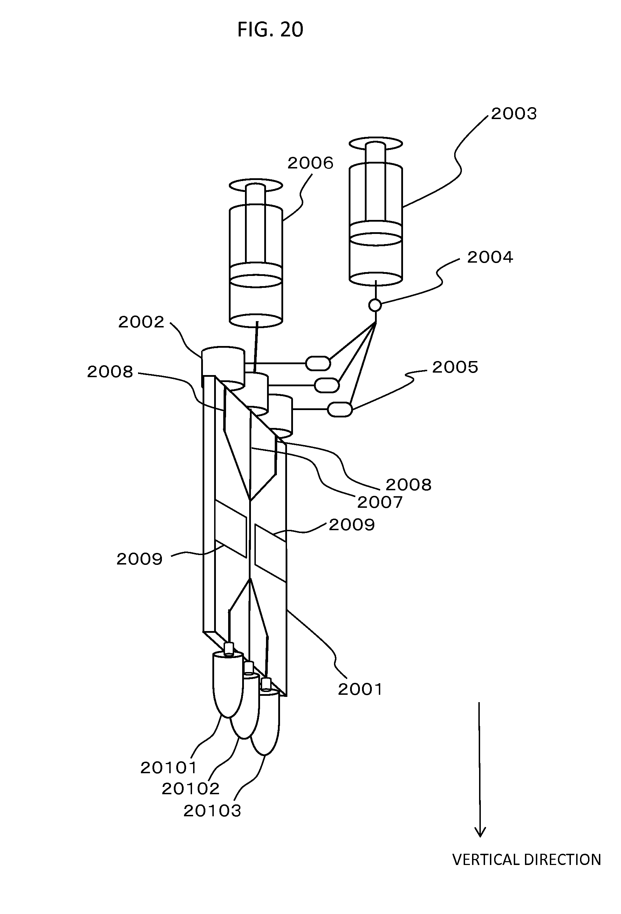

FIG. 20 shows a structure for solving a problem of the conventional art. Conventionally, a cell sorter chip is located horizontally. Therefore, the sample is precipitated at the bottom of the horizontal flow path, and this causes clogging. In the structure shown in FIG. 20, the cell sorter chip is located vertically, so that the precipitation direction of the cells is the same as the direction of the flow. A cell sorter chip 2001 in the cell sorter system is located vertically as shown in FIG. 20, and an upstream part of the flow is in a top part and a downstream part of the flow is in a bottom part. A sample solution and a buffer solution are provided from a sample solution introduction device 2006 and a buffer introduction device 2003 respectively with a pressure applied thereto being controlled by use of a pressure sensor 2004. The sample solution and the buffer solution are introduced into respective reservoirs 2002 connected to a top surface of the chip. In the case where the sample or the buffer solution is to be introduced from one introduction device to a plurality of reservoirs, the flow in each flow path can be fine-adjusted by fine-adjusting the open/close state of a corresponding distribution valve 2005 in accordance with the pressure or the state of each reservoir. Next, the liquids introduced from the reservoirs are joined together via a sample solution flow path 2007 or buffer flow paths 2008. The cells are transferred vertically along the flow in the flow path by sorting external force application mechanisms 2009. Each cell is transferred as described above based on the type of the cell distinguished by image acquisition or the results of quantitative measurement of fluorescence or scattered light, for example, the results of labeling and measurement of fluorescence or the like. As a result, the cells can be recovered by a plurality of downstream reservoirs 20101, 20102 and 20103.

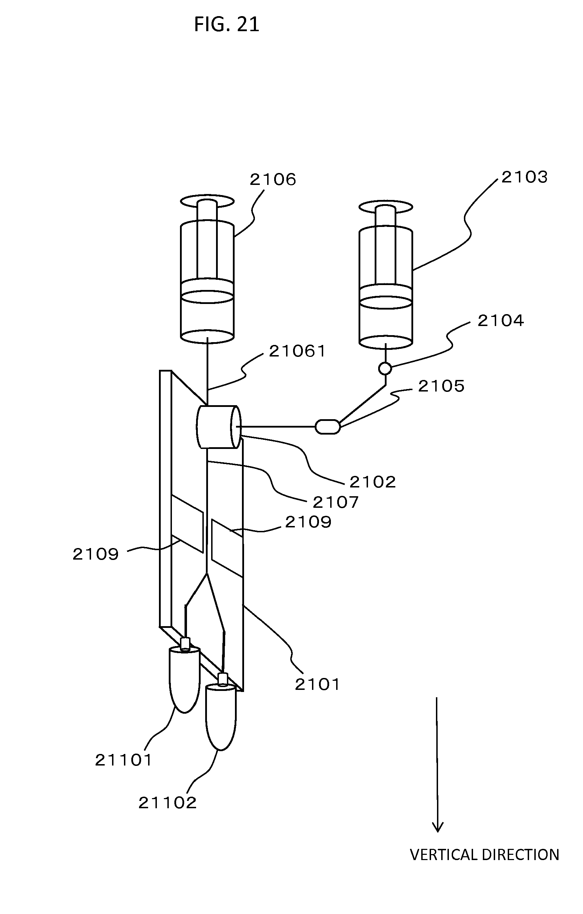

FIG. 21 shows an example of another structure of the cell sorter shown in FIG. 20 in which the chip is located vertically. As shown in FIG. 21, like in FIG. 20, a cell sorter chip 2101 in the cell sorter system is located vertically. An upstream part of the flow is in a top part and a downstream part of the flow is in a bottom part. A sample solution and a buffer solution are provided from a sample solution introduction device 2106 and a buffer introduction device 2103 respectively with a pressure applied thereto being controlled by use of a pressure sensor 2104. The sample solution and the buffer solution are introduced into a reservoir 2102 connected to a top surface of the chip. The sample solution, which is introduced into the reservoir 2102 after passing a sample solution introduction flow path 21061 formed of a capillary tube, is introduced into a flow path 2107 as being held between the buffer solutions. Next, the cells flowing in the flow path 2107 are transferred vertically along the flow in the flow path by sorting external force application mechanisms 2109. Each cell is transferred as described above based on the type of the cell distinguished by image acquisition or the results of quantitative measurement of fluorescence or scattered light, for example, the results of labeling and measurement of fluorescence or the like. As a result, the cells can be recovered by a plurality of downstream reservoirs 21101 and 21102.

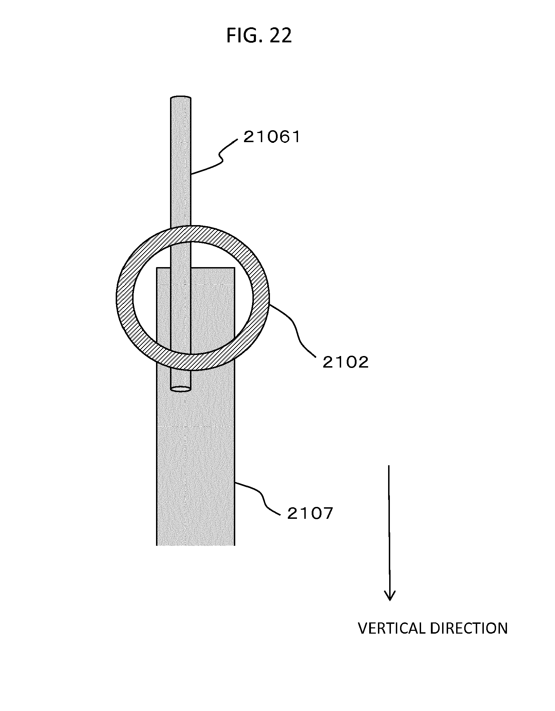

FIG. 22 schematically shows an example of a structure of a part of the cell sorter chip shown in FIG. 21, where a sample solution and a buffer solution are joined together. A sample solution is introduced into the flow path 2107 from the capillary tube 21061, and a buffer solution is introduced into the reservoir 2102 connected to the top surface of an end point of the flow path 2107. The reservoir 2102 is located so as to introduce the buffer solution into the flow path 2107. In this structure, an exit of the capillary tube 21061 is located downstream of the reservoir section, so that the sample solution can be introduced into a desired position in the flow path of the buffer solution. In the case where the cells are to be sorted, it is desirable that the capillary tube 21061 is located closer to one lateral side of the flow path 2107. In this example, the capillary tube is used to introduce the sample solution into the flow path of the buffer solution. Alternatively, the capillary tube does not need to be used as long as the sample solution can be introduced into an area where a flow of the buffer solution is generated. Substantially the same effect is provided by a combination of precision-processed flow paths.

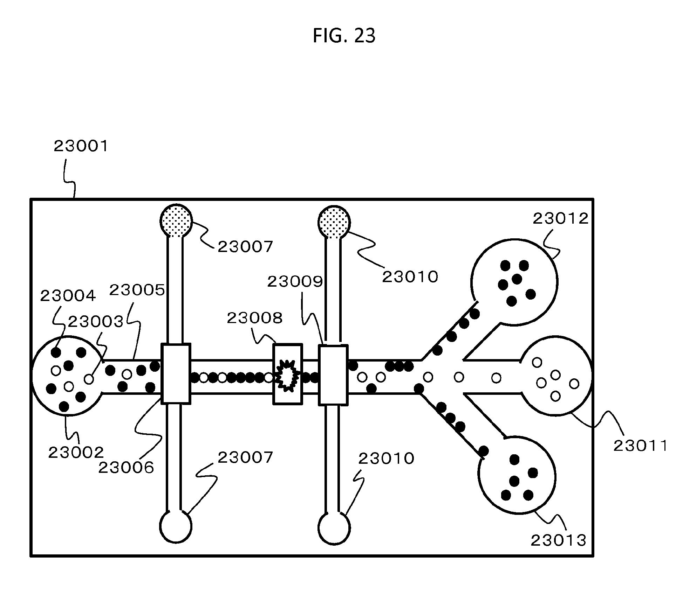

FIG. 23 schematically shows an example of a structure of a chip that is in a cell sorter system and includes a mechanism that performs cell sorting. In a chip 23001, a sample introduction opening 23002 is located in an upstream part. A sample solution introduced into the sample introduction opening 23002 contains, in a mixed state, target particles 23003 to be recovered and unnecessary particles 23004 to be abolished. The sample solution flows in a micro-flow path 23005 and is first introduced into a particle arraying mechanism 23006. In the particle arraying mechanism, particle arraying external force inputs (electric force or sheath flow) 23007 are located. In the case where the sample is to be arrayed by use of an electric force, gel electrodes are located at both of two sides of the flow path 23005 to apply an electric field. In the case where the sample is to be arrayed by use of a sheath flow, a buffer solution is introduced. Next, the type of each particle in the sample arrayed in a line is identified by a particle detection mechanism 23008, and in the next stage, the particles are separated by a particle purification mechanism 23009. In the particle detection mechanism 23008, particle purification external force inputs ((gel or metal) electrodes + electric force) 23010 are located. An electric force can be provided to the position of the particle purification mechanism 23009 by use of gel electrodes or metal electrodes. When, for example, a target particle to be recovered reaches the particle purification mechanism 23009, the target particle is allowed to flow downstream with no application of an external force and is recovered at a recovery opening 23011. When a particle other than the particle to be recovered reaches the particle purification mechanism 23009, the particles are provided with an external force by the particle purification mechanism 23009 and thus can be guided to unnecessary particle reservoirs 23012 and 23013.

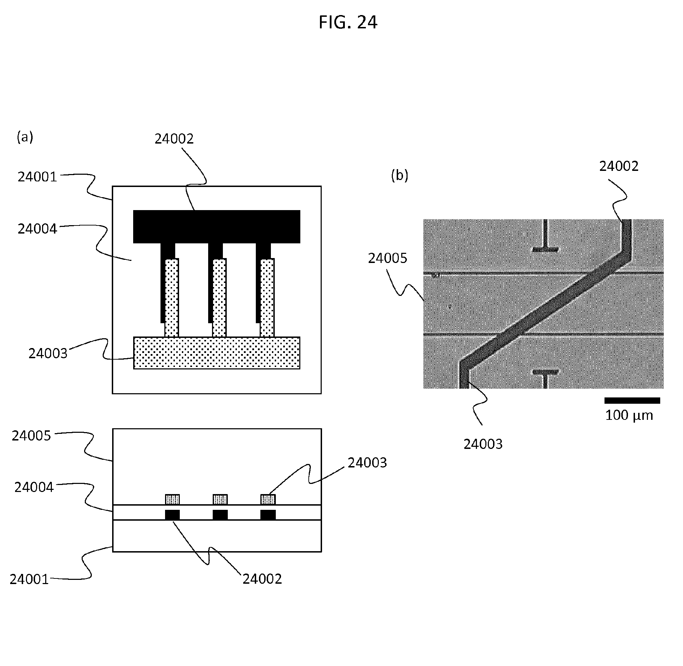

FIG. 24 shows an example of electrode arrangement for providing an external force in a flow path of a cell sorter system. FIG. 24(a) is a schematic plan view, and FIG. 24(b) is a schematic cross-sectional view. As shown in FIG. 24(a) and FIG. 24(b), on a support substrate 24001, two comb-like electrode parts, namely, a metal thin film electrode first layer 24002 and a metal thin film electrode second layer 24003 overlap with each other while being shifted slightly. An insulating layer 24004 is provided between the layers 24002 and 24003. The second electrode 24003 is in direct contact with a sample flow path 24005. FIG. 24(c) shows a photograph of an example in which an array of comb-like electrodes is actually located on a bottom surface of the flow path with the above-described structure.

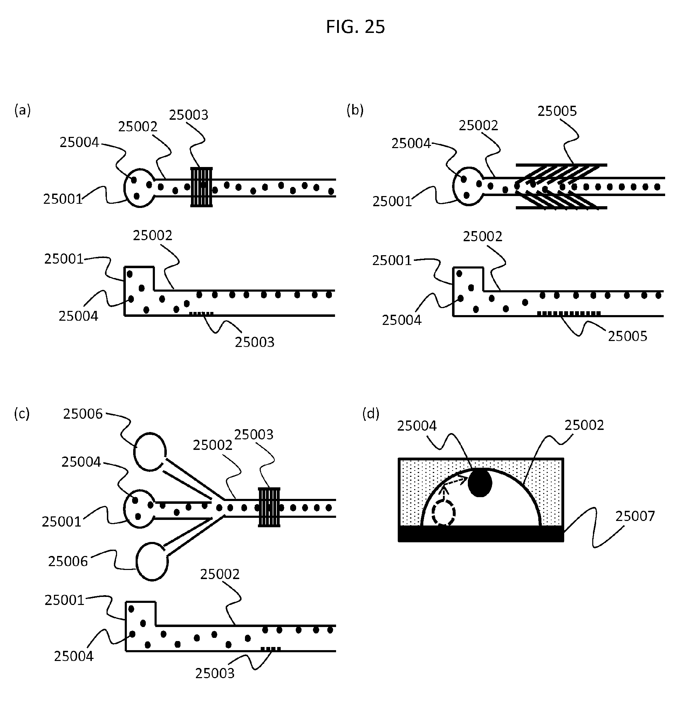

FIG. 25 shows an example of electrode array arrangement for providing an external force in a flow path of a cell sorter system. FIG. 25(a) shows that sample particles 25004 introduced into a sample introduction opening 25001 flow in a micro-flow path 25002. It is now assumed that metal thin film-stacked parallel comb-like electrodes 25003 having the structure as shown in FIG. 24 are located in the flow path. When an AC electric field is generated in the metal thin film-stacked parallel comb-like electrodes 25003, the sample particles 25004 advance toward a top surface by a dielectric electrophoretic force. As shown in FIG. 25(d), the flow path has a semicircular cross-section. With such a structure, when the particles advance toward the top surface upon receipt of a repulsive force from a bottom surface of the flow path, the particles are gathered at a particular position of the top surface of the flow path. In this example, the flow path has a semicircular cross-section. Alternatively, the flow path may have a triangular cross-section. In FIG. 25(b), metal thin film-stacked V-shaped comb-like electrodes 25005 are used. In substantially the same manner, a repulsive force is generated from an electrode 25007 on the bottom surface of the flow path to guide the particles to the top wall, so that the particulates can be arrayed in a line at a center of the flow path owing to the shape of the top wall. In FIG. 25(c), sheath flow paths 25006 for a better solution are additionally provided on both of two sides of the flow path 25002, so that the particulates can be arrayed.

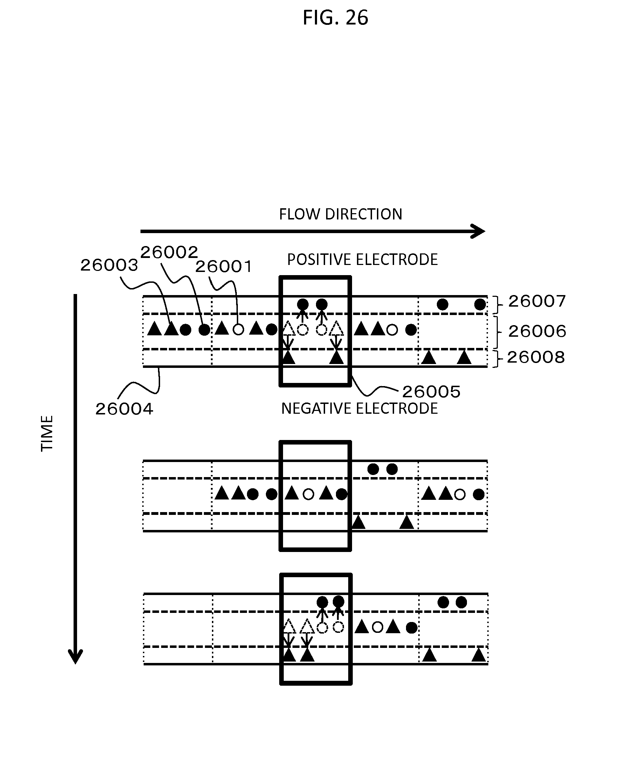

FIG. 26 schematically shows an example of a process of cell purification performed in a flow path of a cell sorter system. A step of cell purification after the cell identification will be described. Target particles 26001, unnecessary particles (charged negative) 26002 and unnecessary particles (charged positive) 26003 flow in a micro-flow path 26004 in the state of being arrayed in a line. When a target particle is in a particle purification electrode 26005 area, the target particle is guided to a flow 26006 directed toward a target particle recovery opening because the electrodes are in an off state. By contrast, when an unnecessary particle reaches the particle purification electrode 26005 area, an external electric field is turned on. Then, the unnecessary particle is attracted to a positive electrode or a negative electrode and thus is removed from the center of the flow path and guided to a flow 26007 or 26008 to an unnecessary particle reservoir.

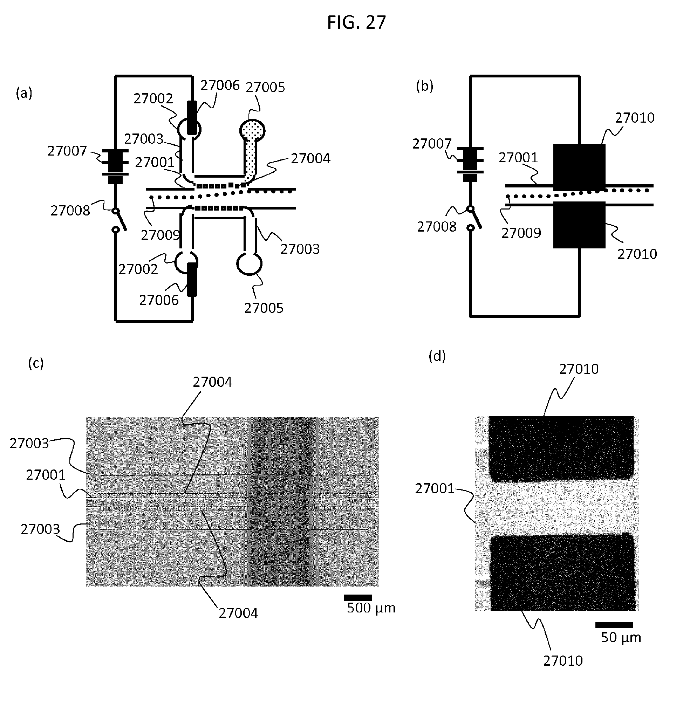

FIG. 27 provides schematic views showing an example of gel electrode arrangement for providing an external electric field in a flow path of a cell sorter system, and photographs showing an example of an actual chip. As shown in FIG. 27(a) and FIG. 27(c), electrode gel liquid junction sections 27004 are provided adjacent to a micro-flow path 27001. The electrode gel liquid junction sections 27004 each act as a border that isolates the flow path 27001 from the gel. When the gel is introduced into electrode gel paths 27003 via respective electrode gel injection openings 27002 and fills the electrode gel paths 27003 up to electrode gel discharge openings 27005, the electrode gel liquid junction sections 27004 prevent, by surface tension, the gel from leaking to the flow path 27001. Specifically, side surfaces of the flow path 27001 are each formed of a line of a large number of columns instead of a wall, and the gel and the liquid flowing in the flow path contact each other via inter-column spaces. The width of each of the inter-column spaces is desirably 500 $\mu$m or less. End points of the gel filled with an electrolyte are each connected to a metal cable 27006. When an electric field is applied to the electrodes, bubbles are generated at the positions of the electric cables, not in the flow path. The electrodes are connected to a DC voltage source 27007, which performs ON/OFF control based on the observation results on the flowing particles by use of a voltage application switching mechanism 27008. For example, when a sample particle 27009 to be recovered reaches an area between the electrodes, an electric field is applied to the electrodes. As a result, the position of the particle in the flow path can be changed. In this manner, the particles can be purified. In this example, the gel electrodes are used. Alternatively, in the case where the voltage to be applied does not reach the potential at which bubbles are generated, metal thin film electrodes 27010 may be used (FIGs. 27(b) and (d)). Owing to such a structure in which an array of end points of the gel in the gel electrodes contacts the sample solution flowing in the flow path in which the sample particles flow, a sufficient level of external force can be provided to the sample and thus the cells can be transferred sufficiently, unlike in the case where an external electric field is applied to one point.

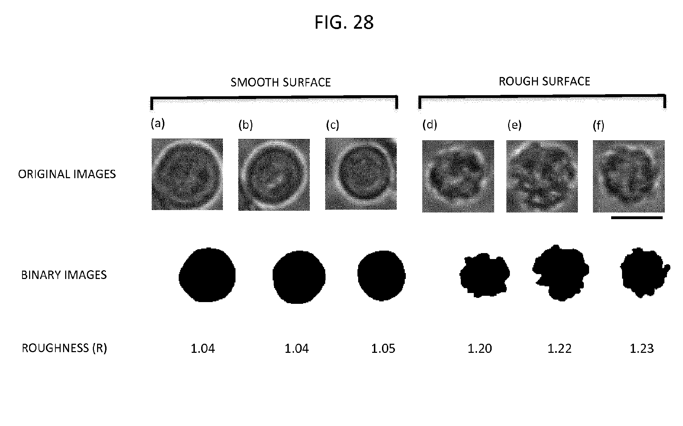

FIG. 28 is a schematic view showing an example of an identification process performed to separate cardiac muscle cells and fibroblasts from each other by use of an image in an image-recognizing cell sorter system. As can be seen from images in the top part of FIG. 28 that are captured by an image capturing mechanism of the cell sorter (top part: original images), a surface of the cardiac muscle cell is very smooth (smooth surface). By contrast, a surface of the fibroblast is very rough (rough surface). These images are binarized into the images in the middle part of FIG. 28 (binary images) so that an image of a boundary surface of each type of cell is made clear. Length l of the perimeter of the boundary surface is measured based on the number of pixels in the boundary surface, and also the surface area S of the fully painted inner surface is calculated based on the number of the fully painted pixels. Now, the actual length of the perimeter and the length of the perimeter converted from the surface area with an assumption that the inner surface is a circle are compared with each other by use of:

[Expression 3]

$$ R = \frac{l}{\sqrt{4\pi S}} $$

$$ \dots (1) $$

Thus, the roughness (R) of the surface can be quantified. In a bottom part of FIG. 28, values of R are actually shown. As a result of performing a more detailed measurement, it has been found that when the value of R is less than about 1.1, the cell is a cardiac muscle cell, whereas when the value of R is larger than this, the cell is some other cell. In this manner, a specific value of R is used as an index for purification of the cardiac muscle cell by image recognition, and thus the cells can be identified based on the difference in the roughness of the surface of the cell.

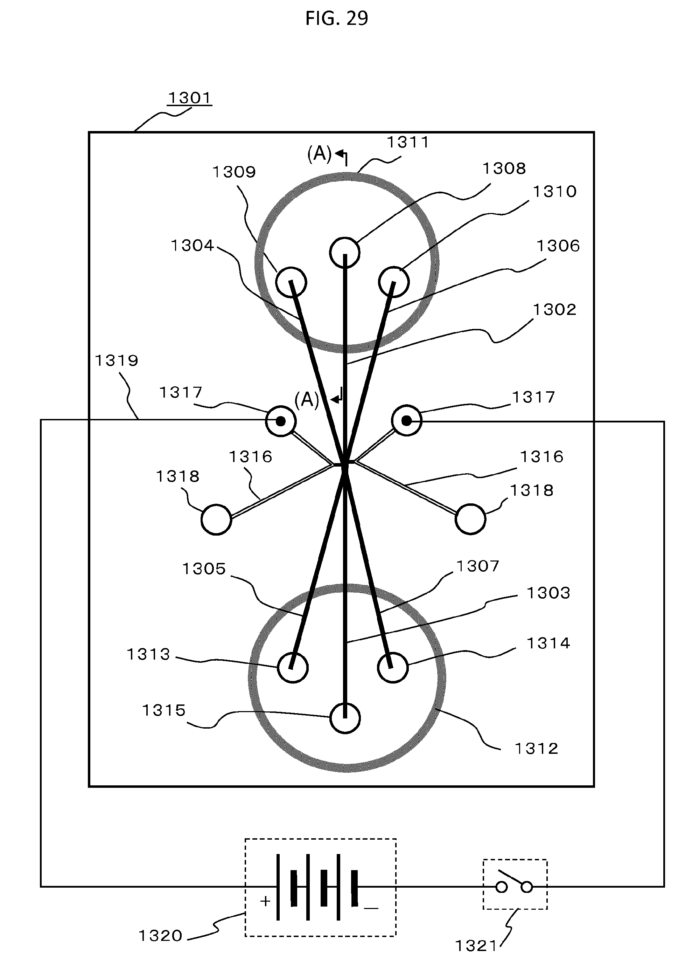

FIG. 29 is a schematic view showing an example structure of a cell sorter system using a combination of water and oil. In this case, a sheath liquid reservoir 1311 is filled with oil such as silicone oil or the like having a specific gravity smaller than that of water. When an aqueous solution of sample is dripped to a sample entrance opening 1308, the sample-containing water flows only in a flow path 1302, whereas oil flows in side flow paths 1304 and 1306. Since water and oil are not mixed together, it is not necessary to put a cap or the like at each of the entrances for the sheath liquid and the sample solution to isolate water and oil from each other.

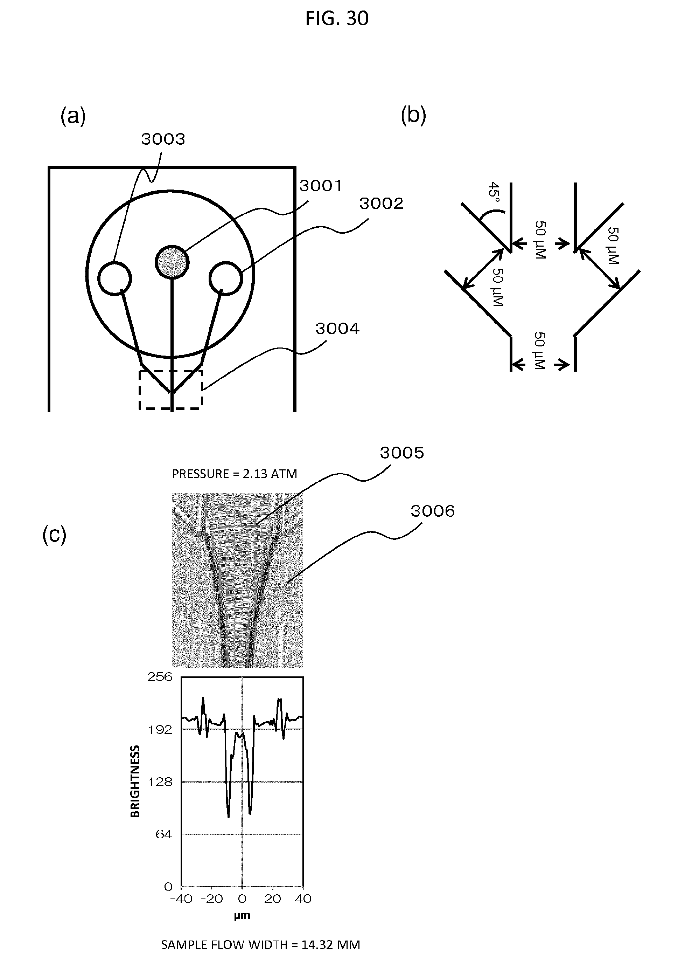

FIG. 30 provides schematic views showing an example structure of an area where water and oil are joined together, in a cell sorter system using a combination of water and oil. As described above with reference to FIG. 29, an aqueous solution of sample is introduced into an aqueous sample solution introduction opening 3001 in a reservoir filled with oil, and the oil in the reservoir is introduced into oil introduction openings 3002 and 3003. The introduced aqueous sample solution and oil are joined together in a joining area 3004. However, when the flow paths have a structure shown in FIG. 30(b), the flow of the aqueous sample solution 3005 can be narrowed by oil 3006 as shown in FIG. 30(c). An advantage of this structure is that since water and oil are never mixed together, the aqueous sample solution can be easily recovered without being diluted.

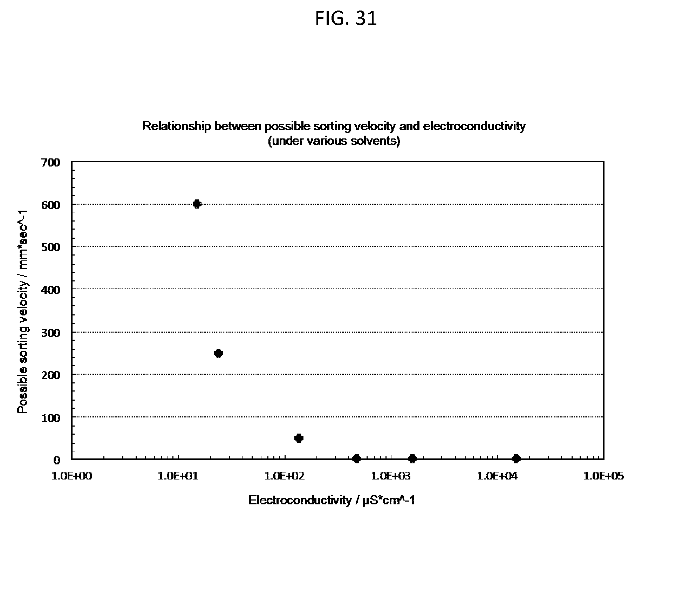

FIG. 31 is a graph showing the relationship between the amount of electrolyte (electroconductivity) in an aqueous solution and the possible cell separation velocity under various solution conditions. As can be seen from the figure, it is desirable that the composition of the sample solution has an ionic strength at which the conductivity of the aqueous sample solution is $10^2$ μs/cm or less. With such a composition, the particulates in the sample solution can be easily transferred by an electric field. Specifically, it is important that the solution has a composition which lowers the ionic strength while maintaining the osmic pressure when the cells are sorted alive. At the time of cell purification, it is desirable to use, for a sample solution, molecules that do not directly contribute to an increase of the ionic strength such as, for example, sugars or polymers.

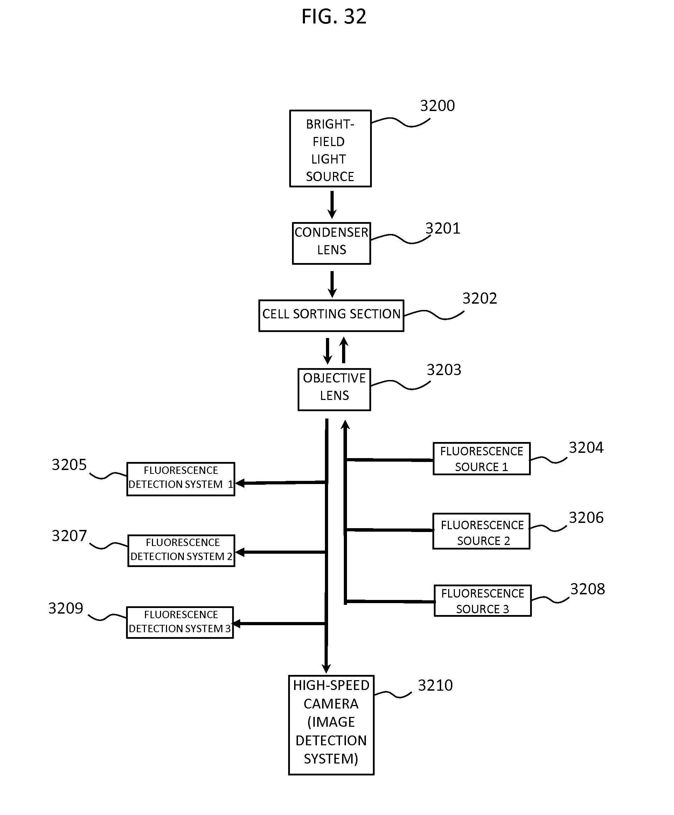

FIG. 32 schematically shows an example structure of an analysis system that performs measurement of the fluorescence intensity and acquisition of a high-speed bright-field microscopic image at the same time. Single-color light for observation that is emitted from a bright-field light source 3200 such as an LED flash light source or the like synchronized to a frame interval of a high-speed camera is collected by a condenser lens 3201, and is directed toward a flow path in which target cells in the blood flow as described above and also toward cells in a cell sorting section 3202 including a cell sorting chip that includes a cell sorting mechanism. The cells in the flow path can be focused on by an objective lens 3203. A depth-of-field improving technology using the above-described zoom lens system may be used. By fluorescence excitation light directed toward the objective lens from a plurality of fluorescence sources 3204, 3206 and 3208 such as single-color lasers or the like, fluorescence can be generated from fluorescence antibodies bonded to the cells in the flow path, nuclei stained with nucleus staining fluorescent dyes (DAPI, Hoechst 32258, etc.) or the like. The intensity of the obtained fluorescence can be quantitatively measured by fluorescence detection systems 3205, 3207 and 3209 formed of fluorescence intensity measurement systems such as photomultiplier tubes, photodiodes or the like. In this example, three excitation light sources and three fluorescence detection systems are provided. Alternatively, a single excitation light source can excite a plurality of fluorescence components. Therefore, any number of excitation light source(s) and any number of fluorescence detection system(s) can be combined. In this manner, while fluorescence detection of cells is performed, a bright-field image of the cells can be acquired at the same time by a high-speed camera 3210.

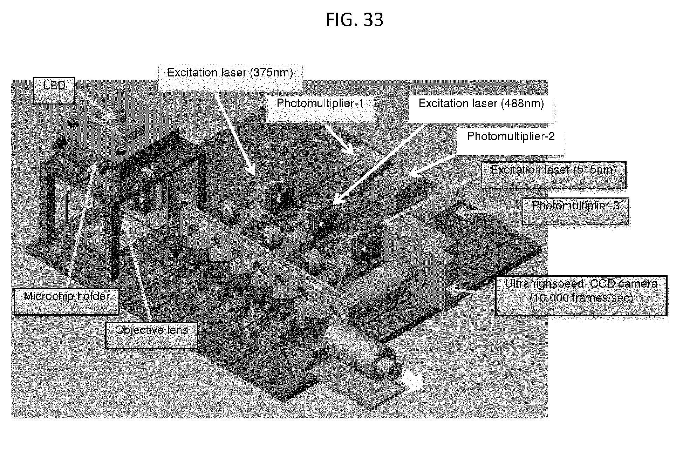

FIG. 33 schematically shows a specific example structure of an analysis system shown in FIG. 32 that performs measurement of the fluorescence intensity and acquisition of a high-speed bright-field microscopic image at the same time. In this example, a high-luminance LED flash light source that emits single-color light in an infrared range is used as a light source for a bright field (high-speed camera), and 375 nm, 488 nm and 515 nm lasers are used as excitation light sources of fluorescence dyes. As shown in FIG. 33, excitation lasers for introducing fluorescence into a fluorescence detector by use of a dichroic mirror are located such that the wavelength of light is monotonously increased from a short wavelength to a long wavelength in a step-wise manner. The longest-wavelength excitation laser is located closest to the high-speed camera. Owing to this structure, measurement of the fluorescence intensity and acquisition of a bright-field image can be performed at the same time at various wavelengths on the cells in the cell sorter chip located in a microchip holder.

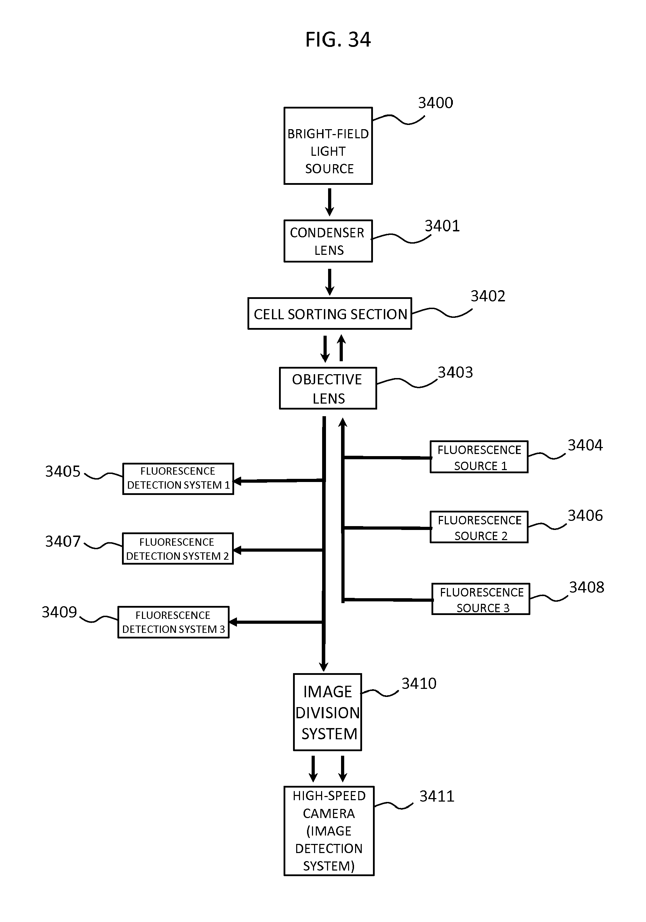

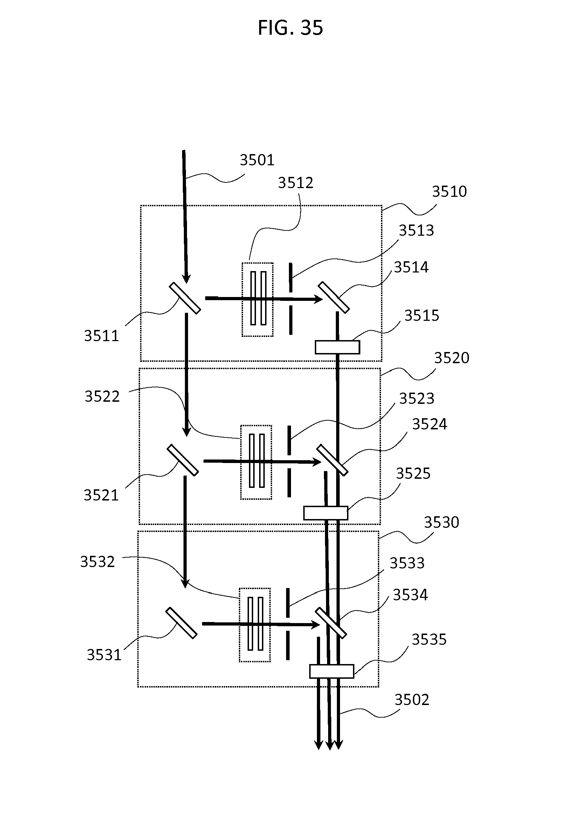

FIG. 34 schematically shows an example structure of an analysis system in the device shown in FIG. 32 that performs measurement of the fluorescence intensity and acquisition of a high-speed bright-field microscopic image and also performs acquisition of high-speed fluorescence microscopic images at the same time. Single-color light for observation that is emitted from a bright-field light source 3400 such as an LED flash light source or the like synchronized to a frame interval of a high-speed camera is collected by a condenser lens 3401, and is directed toward a flow path in which target cells in the blood flow as described above and also toward cells in a cell sorting section 3402 including a cell sorting chip that includes a cell sorting mechanism. The cells in the flow path can be focused on by an objective lens 3403. A depth-of-field improving technology using the above-described zoom lens system may be used. By fluorescence excitation light directed toward the objective lens from a plurality of fluorescence sources 3404, 3406 and 3408 such as single-color lasers or the like, fluorescence can be generated from fluorescence antibodies bonded to the cells in the flow path, nuclei stained with nucleus staining fluorescent dyes (DAPI, Hoechst 33258, etc.) or the like. The intensity of the resultant fluorescence can be quantitatively measured by fluorescence detection systems 3405, 3407 and 3409 formed of fluorescence intensity measurement systems such as photomultiplier tubes, photodiodes or the like. In this example, three excitation light sources and three fluorescence detection systems are provided. Alternatively, a single excitation light source can excite a plurality of fluorescence components. Therefore, any number of excitation light source(s) and any number of fluorescence detection system(s) can be combined. In a stage after this, an image division system 3410 that divides an optical microscopic image into a bright-field image and a fluorescence image and thus acquires a plurality of images at the same time by one high-speed camera is provided. An example of the image division system 3410 will be described later with reference to FIG. 35. By use of the image division system 3410, while fluorescence detection of cells is performed, a bright-field microscopic image of the cells can be acquired at the same time by the high-speed camera 3411.

FIG. 35 schematically shows an example structure of a device that acquires bright-field microscopic images and fluorescence microscopic images at the same time at a light receiving surface of one high-speed camera. Image data that enters an input optical path 3501 is first introduced into a first image division section 3510. This section includes a dichroic mirror 3511 which has an angle adjustment function and thus can fine-adjust the light reflection direction three-dimensionally. The dichroic mirror 3511 uses a particular wavelength as a cut-off wavelength, and reflects light having a wavelength longer or shorter than the cut-off wavelength so that the light is introduced into a filter system 3512. The filter system 3512 is formed of an intensity adjustment ND filter for aligning the intensities of the bright field or fluorescence on the high-speed camera to some extent, or formed of a bandpass filter for acquiring a fluorescence image of a wavelength band at which the image appears sharper. Then, the light is transferred to an image size adjustment system 3513. The image size adjustment system 3513 is formed of a movable light blocking plate and decreases the image size such that a plurality of divided images do not overlap with each other on a light receiving surface of the high-speed camera. Then, the light is transferred to a dichroic mirror 3514 that has an angle adjustment function and thus can fine-adjust the light reflection direction three-dimensionally. The light is next transferred to an optical lens system 3515. The optical lens system 3515 compensates for the difference in the image forming position from an image of another path. Such a difference is caused by, for example, the difference in the path in the optical system including the difference in the wavelength of light to be processed. After this, the light is introduced into a second image division section 3520, which has substantially the same structure as that of the first image division section 3510. Then, the light is introduced into a third image division section 3530, which has substantially the same structure as that of the first image division section 3510. An image 3502 output as a final image includes microscopic images formed of single-color light of different wavelengths. The microscopic images each have a part thereof cut off so as not to overlap with each other on the light receiving surface of the high-speed camera. In this figure, three image division sections having substantially the same structure are combined. Alternatively, two, or four more, image division sections may be combined. As shown in FIG. 35, a part of the division systems, namely, an "assembly of the dichroic mirror 3511 having an angle adjustment function and thus being capable of fine-adjusting the light reflection direction three-dimensionally, the filter system 3512, the image size adjustment system 3513 formed of a movable light-blocking plate that partially cuts off the image to decrease the image size, the dichroic mirror 3514 having an angle adjustment function and thus being capable of fine-adjusting the light reflection direction three-dimensionally, and the optical lens system 3515 that compensates for the difference in the image forming position" is provided as a module. Owing to this, a necessary number of divided images can be combined freely and flexibly by merely adjusting the number of the image division sections as described above.

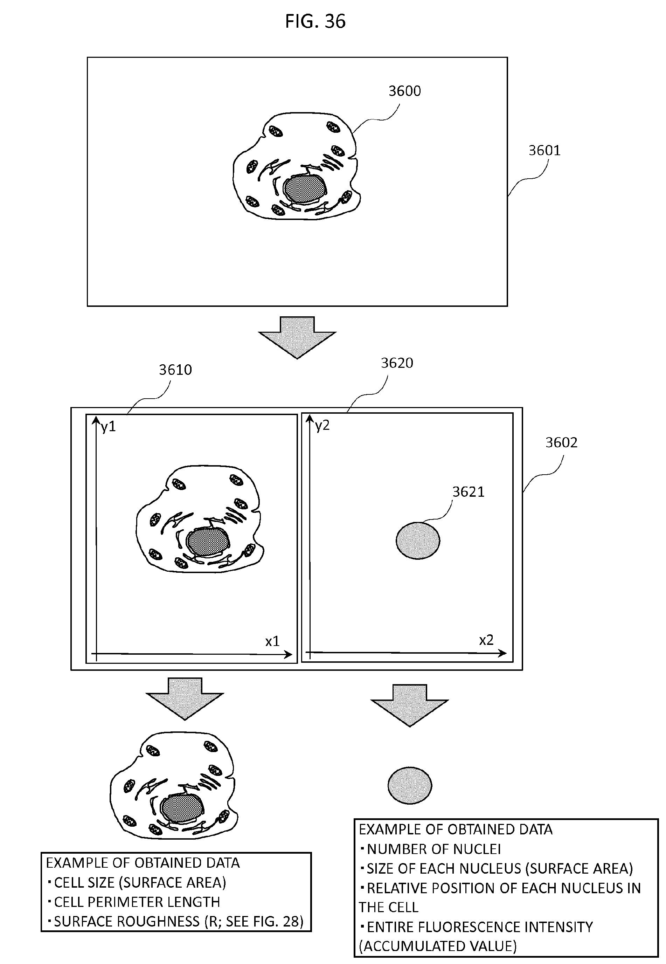

FIG. 36 schematically shows an example of one high-speed bright-field microscopic image and one high-speed fluorescence image acquired at the same time by one high-speed camera and an example of the information analysis. When entering the first division system, an image 3601 is cell 3600 data including the information of light of a plurality of wavelengths. The image data is processed by the division systems shown in FIG. 35, and as a result, a bright-field image 3610 and a nucleus fluorescence image 3620 can be acquired on one light receiving surface 3602 at the same time. As can be seen, extra parts on both of two sides of the input image are cut off by the image size adjustment system in the division system, and as a result, a plurality of images can be acquired with such a size so as not to overlap with each other on the light receiving surface. The position of each image on the light receiving surface of the high-speed camera can be freely adjusted by adjusting the position of the surface of the dichroic mirror having an angle adjustment function and thus being capable of fine-adjusting the light reflection direction to a plurality of three-dimensional directions. The optical lens system 3515 may be used to enlarge or reduce the bright-field image or the fluorescence image, so that images of different magnification ratios can be formed on the light receiving surface 3602 of one high-speed camera. By use of this function, the magnification ratio of a bright-field image can be decreased in order to check the state of the cell and also the vicinity thereof, and the magnification ratio of a fluorescence image can be increased in order to check details in the cells. The optical system that forms such a combination of images of different magnification ratios can be provided as a part of the module described above with reference to FIG. 35, namely, the "assembly of the dichroic mirror 3511 having an angle adjustment function and thus being capable of fine-adjusting the light reflection direction three-dimensionally, the filter system 3512, the image size adjustment system 3513 formed of a movable light-blocking plate that partially cuts off the image to decrease the image size, the dichroic mirror 3514 having an angle adjustment function and thus being capable of fine-adjusting the light reflection direction three-dimensionally, and the optical lens system 3515 that compensates for the difference in the image forming position". Such a module is not limited to being used in an imaging cell sorter, and may be incorporated into a general optical bright-field/fluorescence microscope system usable for performing substantially the same observation on stationary cell samples sorted and recovered by an imaging cell sorter.

A plurality of obtained images, for example, bright-field images, can be processed as follows. For example, pre-recorded image data for the case where there is no cell in the flow path may be subtracted from the image data of a cell in the flow path, so that an image of only the cell can be extracted. Therefore, the size (surface area) of the cell can be found based on the total number of pixels in an area having data left after the subtraction, and the perimeter length of the cell can be found based on the number of pixels at the boundary of the area having data left after the subtraction. These two pieces of data can be used to find the roughness R of the cell represented by expression 1 shown above. When R is about 1.3 or greater, it can be determined merely from a bright-field image that the cell is a cell cluster.

In substantially the same manner, a nucleus fluorescence image 3621 is obtained from a fluorescence image (stained nucleus) 3620, and thus the number of nucleus (nuclei), the surface area thereof, and the entire fluorescence intensity, namely, the accumulated value of luminance (corresponding to the photomultiplier data) can be obtained. The bright-field image and the fluorescence image are of the same site captured at different wavelengths, and thus the coordinate axes thereof match each other. Therefore, although the shape of the cell cannot be measured in the fluorescence image, the relative position of the stained nucleus in the cell can be estimated by use of the coordinate thereof in the bright-field image. FIG. 36 shows an example of a single normal cell containing one nucleus. Data can be obtained on a cell cluster or a multinucleated cell in substantially the same manner. For the above-described system in which a plurality of images of different magnification ratios are formed, substantially the same process can be performed by combining relative coordinates centered around the same origin (center of the image) in consideration of the difference in the magnification ratio.

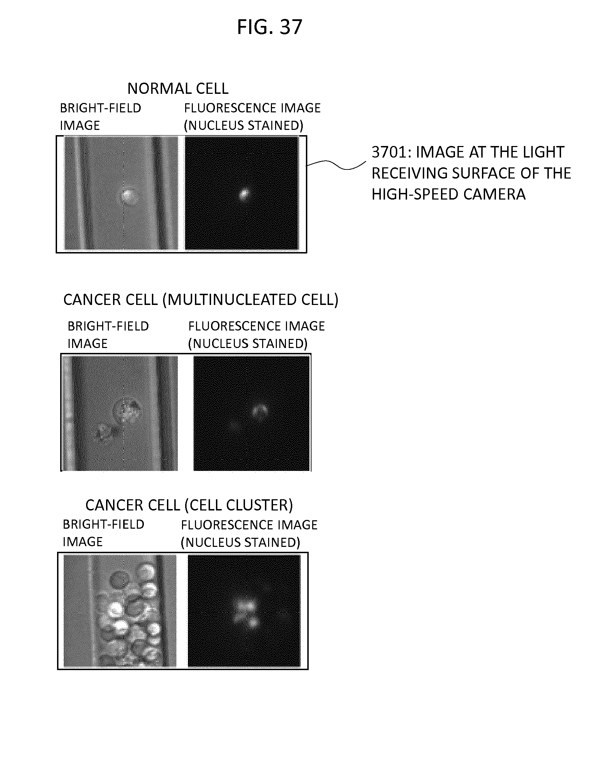

FIG. 37 shows an example of images of a high-speed bright-field microscopic image and a high-speed fluorescence microscopic image of a stained nucleus that are acquired at the same time at the light receiving surface of one high-speed camera. As described above, the relative coordinates of the two images are matched to each other in advance. Thus, by comparing the relative coordinates of the two images to each other, it can be checked at which site the nucleus, identifiable by the fluorescence image, is located in the cell image or the cell cluster image in the bright-field image. As a result of the comparison of the relative coordinates, it is seen that in a normal cell having a smooth surface and a normal size, one nucleus is fluorescent. By contrast, multinucleation is one index for a cancer cell. As shown the photographs, it is understood by the comparison of the relative coordinates that a plurality of nuclei are fluorescent in a cell larger than the normal cell. In the normal blood, there is no cell cluster. By contrast, in the blood of an animal of a cancer metastasis case, a plurality of nuclei are fluorescent. This demonstrates that there are a plurality of cells, and it is seen that there is a cell cluster. From the bright-field image of the cluster, it is seen that R > 1.3 at this point. A cluster can be identified merely by use of a bright-field image, even with no use of a fluorescence image. With the device according to the present invention, the following techniques can be used: (1) a cell cluster that is not present in the normal blood is identified as a cancer cell candidate in the blood and selectively recovered; (2) a multinucleated cell that is not present in the normal blood is identified as a cancer cell candidate in the blood and selectively recovered; (3) a cytomegalic cell that is not present in the normal blood is identified as a cancer cell candidate in the blood and selectively recovered; (4) the technique (1), (2) or (3) is combined with an analysis result that a fluorescent antibody (EpCam antibody, K-ras antibody, cytokeratin antibody, etc.) exhibits fluorescence intensity to one or a plurality of biomarkers for cancer cells as a result of the fluorescence intensity measurement described above with reference to FIG. 32, and a relevant cell is identified as a cancer cell and selectively recovered. By use of such a technique, a cancer cell in the blood can be identified by a novel biomarker, namely, "image of the shape of the cell, cluster state, or inner structure of multinucleation, etc.", not by the conventional molecular biomarker. The cancer cell candidate in the blood recovered by the above-described technique may be subjected to measurement regarding gene mutation by use of gene analysis means such as, for example, the above-described PCR analysis technology for microscopic cells. In this manner, a final determination is made on whether the cancer cell candidate is actually a cancer cell, or in the case where the cancer cell candidate is a cancer cell, the features of the cancer cells are identified. Regarding technique (1), the cell can be identified as a cancer cell candidate based on whether R > 1.3 in the bright-field image, or based on the size of the cell in the bright-field image and the number and distribution of the nuclei in the fluorescence image (i.e., based on the distance between the centers of gravity of a plurality of adjacent nuclei being 3 $\mu$m or longer). Regarding technique (2), the cell can be identified as a cancer cell candidate based on whether R < 1.3 in the bright-field image, and based on the number and distribution of the nuclei (i.e., based on the distance between the centers of gravity of a plurality of adjacent nuclei being within 3 $\mu$m). Regarding technique (3), the cell can be identified as a cancer cell candidate based on whether R < 1.3 in the bright-field image, and based on the size of the cell exceeding 20 $\mu$m when being converted into the diameter. Alternatively, a cell fulfilling at least one of the conditions of (1) through (3) is determined to be a cancer cell.

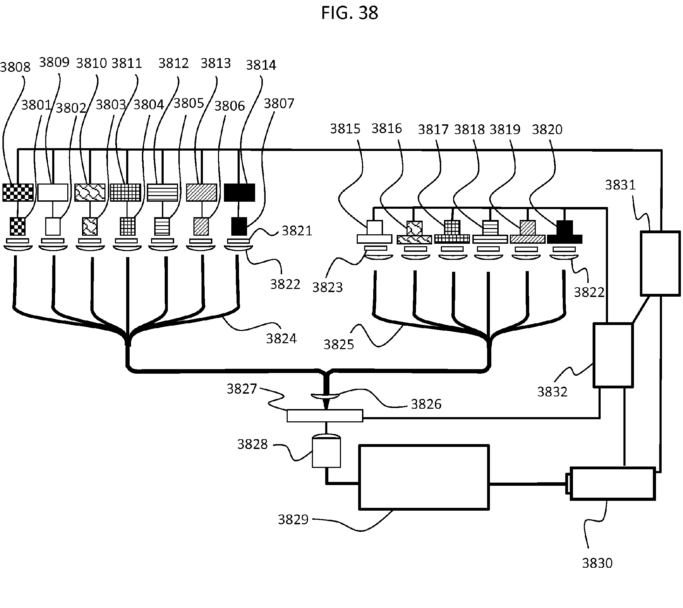

FIG. 38 schematically shows an example of a device structure by which a plurality of wavelengths of fluorescence excitation light are directed at the same time toward cells such as blood cells or the like flowing in a microchip by use of an optical fiber array, the fluorescence amounts of the emitted plurality of wavelengths are found at the same time, and fluorescence images of the plurality of wavelengths are acquired at the same time.

The device in the example shown in FIG. 38 includes an excitation light source section (3801 through 3807) including six fluorescence excitation light sources that generate single-color excitation light of different colors and one bright-field microscopic image light source. The excitation light sources are respectively connected to controllers 3808 through 3814 that are capable of independently controlling the light emission timing, the light emission time duration and the light emission intensity. The excitation light sources may each be, for continuous light emission, a combination of a common wide-band light emission source such as a xenon lamp, a mercury lamp or the like and a filter, or a laser light source such as a semiconductor excitation solid-state laser, a He-Ne laser or the like. One of the most suitable light sources is an LED light source, which outputs a narrow wavelength band of light with the intensity being stably controllable, is compact, and emits pulsed light at an interval shorter than a millisecond in an easily controllable manner. The controllers 3808 through 3814 connected to the excitation light sources can control the intensity and the output time duration of light emitted from the excitation light sources, and thus the excitation light sources can emit light ranging from continuous light to pulsed light. One example of a pulse exposure method by use of the pulsed light sources can be performed as described above with reference to FIG. 18. The excitation light sources are usable for the purpose of exciting fluorescence, needless to say. At least one of the excitation light sources can be used as a bright-field light source for the purpose of acquiring a bright-field image. In the case where a light source that outputs light of a wide wavelength band such as a xenon lamp or the like is used as the excitation light source, only light of a particular wavelength can be allowed to transmit the excitation light filter 3821 and be directed toward the sample.

At excitation light exits of the excitation light sources, the filters 3821 that are optimal for the optical bands of the excitation light emitted by the respective light sources 3801 through 3807 are provided. In a stage after this, lenses 3822 are provided. Excitation light generated by each excitation light source is converged and directed toward an end surface of a corresponding optical fiber 3824 and introduced thereto. The optical fibers are bundled, and the light is output from the surface of the other end of the bundle of the optical fibers, passes a light collection microlens 3826 and is directed toward a sample flowing in a micro-flow path in a microchip 3827. A representative flow path shape of the microchip may be as shown in FIG. 13. Each optical fiber typically has a diameter of 100 micrometers, and the micro-flow path in the microchip in which the sample cells flow typically has a width of 10 micrometers to 100 micrometers. The light collection microlens 3826 for the excitation light is provided just above the microchip in order to narrow the radiation area of the excitation light. By adjusting the focal point position of the microlens 3826, the diameter of the radiation area of the excitation light can be decreased so that the excitation light is directed toward only a very small area of about 1 micrometer in the micro-flow path in the microchip, or the diameter of the radiation area of the excitation light can be made about 100 microns so that the excitation light is directed to the entire width of the micro-flow path.

When the sample containing fluorescence-stained cancer cells or the like that is flowing in the micro-flow path is irradiated with the above-converged excitation light, fluorescence of a particular wavelength radiates from the sample containing the cancer cells or the like in spherical waves. Among the fluorescence, fluorescence radiating toward one semispherical part (toward a top surface of the chip in the example in FIG. 38) is guided by the microlens 3826 toward an end surface at which a bundle of optical fibers 3825 are prepared in a number corresponding to the number of the fluorescence wavelength bands to be measured. Then, the fluorescence is guided via the optical fibers to a fluorescence intensity detection section that detects the fluorescence intensity.

In this example, the fluorescence intensity detection section includes, for example, fluorescence detectors 3815 through 3820 that respectively detect the fluorescence intensity in six different fluorescence wavelength ranges. Fluorescence radiating from the end surface of each optical fiber 3825 is first guided to a lens 3822 located at an end point of the corresponding optical fiber, and then is guided to a filter 3823 for the fluorescence wavelength to be measured and to the corresponding fluorescence detector among the fluorescence detectors 3815 through 3820. Thus, the fluorescence measurement is performed. An appropriate fluorescence detector is a photomultiplier tube that can detect weak light and allows the amount of received light to be quantified easily. For both of the excitation light sources and the fluorescence detectors, it is desirable to select an appropriate device for a measurement target, such as a semiconductor element having an opto-electric conversion function, for example, an avalanche photodiode or the like. At a detection opening of each fluorescence detector, the fluorescence filter 3823 that is replaceable in accordance with the measurement conditions may be provided.

The detected fluorescence amount is analyzed by a fluorescence detection control unit 3832. When particular fluorescence or fluorescence combination is detected, or when particular fluorescence and a particular cell shape or cell cluster state obtained from a high-speed camera 3830 are observed, a feedback signal (pulsed voltage) for cell separation is transmitted to the microchip 3827. When, for example, a fluorescence-stained cancer cell flows in the micro-flow path and the fluorescence amount detected by the fluorescence detector reaches a threshold level set by the fluorescence detection control unit 3832 in advance or higher, a feedback signal is transmitted to the microchip. As a result, a voltage is applied to the electrodes mounted on the microchip, and thus the target cancer cell is recovered.

The fluorescence radiating toward the other semispherical part (toward a bottom surface of the chip in the example in FIG. 38) passes an objective lens 3828, then passes a device 3829 that acquires a bright-field microscopic image and a fluorescence microscopic image at the same time at the light receiving surface of one high-speed camera (multi-view system; FIG. 35 shows an example of the detailed structure thereof), and enters the high-speed camera 3830. In this manner, a bright-field microscopic image and a plurality of fluorescence microscopic images can all be acquired at the same time. The high-speed camera 3830 is connected to a light source control unit 3831, and the light source control unit 3831 is connected to each of the excitation light source controllers 3803 through 3814. Therefore, a clear cell image with no shape distortion can be acquired by synchronizing the timing of imaging by the high-speed camera to the timing of pulsed light emission by the excitation light source (an example of details of the synchronization is as shown in FIG. 18).

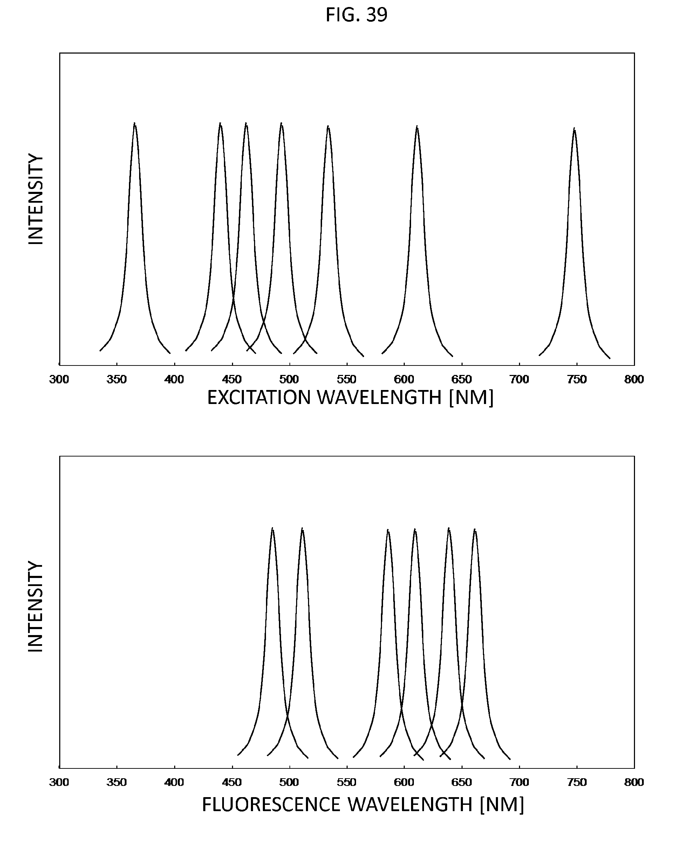

FIG. 39 shows an example of six types of fluorescence excitation light sources and one type of bright-field microscopic image acquisition light source that are included in the device shown in FIG. 38, and also shows an example of a selection of wavelength sets of fluorescence to be detected. This figure shows six wavelengths of fluorescence excitation light, one wavelength of light from the bright-field microscopic image light source, and six wavelengths of fluorescence to be detected. The numbers of the wavelengths can be easily increased by increasing the number of the light sources, the detectors and the optical fibers. In FIG. 39, the central wavelengths of the excitation light are 370, 440, 465, 498, 533, and 618 nm, the wavelength of light from the bright-field microscopic image light source is 750 nm, and the central wavelengths of fluorescence are 488, 510, 580, 610, 640 and 660 nm. Owing to this system, any of standard fluorescence reagents for fluorescence detection such as, for example, DAPI, Hoechst 33258, EGFP, FAM, HEX, TRITC, Texas Red, Cy3, Cy5 and the like can be flexibly selected in accordance with the purpose. At the same time, a bright-field microscopic image can be obtained.

A feature of the device system in this example is as follows. The device does not include a group of dichroic mirrors located on an optical path between the objective lens 3828 and the multi-view system 3829 to divide the wavelength band. The excitation light sources 3801 through 3807 and the fluorescence detectors 3815 through 3820 are not provided for the respective wavelength bands. Instead, as shown in the example in FIG. 38, an optical fiber array is provided on the side opposite to the objective lens, and the excitation light sources 3801 through 3807 and the fluorescence detectors 3815 through 3820, each for one wavelength band, are provided respectively for the optical fibers. Owing to this structure, a conventional problem that the fluorescence emitted from a sample such as a cancer cell or the like is attenuated while passing the plurality of dichroic mirrors is avoided. In addition, since it is made unnecessary to split the light into components of different wavelengths, a large number of excitation light components and fluorescence components can be used easily at the same time. Fluorescence that radiates toward the semispherical part opposite to the objective lens, which is conventionally undetectable and thus is wasted, is utilized to detect the fluorescence amount. Thus, the fluorescence radiating toward the other semispherical part, which is conventionally detected, is not attenuated and is entirely used to acquire fluorescence microscopic images. Therefore, the measurement of fluorescence amount and the acquisition of clear fluorescence microscopic images can be performed at the same time.

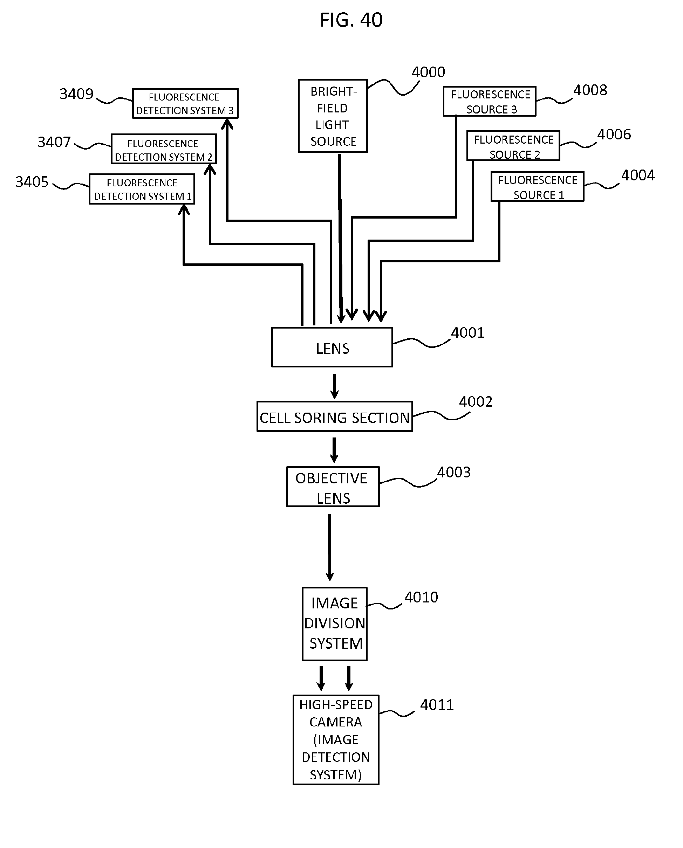

FIG. 40 schematically shows a structure of the device according to the present invention, an example of which is shown in FIG. 38. Single-color light for observation that is emitted from a bright-field light source 4000 such as an LED flash light source or the like synchronized to a frame interval of a high-speed camera is collected by a lens 4002, and is directed toward a flow path in which target cells in the blood flow as described above and also flow toward cells in a cell sorting section 4002 including a cell sorting chip that includes a cell sorting mechanism. The cells in the flow path can be focused on by an objective lens 4003. A depth-of-field improving technology using the above-described zoom lens system may be used. By fluorescence excitation light directed toward the cell sorting section 4002 from a plurality of fluorescence sources 4004, 4006 and 4008 such as single-color lasers or the like, fluorescence can be generated from fluorescence antibodies bonded to the cells in the flow path, nuclei stained with nucleus staining fluorescent dyes (DAPI, Hoechst 32258, etc.) or the like. The intensity of the obtained fluorescence can be quantitatively measured by fluorescence detection systems 4005, 4007 and 4009 formed of fluorescence intensity measurement systems such as photomultiplier tubes, photodiodes or the like. In this example, three excitation light sources and three fluorescence detection systems are provided. Alternatively, a single excitation light source can excite a plurality of fluorescence components. Therefore, any number of excitation light source(s) and any number of fluorescence detection system(s) can be combined. In a stage after this, an image division system 4010, an example of which is described above with reference to FIG. 35 is provided. The image division system 4010 divides an optical microscopic image into a bright-field image and a fluorescence image, so that a plurality of images can be acquired at the same time by one high-speed camera. By use of the image division system 4010, while the fluorescence intensity of cells is detected, a bright-field image of the cells can be acquired at the same time by a high-speed camera 4011.

In this example, the device according to the present invention is described as including image detection sections. Needless to say, the device according to the present invention is usable without the high-speed camera or the image detection sections. In such a case, the device is used as a detection system that detects a plurality of excitation light components and a plurality of fluorescence components at the same time with merely an optical fiber array.

So far, embodiments of the present invention have been described with reference to the drawings. The present invention is not limited to these embodiments and may be modified in various manners without departing from the spirit of the present invention.

INDUSTRIAL APPLICABILITY

The present invention is useful for purifying a trace amount of target cells in the blood in units of one cell and for performing analysis to provide, for example, correct gene information and expression information on target cells. The present invention is useful for purifying a trace amount of target cells such as spores of Bacillus anthracis or the like in units of one cell and for performing analysis to provide, for example, correct gene information and expression information ontarget cells at high speed.

The present invention is also useful as a technology for identifying and/or recovering a cancer cell circulating in the blood.

REFERENCE SIGNS LIST

| | |
|---|---|
| 1 | Cell analysis device system |
| 10 | Cell enrichment/staining/decoloration module |
| 101 | Cell sample vessel |
| 102 | Staining agent vessel |
| 103 | Washing detergent vessel |
| 104 | Separation head |
| 105 | Turntable |
| 106 | Enrichment/decoloration filter |
| 107 | Enrichment chamber |
| 108 | Chamber |
| 109 | Pressure pump |

| 110 | Waste liquid recovery tube |
|---|---|
| 111 | Recovery head |
| 112 | Recovery tube |
| 113 | Recovery chip |
| 114 | Chassis |
| 115 | Rotation arm |
| 20 | Image-detecting one-cell separation/purification module |
| 201 | Laser |
| 202 | Mirror |
| 203 | Collection lens |
| 204 | Dichroic mirror |
| 205 | Filter |
| 206 | Fluorescence-detecting photomultiplier |
| 207 | High-speed camera |
| 208 | Forward scattered light-detecting photodiode |
| 209 | Cell sorter chip |
| 210 | Chip electrode |
| 211 | Micro-flow path |
| 212 | Inlet |
| 213 | Outlet |
| 214 | Cell-enriched solution inlet |
| 215 | Cell enrichment section |
| 216 | Convergence section |
| 217 | Sorting section |
| 218 | Cell detection area |
| 219 | Flow of post-voltage application cells |
| 220 | Flow of pre-voltage application cells |
| 221 | Outlet |
| 222 | Outlet |
| 223 | Waste liquid recovery section |
| 224 | Cell recovery section |
| 225 | V-shaped comb-like electrode |
| 30 | One-cell genome analysis/expression analysis module |
| 31 | First temperature control unit |
| 32 | Second temperature control unit |
| 301 | Reaction tank |
| 302 | Heat exchange tank |
| 303 | Liquid reservoir tank |
| 304 | Pump |
| 305 | Switching valve |
| 306 | Assisting temperature control mechanism |
| 307 | Inlet A |
| 308 | Inlet B |
| 309 | Outlet A |
| 310 | Outlet B |
| 311 | Sample liquid |
| 312 | Fluorescence detector |
| 313 | Control analysis section |
| 314 | Check valve |
| 315 | Control signal |
| 40 | Liquid transfer module |
| 401 | Separation head |
| 402 | Separation chip |
| 403a, b | Z-axis transfer guide |
| 404 | Z-axis transfer motor |
| 405a, b | Arm rotation motor |
| 406 | Chassis |
| 50 | Control/analysis module (computer) |

| | |
|---|---|
| 801 | Vessel |
| 802 | Trace amount of sample |
| 803 | Rotator |
| 804 | Rotation shaft |
| 805 | Sample |
| 806 | Grinding agent |
| 810 | Rotator |
| 811 | Vessel |
| 812 | Flexible body |
| 813 | Rotation shaft |
| 814 | Tip |
| 815 | Spring mechanism |
| 820 | Curved cut |
| 821 | Mortar-like cut |
| 822 | Engaging structure |
| 823 | Semispherical rotator |
| 824 | Egg-like rotator |
| 825 | Protrusion-like rotator |
| 826 | Dish-like rotator |
| 830 | Vessel |
| 831 | Rotator |
| 832 | Grinding agent |
| 833 | Seal |
| 834 | Sample |
| 835 | Rotation shaft |
| 836 | Component |
| 840 | Integral vessel |
| 841 | Opening cutter |
| 1301 | Cell sorter chip |
| 1302, 1304, 1306 | Upstream flow path |
| 1303, 1305, 1307 | Downstream flow path |
| 1308 | Entrance opening for sample solution |
| 1309, 1310 | Entrance opening for sheath liquid |
| 1311 | Sheath liquid reservoir |
| 1312 | Waste liquid reservoir |
| 1313, 1314 | Exit opening for sheath liquid |
| 1315 | Exit opening for purified sample solution |
| 1316 | Flow path to be filled with gel |
| 1317 | Entrance opening for gel |
| 1318 | Exit opening for gel |
| 1319 | Cable |
| 1320 | Power source |
| 1321 | Switch |
| 1322 | Sample solution reservoir |
| 1323 | Purified sample recovering reservoir |
| 1401 | Cell sorter chip |
| 1402 | Cap |
| 1403 | Sheath liquid reservoir |
| 1404 | Sample solution reservoir |
| 1405 | Pressurized air introduction pipe |
| 1406 | Sample solution introduction tube |
| 1407 | Sheath liquid introduction tube |
| 1408 | Water level measurement sensor |
| 1409 | Flow path |
| 1501 | Cell sorter chip |
| 1502 | Large reservoir |
| 1503 | Air pressure application device |
| 1504 | Pressure sensor |

| | |
|---|---|
| 1505 | Distribution valve |
| 1506 | Sorted sample recovery reservoir |
| 1507 | Waste liquid recovery reservoir |
| 1601 | Sample solution flow |
| 1602, 1603 | Side sheath flow |
| 1604 | Cell monitor area |
| 1605 | Gel electrode |
| 1606 | Sorted sample recovery flow path |
| 1607 | Side sheath flow |
| 2001 | Cell sorter chip |
| 2002 | Solution reservoir |
| 2003 | Buffer introduction device |
| 2004 | Pressure sensor |
| 2005 | Distribution valve |
| 2006 | Sample solution introduction device |
| 2007 | Sample solution flow path |
| 2008 | Buffer flow path |
| 2009 | Sorting external force application mechanism |
| 20101, 20102, 20103 | Sorted sample/waste liquid recovery reservoir |
| 2101 | Cell sorter chip |
| 2102 | Buffer reservoir |
| 2103 | Buffer introduction device |
| 2104 | Pressure sensor |
| 2105 | Valve |
| 2106 | Sample solution introduction device |
| 21061 | Sample solution introduction flow path |
| 2107 | Flow path for sample solution and buffer solution |
| 2109 | Sorting external force application mechanism |
| 21101, 21102 | Sorted sample/waste liquid recovery reservoir |
| 23001 | Chip |
| 23002 | Sample introduction opening |
| 23003 | Target particle |
| 23004 | Unnecessary particle |
| 23005 | Micro-flow path |
| 23006 | Particle arraying mechanism |
| 23007 | Particle arraying external force input (electric force or sheath flow) |
| 23008 | Particle detection mechanism |
| 23009 | Particle purification mechanism |
| 23010 | Particle purification external force input ((gel or metal) electrodes + electric force) |
| 23011 | Target particle recovery opening |
| 23012, 23013 | Unnecessary particle reservoir |
| 24001 | Support substrate |
| 24002 | Metal thin film electrode first layer |
| 24003 | Metal thin film electrode second layer |
| 24004 | Insulating layer |
| 24005 | Sample flow path |
| 25001 | Sample introduction opening |
| 25002 | Micro-flow path |
| 25003 | Metal thin film-stacked parallel comb-like electrode |
| 25004 | Sample particle |
| 25005 | Metal thin film-stacked V-shaped comb-like electrode |
| 25006 | Sheath flow path |
| 25007 | Electrode on the bottom surface |
| 26001 | Target particle |
| 26002 | Unnecessary particle (charged negative) |
| 26003 | Unnecessary particle (charged positive) |
| 26004 | Micro-flow path |
| 26005 | Particle purification electrode |

| | |
|---|---|
| 26006 | Flow to the target particle recovery opening |
| 26007, 26008 | Flow to the unnecessary particle reservoir |
| 27001 | Micro-flow path |
| 27002 | Electrode gel injection opening |
| 27003 | Electrode gel path |
| 27004 | Electrode gel liquid junction section |
| 27005 | Electrode gel discharge opening |
| 27006 | Metal cable |
| 27007 | DC voltage source |
| 27008 | Voltage switching mechanism |
| 27009 | Sample particle |
| 27010 | Metal thin film electrode |
| 3001 | Aqueous sample solution introduction opening |
| 3002, 3003 | Oil introduction opening |
| 3004 | Joining area |
| 3005 | Aqueous sample solution |
| 3006 | Oil |
| 3200 | Bright-field light source |
| 3201 | Condenser lens |
| 3202 | Cell sorting section |
| 3203 | Objective lens |
| 3204, 3206, 3208 | Fluorescence source |
| 3205, 3207, 3209 | Fluorescence detection system |
| 3210 | High-speed camera (image detection system) |
| 3400 | Bright-field light source |
| 3401 | Condenser lens |
| 3402 | Cell sorting section |
| 3403 | Objective lens |
| 3404, 3406, 3408 | Fluorescence source |
| 3405, 3407, 3409 | Fluorescence detection system |
| 3410 | Image division system |
| 3411 | High-speed camera (image detection system) |
| 3501 | Input optical path (image) |
| 3510, 3520, 3530 | Image division section |
| 3511, 3521, 3531 | Dichroic mirror with an angle adjustment function |
| 3512, 3522, 3532 | Filter system |
| 3513, 3523, 3533 | Image size adjustment system |
| 3514, 3524, 3534 | Dichroic mirror with an angle adjustment function |
| 3515, 3525, 3535 | Optical lens system |
| 3600 | Cell |
| 3601 | Input image |
| 3602 | Light receiving surface of high-speed camera |
| 3610 | Output image 1 (bright-field) |
| 3620 | Output image 2 (fluorescence: stained nucleus) |
| 3621 | Nucleus fluorescence image |
| 3701 | Light receiving surface of high-speed camera |
| 3801-3807 | Fluorescence excitation light source |
| 3808-3814 | Excitation light source controller |
| 3815-3820 | Fluorescence detector |
| 3821 | Excitation light filter |
| 3822 | Lens |
| 3823 | Fluorescence filter |
| 3824, 3825 | Optical fiber |
| 3826 | Light collection microlens |
| 3827 | Microchip |
| 3828 | Objective lens |
| 3829 | Multi-view unit |
| 3830 | High-speed camera |

| 3831 | Light source control unit |
| 3832 | Fluorescence detection control unit |
| 4000 | Bright-field light source |
| 4001 | Condenser lens |
| 4002 | Cell sorting section |
| 4003 | Objective lens |
| 4004, 4006, 4008 | Fluorescence source |
| 4005, 4007, 4009 | Fluorescence detection system |
| 4010 | Image division system |
| 4011 | High-speed camera (image division system) |

**Claims**

1. An on-chip cell sorter system, comprising:

a cell sorter chip including a first flow path in which a specimen solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;
an optical system that acquires a digital image of a cell in the sample solution flowing in the first flow path in a first area upstream of the branch point, so that the target cell is identified by digital analysis performed on the image;
an external force application mechanism that applies an external force to the target cell or the cell other than the target cell flowing in the first flow path in a second area substantially matching the first area upstream of the branch point, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and
a control section that controls an operation of the optical system and the external force application mechanism.

2. The on-chip cell sorter system according to claim 1, which is structured such that a time lag between the timing when the cell identification result is obtained from the digital image acquired by the optical system and the timing when the external force is applied by the external force application mechanism is minimized.

3. An on-chip cell sorter system, comprising:

a cell sorter chip including a first flow path in which a specimen solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;
an optical system that acquires a digital image of a cell in the sample solution flowing in the first flow path in a first area upstream of the branch point, so that the target cell is identified by digital analysis performed on the image;
an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and
a control section that controls an operation of the optical system and the external force application mechanism;
wherein the optical system includes a microscope including an objective lens having a numerical aperture of 0.3 or less and a zoom lens optically coupled to the objective lens.

4. An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the external force application mechanism; wherein the cell sorter chip is located such that the first flow path is substantially parallel to a direction of the gravitational force and thus the sample solution flows substantially vertically from an upstream part to the downstream part of the first flow path.

5. An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the external force application mechanism; wherein the external force application mechanism includes a gel electrode or a metal electrode that applies an electric force to particulates containing cells flowing in the first flow path, and the sample solution has a conductivity of $10^2$ µS/cm or less.

6. The on-chip cell sorter system according to any one of claims 1 through 5, further comprising another external force application mechanism that applies, to the cells in the sample solution, an external force for arraying the cells in a third area upstream of the first area in the upstream part of the first flow path.

7. The on-chip cell sorter system according to claim 6, wherein the another external force application mechanism that applies, to the cells in the sample solution, an external force for arraying the cells applies the external force by use of an electric force or a sheath flow.

8. An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

an optical system that acquires a digital image of a cell in the sample solution flowing in the first flow path in a first area upstream of the branch point, so that the target cell is identified by digital analysis performed on the image;

an external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path;

a control section that controls an operation of the optical system and the external force application mechanism;

a reservoir that is in fluid communication with an upstream part of the first flow path and accommodates a buffer solution for a sheath liquid; and

a sample solution introduction flow path which is in fluid communication with the upstream part of the first flow path and from which the sample solution containing the cells is introduced into the first flow path;

wherein a tip part of the sample solution introduction flow path that is in fluid communication with the first flow path extends to a position downstream of a position at which the buffer solution is introduced into the first flow path.

9. An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

a first external force application mechanism that applies to the cells in the sample solution flowing in the first flow path an external force for arraying the cells in a preliminary area upstream of the branch point;

an optical system that acquires a digital image of a cell in the sample solution flowing in the first flow path in a first area upstream of the branch point and downstream of the preliminary area, so that the target cell is identified by digital analysis performed on the image;

a second external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the first and second external force application mechanisms;

wherein the first external force application mechanism is a comb-like electrode that is located on a surface of the first flow path and provides a repulsive force to particulates containing the cells in the sample solution, a cross-section of the first flow path perpendicular to a flow direction therein being tapered or protruding toward a center of a surface facing the surface on which the electrode is located, so that the arraying of the particulates is promoted.

10. An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

a first external force application mechanism that applies, to the cells in the sample solution flowing in the first flow path, an external force for arraying the cells in a preliminary area upstream of the branch point;

an optical system that acquires a digital image of a cell in the sample solution flowing in the first flow path in a first area upstream of the branch point and downstream of the preliminary area, so that the target cell is identified by digital analysis performed on the image;

a second external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the first and second external force application mechanisms;

wherein the second external force application mechanism includes gel electrodes located so as to contact the sample solution at both of two sides of the first flow path via an array of slits provided at a certain interval along the two side surfaces of the first flow path, so that in the case where the gel is in a sol solution state, the sol solution is prevented, by use of a surface tension of the sol solution, from leaking to the first flow path.

11. An on-chip cell sorter system, comprising:

a cell sorter chip including a first flow path in which a sample solution containing cells that contain a target cell flows, the first flow path being branched at a branch point in a downstream part into a target cell recovery flow path from which a liquid containing the target cell is recovered and a waste liquid recovery flow path from which a liquid containing a cell other than the target cell is recovered;

a first external force application mechanism that applies, to the cells in the sample solution flowing in the first flow path, an external force for arraying the cells in a preliminary area upstream of the branch point;

an optical system that acquires a digital image of a cell in the sample solution flowing in the first flow path in a first area upstream of the branch point and downstream of the preliminary area, so that the target cell is identified by digital analysis performed on the image;

a second external force application mechanism that applies an external force to the target cell or the cell other than the target cell flowing in the first flow path in a second area substantially matching the first area upstream of the branch point or downstream of the first area, based on a cell identification result obtained by the image analysis, and thus shifts an advancing direction of the cell supplied with the external force, so that the target cell is guided to the target cell recovery flow path while the cell other than the target cell is guided to the waste liquid recovery flow path; and

a control section that controls an operation of the optical system and the first and second external force application mechanisms;

wherein the first external force application mechanism includes a pair of flow paths which are in fluid communication with an upstream part of the first flow path and in which a side sheath liquid, for forming a side sheath flow, flows, and the side sheath liquid is of oil, which has a specific gravity smaller than that of water and thus is not well mixed with water.

12. The on-chip cell sorter system according to any one of claims 9 through 11, which is structured such that a time lag between the timing when the cell identification result is obtained from the digital image acquired by the optical system and the timing when the external force is applied by the second external force application mechanism is minimized.

13. The on-chip cell sorter system according to any one of claims 1 through 12, wherein the external force application mechanism that guides each of the cells to either the target cell recovery flow path or the waste liquid recovery flow path includes a gel electrode or a metal electrode that applies an electric force to the cells.

14. The on-chip cell sorter system according to any one of claims 1 through 13, wherein:

the target cell is a cardiac muscle cell; and
based on shapes of the cells acquired by image recognition performed by the optical system, a cell having R of less than 1.1 is identified as the cardiac muscle cell where R is represented by the following expression:
[Expression 4]

$$R = \frac{l}{\sqrt{4\pi S}}$$

$$(1)$$

15. A method for sorting target cells in a sample solution by use of the on-chip cell sorter system according to any one of claims 1 through 14.

16. An on-chip cell sorter system, comprising:

a cell sorter chip including a flow path in which a sample solution containing fluorescence-stained cells derived from a test subject flows;
an optical system including a bright-field light source and a fluorescence source that emit light toward the cells;
a detection system that acquires, at the same time, a bright-field image of each of the cells in the sample solution flowing in the flow path of the cell sorter chip, a fluorescence intensity of a fluorescence labeling substance bonded to the cell, and a fluorescence image of the cell;
control/analysis means that identify a multinucleated cell and/or a cell cluster flowing in the flow path based on the bright-field image, the fluorescence intensity and the fluorescence image; and

means that selectively recover the identified multinucleated cell and/or cell cluster.

17. The on-chip cell sorter system according to claim 16, wherein the control/analysis means acquire:

i) at least one piece of data selected from the group consisting of size (surface area) of the cell, perimeter length of the cell, and a value of R, which represents surface roughness of the cell obtained from the surface area and the perimeter length; and
ii) at least one piece of data selected from the group consisting of a wavelength spectrum of fluorescence of the fluorescence labeling substance bonded to the cell, an intensity spectrum of the fluorescence, a coordinate in the cell of the center of gravity of at least one fluorescence-stained region in the cell, and a surface area of the region; and

identifies the multinucleated cell and/or the cell cluster flowing in the flow path based on the data.

18. The on-chip cell sorter system according to claim 16 or 17, further comprising means that measure a nucleic acid sequence of a gene derived from the selectively recovered multinucleated cell and/or cell cluster.

19. The on-chip cell sorter system according to any one of claims 16 through 18, further comprising an image division mechanism having a function of dividing a light receiving surface of one high-speed camera so that the bright-field image and the fluorescence image are displayed on the light receiving surface at the same time.

20. The on-chip cell sorter system according to claim 19, further comprising a mechanism that performs adjustment such that magnification ratios of the bright-field image and the fluorescence image are different from each other.

21. The on-chip cell sorter system according to any one of claims 16 through 20, which is useable for identifying a cancer cell candidate in the blood.

22. A method for identifying a cancer cell candidate in the blood from a cell sample solution derived from a test subject by use of the on-chip cell sorter according to any one of claims 16 through 21, the method comprising the steps of:

(1) identifying a cell cluster that is not present in the normal blood as a cancer cell candidate in the blood and selectively recovering the cancer cell candidate;
(2) identifying a multinucleated cell that is not present in the normal blood as a cancer cell candidate in the blood and selectively recovering the cancer cell candidate;
(3) identifying a cytomegalic cell that is not present in the normal blood as a cancer cell candidate in the blood and selectively recovering the cancer cell candidate; and/or
(4) identifying a cell as a cancer cell candidate based on a combination of the step of (1), (2) or (3) and an analysis result that a fluorescent antibody exhibits a fluorescence intensity to one or a plurality of biomarkers for cancer cells, and selectively recovering the cancer cell candidate.

23. The method according to claim 22, wherein the fluorescent antibody is an EpCam antibody, a K-ras antibody or a cytokeratin antibody.

24. The method according to claim 22 or 23, wherein:

in step (1), the identification is performed based on whether R > 1.3 in the bright-field image, or based on the size of the cell in the bright-field image and the number and distribution of nuclei in the fluorescence image (i.e., based on whether the distance between the centers of gravity of a plurality of adjacent nuclei is 3 $\mu$m or longer);
in step (2), the identification is performed based on whether R < 1.3 in the bright-field image, and based on the number and distribution of nuclei (i.e., based on whether the distance between the centers of gravity of a plurality of adjacent nuclei is within 3 $\mu$m);
in step (3), the identification is performed based on whether R < 1.3 in the bright-field image, and based on whether the size of the cell exceeds 20 $\mu$m when being converted into the diameter; or
in step (4), a cell fulfilling at least one of the conditions of (1) through (3) is determined as a cancer cell.

25. An optical module usable in an optical bright-field/fluorescence microscopic system, the optical module comprising:

a first dichroic mirror having an angle adjustment function and thus being capable of fine-adjusting a light reflection direction three-dimensionally;

a filter system into which light having image data and reflected by the dichroic mirror is introduced;

an image size adjustment system which is formed of a movable light-blocking plate that adjusts an image size, the light that has passed the filter system being introduced into the image size adjustment system;

a second dichroic mirror having an angle adjustment function and thus being capable of fine-adjusting a light reflection direction three-dimensionally, the light that has passed the image size adjustment system being introduced into the second dichroic mirror; and

an optical lens system that compensates for a difference in image forming position, the light that has passed the second dichroic mirror being introduced into the optical lens system;

wherein image enlargement and image reduction can be performed by the optical lens system, so that an image including a bright-field image and a fluorescence image formed at different magnification ratios is generated.

26. The optical module according to claim 25, which is usable to acquire, at the same time, a bright-field image of a fluorescence-stained cell contained in a sample solution, a fluorescence intensity of a fluorescence labeling substance bonded to the cell, and a fluorescence image of the cell.

27. An on-chip cell sorter system, comprising:

a cell sorter chip including a flow path in which a sample solution containing fluorescence-stained cells derived from a test subject flows;

an optical system including a bright-field light source and one or at least two fluorescence sources that emit light toward the cells, optical fibers that respectively transmit light of a plurality of wavelengths, and a light-collecting lens that converges light to an observation target at a position irradiated with the light;

a first detection system including optical fiber(s) respectively corresponding to one or at least two fluorescence wavelengths and transmitting fluorescence for detecting a fluorescence intensity of each of the cells in the sample solution flowing in the flow path of the cell sorter chip, a bandpass filter that is located in a stage after the optical fiber(s) and allows transmission of fluorescence of a particular wavelength, and a fluorescence detector, wherein the first detection system acquires, at the same time, a fluorescence intensity of a fluorescence labeling substance bonded to each of the cells, the fluorescence intensity corresponding to each of the one or at least two fluorescence wavelengths;

a second detection system that acquires a bright-field image of each of the cells and a fluorescence image of the cell at the same time;

control/analysis means that control an operation of each of the systems and identify a multinucleated cell and/or a cell cluster flowing in the flow path based on the bright-field image, the fluorescence intensity and the fluorescence image; and

means that selectively recover the identified multinucleated cell and/or cell cluster.

EP 2 832 845 A1

# FIG. 1

ENTIRE PROCESS (ABOUT 35 MINUTES WITH A STANDARD SPECIMEN BY USE OF ALL THE FUNCTIONS)

ABOUT 15 MIN

| BLOOD SAMPLING | CELL DETECTION/EXTRACTION SECTION | CELL FIXATION SECTION |
|---|---|---|
| (ABOUT 5 MIN) | (ABOUT 5 MIN) | (ABOUT 5 MIN) |

BLOOD SAMPLE

CELL ENRICHMENT/ STAINING SECTION

CONTINUOUS ENRICHMENT; THE FUNCTION OF CONFIRMING THAT THE CELLS HAVE BEEN ENRICHED TO A TARGET CONCENTRATION IS ADDED.

ENRICHMENT

STAINING    ~2MIN

WASHING

IMAGE-DETECTING ONE-CELL SEPARATION/PURIFICATION SECTION

PRIMARY DETECTION

PURIFICATION OF BIOLOGICAL PARTICLES    ~1MIN

COLLECT UNTIL A CERTAIN NUMBER OF CELLS ARE OBTAINED FOR EACH CELL TYPE

A CERTAIN NUMBER OF CELLS OF EACH CELL TYPE IS GUARANTEED TO BE SUPPLIED

FEATURE EXTRACTION

~2MIN

DIVISION BY CELL TYPE

~2MIN

GENE ANALYSIS/EXPRESSION ANALYSIS SECTION

CONTAMINATION-FREE RE-CULTURING

DNA DETECTION/ IDENTIFICATION    ~3 MIN

44

FIG. 2

## FIG. 3

10

A

B



# FIG. 4

A

B

C

FIG. 5

FIG. 6

FIG. 7

ENTIRE PROCESS (ABOUT 35 MINUTES WITH A STANDARD SPECIMEN BY USE OF ALL THE FUNCTIONS)

ABOUT 15 MIN

| BLOOD SAMPLING | CELL DETECTION/EXTRACTION SECTION | CELL FIXATION SECTION |

(ABOUT 5 MIN) | (ABOUT 5 MIN) | (ABOUT 5 MIN)

BLOOD SAMPLE

CELL ENRICHMENT/ STAINING SECTION

CONTINUOUS ENRICHMENT; THE FUNCTION OF CONFIRMING THAT THE CELLS HAVE BEEN ENRICHED TO A TARGET CONCENTRATION IS ADDED.

ENRICHMENT

STAINING   ~2MIN

WASHING

IMAGE-DETECTING ONE-CELL SEPARATION/PURIFICATION SECTION

PRIMARY DETECTION PURIFICATION OF BIOLOGICAL PARTICLES   ~1MIN

COLLECT UNTIL A CERTAIN NUMBER OF CELLS ARE OBTAINED FOR EACH CELL TYPE
A CERTAIN NUMBER OF CELLS OF EACH CELL TYPE IS GUARANTEED TO BE SUPPLIED

FEATURE EXTRACTION   ~2MIN

DIVISION BY CELL TYPE   ~0.5 MIN

CELL FRACTURING EXTRACTION OF DNA IN THE CELL   ~2MIN

GENE ANALYSIS/EXPRESSION ANALYSIS SECTION

CONTAMINATION-FREE RE-CULTURING

DNA DETECTION/ IDENTIFICATION   ~3 MIN

50

FIG. 8

FIG. 9

# FIG. 10

a

814

b

820

c

821

d

822

e

823

f

824

g

825

h

826

# FIG. 11

FIG. 12

FIG. 13

FIG. 14

FIG. 15

FIG. 16

FIG. 17

A. CELL STATE

(A) QUIESCENT (WITH NUCLEUS)          (B) DIVIDED (WITHOUT NUCLEUS)

B. CAMERA IMAGE (SCHEMATIC VIEW)

(A) QUIESCENT (WITH NUCLEUS)          (B) DIVIDED (WITHOUT NUCLEUS)

## FIG. 18

1/2,000 SEC. HIGH-SPEED CAMERA TIMING CHART

500 μs

2 μs

CAMERA VERTICAL
SYNCHRONIZING SIGNAL

2 μs

2~496 μs

THE TIME DURATION IN
WHICH THE SHUTTER IS
CLICKED IS EXCLUDED
FROM THE EXPOSURE
TIME.

CAMERA
EXPOSURE TIME

ON THE BASIS OF THE FALL OF THE
VERTICAL SYNCHRONIZING SIGNAL

480 μs     1~5 μs

PULSED LIGHT SOURCE
LIGHT EMISSION SIGNAL

※WHEN A LIGHT EMISSION SIGNAL IS OUTPUT WITH A DELAY
OF ABOUT 480 μS AFTER THE RISE OF THE VERTICAL SYNCHRONIZING
SIGNAL, LIGHT EMISSION OCCURS WITHOUT FAIL.

SPATIAL RESOLUTION OF THE IMAGE
・IMAGE SIZE OF A 1/2,000 SEC. CAMERA: 12 μM x 12 μM
(IMAGE SIZE OF A 1/10,000 SEC. CAMERA: 8 μM 8 μM)
・IMAGE RESOLUTION WHEN OBSERVED BY A 20X
OBJECTIVE LENS: 0.6 μM/PIXEL

CCD camera

Zoom lens
(1 -12x)

Objective lens
(M Plan Apo 10x)

Illumination source

Chassis of upright
microscope

Objective lens

Polystyrene bead

0µ m

25µ m

FIG. 19A

62

# FIG. 19B

# FIG. 19C

## FIG. 20

VERTICAL DIRECTION

FIG. 21

VERTICAL DIRECTION

FIG. 22

21061

2102

2107

VERTICAL DIRECTION

FIG. 23

FIG. 24

(a)

(b)

FIG. 25

FIG. 26

FIG. 27

(a)

27002 27006 27005
27003
27007
27001
27008
27009
27002
27006
27005
27004
27003

(b)

27007
27001
27010
27008
27009
27010

(c)

27004
27003
27001
27003
27004
500 µm

(d)

27010
27001
27010
50 µm

# FIG. 28

# FIG. 29

## FIG. 30

(a)

(b)

(c)

PRESSURE = 2.13 ATM

3005

3006

3003

3001

3002

3004

45°

50 μM

50 μM

50 μM

50 μM

256

192

128

64

0

BRIGHTNESS

-40 -20 0 20 40
μm

SAMPLE FLOW WIDTH = 14.32 MM

FIG. 31

Relationship between possible sorting velocity and electroconductivity
(under various solvents)

## FIG. 32

BRIGHT-FIELD LIGHT SOURCE — 3200

CONDENSER LENS — 3201

CELL SORTING SECTION — 3202

OBJECTIVE LENS — 3203

FLUORESCENCE SOURCE 1 — 3204

FLUORESCENCE DETECTION SYSTEM 1 — 3205

FLUORESCENCE SOURCE 2 — 3206

FLUORESCENCE DETECTION SYSTEM 2 — 3207

FLUORESCENCE SOURCE 3 — 3208

FLUORESCENCE DETECTION SYSTEM 3 — 3209

HIGH-SPEED CAMERA (IMAGE DETECTION SYSTEM) — 3210

FIG. 33

FIG. 34

BRIGHT-FIELD LIGHT SOURCE — 3400

CONDENSER LENS — 3401

CELL SORTING SECTION — 3402

OBJECTIVE LENS — 3403

FLUORESCENCE SOURCE 1 — 3404

3405 — FLUORESCENCE DETECTION SYSTEM 1

FLUORESCENCE SOURCE 2 — 3406

3407 — FLUORESCENCE DETECTION SYSTEM 2

FLUORESCENCE SOURCE 3 — 3408

3409 — FLUORESCENCE DETECTION SYSTEM 3

IMAGE DIVISION SYSTEM — 3410

HIGH-SPEED CAMERA (IMAGE DETECTION SYSTEM) — 3411

FIG. 35

## FIG. 36

3600

3601

3610    3620

y1    y2

3602

3621

x1    x2

EXAMPLE OF OBTAINED DATA
・CELL SIZE (SURFACE AREA)
・CELL PERIMETER LENGTH
・SURFACE ROUGHNESS (R; SEE FIG. 28)

EXAMPLE OF OBTAINED DATA
・NUMBER OF NUCLEI
・SIZE OF EACH NUCLEUS (SURFACE AREA)
・RELATIVE POSITION OF EACH NUCLEUS IN THE CELL
・ENTIRE FLUORESCENCE INTENSITY (ACCUMULATED VALUE)

# FIG. 37

NORMAL CELL

BRIGHT-FIELD
IMAGE

FLUORESCENCE IMAGE
(NUCLEUS STAINED)

3701: IMAGE AT THE LIGHT
RECEIVING SURFACE OF THE
HIGH-SPEED CAMERA

CANCER CELL (MULTINUCLEATED CELL)

BRIGHT-FIELD
IMAGE

FLUORESCENCE IMAGE
(NUCLEUS STAINED)

CANCER CELL (CELL CLUSTER)

BRIGHT-FIELD
IMAGE

FLUORESCENCE IMAGE
(NUCLEUS STAINED)

FIG. 38

FIG. 39

## FIG. 40

4000

3409 — FLUORESCENCE DETECTION SYSTEM 3

BRIGHT-FIELD LIGHT SOURCE

FLUORESCENCE SOURCE 3 — 4008

3407 — FLUORESCENCE DETECTION SYSTEM 2

FLUORESCENCE SOURCE 2 — 4006

3405 — FLUORESCENCE DETECTION SYSTEM 1

FLUORESCENCE SOURCE 1 — 4004

LENS — 4001

CELL SORING SECTION — 4002

OBJECTIVE LENS — 4003

IMAGE DIVISION SYSTEM — 4010

HIGH-SPEED CAMERA (IMAGE DETECTION SYSTEM) — 4011

## INTERNATIONAL SEARCH REPORT

| | International application No. |
|---|---|
| | PCT/JP2013/059453 |

A. CLASSIFICATION OF SUBJECT MATTER

*C12M1/00*(2006.01)i, *C12M1/34*(2006.01)i, *C12Q1/02*(2006.01)i, *G01N21/27* (2006.01)i, *G01N21/64*(2006.01)i, *G01N33/48*(2006.01)i, *G01N33/483* (2006.01)i, *G01N37/00*(2006.01)i

According to International Patent Classification (IPC) or to both national classification and IPC

B. FIELDS SEARCHED

Minimum documentation searched (classification system followed by classification symbols)

C12M1/00, C12M1/34, C12Q1/02, G01N21/27, G01N21/64, G01N33/48, G01N33/483, G01N37/00

Documentation searched other than minimum documentation to the extent that such documents are included in the fields searched

| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
|---|---|---|
| X Y | Masahito HAYASHI et al., "Present state and prospects of measurement technologies for analysis of circulating tumor cell", Cytometry Research, 2011, vol.21, no.2, pages 1 to 6 | 1,2,6,7,13, 15 14 |
| Y | Kenji YASUDA, "Tokushu Nanobiology: Nanotech de Bio o Kaeru, Single Cell Handling and Purification Technologies", Cell technology, 2006, vol.25, no.8, pages 884 to 889 | 7,13-15 |
| Y | Kenji YASUDA, "Development of on-chip cell sorting system for small number of target cells", Cytometry Research, 2007, vol.17, no.1, pages 51 to 57 | 14,15 |

☐ Further documents are listed in the continuation of Box C. ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 21 June, 2013 (21.06.13) | 02 July, 2013 (02.07.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

| International application No. |
| --- |
| PCT/JP2013/059453 |

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
(See extra sheet.)

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.:
Claims 1 and 2, and the parts of claims 6, 7 and 13-15 which refer to claims 1 and 2

**Remark on Protest**      ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.

PCT/JP2013/059453

Continuation of Box No.III of continuation of first sheet(2)

Document 1: Masahito HAYASHI et al., "Present state and prospects of measurement technologies for analysis of circulating tumor cell", Cytometry Research, 2011, vol.21, no.2, pages 1 to 6

Document 2: JP 2011-191199 A (Japan Science and Technology Agency), 29 September 2011 (29.09.2011), entire text (Family: none)

Disclosed in claim 1 is an on-chip cell sorting system including: a cell sorting chip having a first flow path branched into a target cell collecting flow path and a liquid waste collecting flow path; an optical assembly for capturing a digital image of a cell in a liquid sample flowing through the first flow path and identifying a target cell by digital analysis; an external force applying mechanism for applying an external force to the cell on the basis of the cell identification result so as to shift the travel direction of the cell, thereby directing the cell to a desired collecting flow path; and a control unit for controlling the operation of the optical assembly and the external force applying mechanism.

Disclosed in claim 3 is that in the on-chip cell sorting system, the optical assembly includes a microscope which is made up of an objective lens having a numerical aperture of 0.3 or less and a zoom lens coupled optically to the objective lens.

Disclosed in claim 4 is that in the on-chip cell sorting system, the chip is disposed in a manner such that the first flow path is generally parallel to the direction of gravity.

Disclosed in claim 5 is that in the on-chip cell sorting system, the external force applying mechanism includes a gel electrode or a metal electrode, and the liquid sample has a particular electrical conductivity.

Disclosed in claim 8 is that the on-chip cell sorting system further includes a reservoir for storing a buffer liquid to be used for a sheath liquid and a flow path for introducing the liquid sample.

Disclosed in claim 9 is that in the on-chip cell sorting system, a second external force applying mechanism is further provided, and the first external force applying mechanism is a comb-shaped electrode in a particular shape.

Disclosed in claim 10 is that in the on-chip cell sorting system, a second external force applying mechanism is further provided, and the second external force applying mechanism is a gel electrode.

Disclosed in claim 11 is that in the on-chip cell sorting system, a second external force applying mechanism is provided, and the first external force applying mechanism includes a flow path which flows a side sheath liquid for producing a side sheath flow, the side sheath liquid being an oil having a specific gravity less than that of water.

Disclosed in claims 16 to 24 and 27 are an on-chip cell sorting system and a cell identifying method using the system, the on-chip cell sorting system including: a cell sorting chip with a flow path for flowing a liquid sample containing a cell dyed with fluorescence; an optical assembly; a detector unit for acquiring a bright-field image, a fluorescent intensity, and a fluorescent image of the cell; a control and analysis means for identifying a multinucleated cell or a cell cluster on the basis of the bright-field image, the fluorescent intensity, and the fluorescent image; and a means for selectively collecting the identified cell.

(Continued to next extra sheet)

**INTERNATIONAL SEARCH REPORT**

International application No.

PCT/JP2013/059453

Disclosed in claims 25 and 26 is an optical module including first and second dichroic mirrors, a filter assembly, and an optical lens assembly, wherein the optical lens assembly can magnify and contract an image.

Here, first, a comparison between the invention disclosed in claims 1, 3 to 5, and 8 to 11 and the invention disclosed in claims 16 to 24, and 27 shows that both the inventions are in common in that the inventions are an on-chip cell sorting system which includes a cell sorting chip with a flow path, an optical assembly, and a cell collecting means.

However, since disclosed in Document 1 is an on-chip cell sorting system that includes a cell sorting chip with a flow path, an optical assembly, and a cell collecting means, the invention disclosed in claims 1, 3 to 5, and 8 to 11, and the invention disclosed in claims 16 to 24, and 27 are not considered to have a common technical feature that makes a contribution over the prior art, and thus are not considered so linked as to form a single general inventive concept.

Furthermore, the inventions each disclosed in respective claims 1, 3 to 5, and 8 to 11 are all in common in that the inventions are an on-chip cell sorting system which includes: a cell sorting chip with a first flow path branched into a target cell collecting flow path and a liquid waste collecting flow path; an optical assembly for capturing a digital image of a cell in a liquid sample flowing through the first flow path and identifying a target cell by digital analysis; an external force applying mechanism for applying an external force to the cell on the basis of the cell identification result so as to shift the travel direction of the cell, thereby directing the cell to a desired collecting flow path; and a control unit for controlling the operation of the optical assembly and the external force applying mechanism.

However, since the on-chip cell sorting system configured as mentioned above is described in lines 3 to 5 in Document 1, each of the inventions mentioned above are not considered to have a common special technical feature and are not considered so linked as to form a single general inventive concept.

Furthermore, the invention disclosed in claim 1 and the invention disclosed in claims 25 and 26 are in common in that both the inventions relate to an optical assembly for capturing an image.

However, the technique for capturing an image by an optical assembly is well known at the time of filing of the present application. Thus, both the inventions are not considered to have a common technical feature that makes a contribution over the prior art, and are not considered so linked as to form a single general inventive concept.

Furthermore, the invention disclosed in claims 25 and 26 and the invention disclosed in claims 16 to 24, and 27 are in common in that both the inventions relate to an optical assembly for capturing a bright-field image and a fluorescent image.

However, since a microscope system which includes light sources each for capturing a bright-field image and a fluorescent image is disclosed in Document 2, both the inventions mentioned above are not considered to have a common technical feature that makes a contribution over the prior art, and are not considered so linked as to form a single general inventive concept.

(Continued to next extra sheet)

Form PCT/ISA/210 (extra sheet) (July 2009)

INTERNATIONAL SEARCH REPORT

International application No.

PCT/JP2013/059453

Consequently, the present application involves ten inventions in total, which comprise eight inventions that are set forth in claims 1, 3-5 and 8-11 respectively, the inventions set forth in claims 16-24 and 27, and the inventions set forth in claims 25 and 26

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2003107099 A
- JP 2004085323 A
- WO 2004101731 A
- JP 2011257241 A
- JP 2012055267 A
- WO 2012060163 A

**Non-patent literature cited in the description**

- **KAMARCK, M. E.** *Methods Enzymol.,* 1987, vol. 151, 150-165
- Micro Total Analysis. Kluwer Academic Publishers, 1988, vol. 98, 77-80
- *Analytical Chemistry,* 1988, vol. 70, 1909-1915